(19) Europäisches Patentamt
European Patent Office
Office européen des brevets

(11) EP 2 606 888 A1

(12) EUROPEAN PATENT APPLICATION

(43) Date of publication:
26.06.2013 Bulletin 2013/26

(21) Application number: 11194365.0

(22) Date of filing: 19.12.2011

(51) Int Cl.:
A61K 31/421 (2006.01) A61K 31/422 (2006.01)
A61K 31/426 (2006.01) A61K 31/427 (2006.01)
C07D 263/40 (2006.01) C07D 263/48 (2006.01)
C07D 277/34 (2006.01) C07D 277/42 (2006.01)
C07D 277/56 (2006.01) C07D 417/02 (2006.01)
C07D 413/02 (2006.01) C07D 403/02 (2006.01)
A61P 29/00 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicant: Dompe' S.P.A.
67100 L'Aquila (IT)

(72) Inventors:
- Moriconi, Alessio
  I-67100 L'AQUILA (IT)
- Bianchini, Gianluca
  I-67100 L'AQUILA (IT)
- Colagioia, Sandro
  I-67100 L'AQUILA (IT)
- Brandolini, Laura
  I-67100 L'AQUILA (IT)
- Aramini, Andrea
  I-67100 L'AQUILA (IT)
- Liberati, Chiara
  I-20132 MILAN (IT)
- Bovolenta, Silvia
  I-20132 MILAN (IT)

(74) Representative: Sutto, Luca
Ponzellini, Gioia e Associati S.r.l.
Via Mascheroni, 31
20145 Milano (IT)

# (54) Trpm8 antagonists

(57) The invention relates to compounds acting as selective antagonists of Transient Receptor Potential cation channel subfamily M member 8 (TRPM8), and having formula:

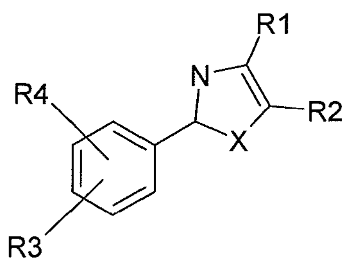

Said compounds are useful in the treatment of diseases associated with activity of TRPM8 such as pain, inflammation, ischaemia, neurodegeneration, stroke, psychiatric disorders, inflammatory conditions and urological disorders.

EP 2 606 888 A1

## Description

Brief description of the invention

The present invention relates to 2-aryl oxazole and thiazole derivatives that are useful for the prevention, reduction of the risk of, amelioration and/or treatment of diseases associated with the activity of the Transient Receptor Potential cation channel subfamily M member 8 (hereinafter TRPM8) also known as Cold Menthol Receptor 1 (CMR-1), and in particular for the prevention, reduction of the risk of, amelioration and/or treatment of inflammatory conditions, ischaemia, pain, neurodegeneration, psychiatric disorders, stroke and urological disorders. The invention further relates to pharmaceutical compositions containing the above compounds.

Background of the invention

Transient Receptor Potential (TRP) channels are one of the largest group of ion channels and, based on their sequence homology, are classified into 6 sub-families (TRPV, TRPM; TRPA, TRPC, TRPP and TRPML). TRP channels are cation-selective channels activated by several physical (such as temperature, osmolarity and mechanical stimuli) and chemical stimuli. TRPM8, which was cloned in 2002, is a non-selective cation channel of the TRP family expressed on a subpopulation of somatic sensory nerves on dorsal root ganglion and trigeminal ganglia that causes sensory nerve excitation. It is activated by mild cold temperatures and synthetic cool-mimetic compounds such as menthol, eucalyptol and icilin [McKemy D.D. et al., Nature (2002) 416, 52-58; Peier A.M. et al. Cell (2002) 108, 705-715]. Like several other TRP channels, TRPM8 is also gated by voltage [Nilius B. et al., J. Physiol. (2005) 567, 35-44]. The voltage dependence of TRPM8 is characterized by a strong outward rectification at depolarized transmembrane potential and a rapid and potential-dependent closure at negative membrane potentials. Cooling agents and menthol application shifts the activation curve towards more negative potentials, increasing the possibility for the opening of the channel and boosting inward currents at physiological membrane potentials. Other endogenous factors, such as phospholipase $A_2$ products [Vanden Abeele F. et al., J. Biol.Chem. (2006) 281, 40174-40182], endocannabinoids [De Petrocellis L. et al., Exp.Cell. Res. (2007) 313, 1911-1920] and PIP2 [Rohacs T. et al., Nat. Neurosci. (2005) 8, 626-634] also participate in channel regulation.

There is a lot of direct and indirect evidence of a pivotal role of TRPM8 channel activity in diseases such as pain ischemia and inflammatory disorders Further, it has been demonstrated that TRP channels transduce reflex signals that are involved in the overactive bladder of patients with damaged or abnormal spinal reflex pathways [De Groat W.C. et al., Urology (1997) 50, 36-52]. TRPM8 is activated by temperatures between 8°C and 28°C and expressed on the primary nociceptive neurons, including bladder urothelium, dorsal root ganglia, A-delta and C-fibers. The intravesical ice water or menthol also induce C-fiber mediated spinal micturition reflex in patients with urgency and urinary incontinence [Everaerts W. et al., Neurol. Urodyn. (2008) 27, 264-73].

Furthermore, TRPM8 is known to regulate $Ca^{2+}$ concentration influxes in response to cold temperature or pharmacological stimuli. Finally, in a recent paper, the potential role of TRPM8 in cold-induced asthma and in asthma exacerbation has been proposed, suggesting TRPM8 also a relevant target for the management of these pathologies [Xing H. et al., *Molecular Pain* (2008), **4**, 22-30].

The expression of the channel in brain, lung, bladder, gastrointestinal tract, blood vessels, prostate and immune cells provide further possibility for therapeutic modulation of the activity of TRPM8 in a wide range of pathologies. In particular, the disorders or diseases that have been proven to be affected by the modulation of TRPM8 are pain such as chronic pain, neuropathic pain including cold allodynia and diabetic neuropathy, postoperative pain, osteoarthritic pain, rheumatoid arthritic pain, cancer pain, neuralgia, neuropathies, algesia, fibromyalgia, nerve injury, migraine, headaches; ischaemia, neurodegeneration, stroke, psychiatric disorders, including anxiety and depression, and inflammatory conditions such as itch, irritable bowel syndrome, or respiratory diseases such as pulmonary hypertension asthma and COPD; urological disorders such as painful bladder syndrome, interstitial cystitis, detrusor overactivity (overactive bladder), urinary incontinence, neurogenic detrusor overactivity (detrusor hyperflexia), idiopathic detrusor overactivity (detrusor instability), benign prostatic hyperplasia, lower urinary tract disorders and lower urinary tract symptoms [Nilius B. et al. Science STKE (2005), 295, re8; Voets T. et al., Nat. Chem. Biol. (2005), 1, 85-92; Mukerji G. et al., Urology (2006), 6, 31-36; Lazzeri M. et al., Ther. Adv. Urol. (2009), 1, 33-42; Nilius B. et al., Biochim. Biophys. Acta (2007), 1772, 805-12; Wissenbach U. et al., Biol. Cell. (2004), 96, 47-54; Nilius B. et al., Physiol. Rev. (2007), 87, 165-217; Proudfoot C.J. et al., Curr. Biol. (2006), 16, 1591-1605].

Along the last few years, several classes of non peptide TRPM8 antagonists have been disclosed. WO 2006/040136, WO 2007/017092, WO 2007/017093, WO 2007/017094, and WO 2007/080109 describe benzyloxy derivatives as TRPM8 antagonists for the treatment of urological disorders; WO 2007/134107 describes phosphorous-bearing compounds as TRPM8 antagonists for the treatment of TRPM8-related disorders; WO 2009/012430 describes sulfonamides for the treatment of diseases associated with TRPM8; WO 2010/103381 describes the use of spirocyclic

piperidine derivatives as TRPM8 modulators in prevention or treatment of TRPM8-related disorders or diseases; and, WO 2010/125831 describes sulfamoyl benzoic acid derivatives as modulators of the TRPM8 receptor and their use in the treatment of inflammatory, pain and urological disorders.

A therapeutic area in which there is a particularly high need for the development of antagonists of TRPM8 is that of urological-related disorders. In fact, traditional drugs and medications currently available for the treatment of urinary incontinence and disorders are characterized by several side effects. For example, at the moment, the therapy of overactive bladder syndrome is based on the use of drugs, especially anticholinergic agents that affect peripheral neural control mechanisms or bladder detrusor smooth muscle contraction. These drugs inhibit parasympathetic nerves exerting a direct spasmolytic effect on the muscle of the bladder. The result of this action is the decrease of intravesicular pressure, an increase in capacity and a reduction in the frequency of bladder contraction. However, the use of anticholinergic agents is associated with serious side effects, such as dry mouth, abnormal visions, constipation and CNS disturbances, that impair the overall patient compliance. The inadequacies of the actual therapies highlight the need for novel, efficacious and safe drugs with fewer side effects.

Summary of the invention

The aim of the present invention is to provide novel antagonists of TRPM8 with high selectivity for this specific receptor and an adequate pharmacokinetic profile for use in therapy.

The present inventors have now found a class of 2-aryl oxazole and thiazole compounds acting as selective antagonists of Transient Receptor Potential cation channel subfamily M member 8 (hereinafter referred to as TRPM8) and satisfy the above desiderata.

These compounds are useful in the treatment of pathologies associated with the activity of TRPM8.

Detailed description of the Figures

Figure 1 shows a graphical representation of the 384 wells Compound Dilution Plate Layout used for the biological evaluation of the compounds of the invention as described in Example 119 wherein: in Column 1 wells contain assay buffer plus 0.5% DMSO; in Column 2: wells alternate Max signal control in first injection (Cooling agent 10 at 100$\mu$M, corresponding to $EC_{100}$) and Min signal control in first injection (assay buffer plus 0.5% DMSO final); in columns 3-22: wells contain assay buffer plus 0.5% DMSO final and to each of these wells a compound to be tested is added, at 3x concentrations; in Column 23 : wells alternate Max signal control in second injection (Assay Buffer) and Min signal control in second injection (Capsazepine at 50 mm, corresponding to $IC_{100}$); in Column 24: wells contain Capsazepine at 8 different concentrations in duplicate as reported in Example 119.

Figure 2 shows a graphical representation of the 384 wells Activator Plate Layout used for the biological evaluation of the compounds of the invention as described in Example 119 wherein in Column 1 wells contain Cooling Agent 10 at 8 concentrations dose-response in duplicate at different concentrations as reported in Example 119; in Columns 2-24 wells contain Cooling Agent 10 at at $EC_{50}$ (3x concentrations, the highest being 20$\mu$M final).

Figure 3 shows a graph with a typical kinetic response obtained in the test described in Example 119(a) for the compounds of Table 1. Signal expressed as Relative Light Units (y-axis), is reported vs time (sec. (x-axis) following the injection of a definite amount of control/ the test compounds. CA refers to the phase of Compound Addition, while TA to the Target Activation Phase perfomed in presence of the agonist, to increase the MAX Signal control, followed by the injection of a reference inhibitor for the complete abolition of the signal and the registration of the MIN Signal control.

Figure 4 shows the value of Maximum Possible Effect, measured as described in Example 119(b), observed after 2 hours from treatment with Control (1), Compound 10 (2) or Compound 45 (3).

Detailed description of the invention

A first object of the present invention are compounds of formula (I):

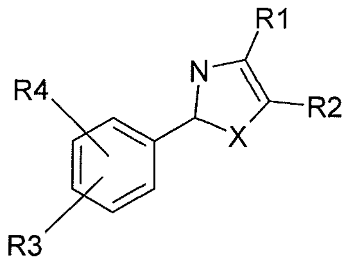

(I)

and pharmaceutically acceptable salts thereof,
wherein
**X** is selected from S or O;
**$R_1$** is selected from the group consisting of:

**-$OR_5$** wherein $R_5$ is selected from H; $C_1$-$C_4$ alkyl, trifluoromethanesulfonyl, benzyl, (trifluoromethyl)benzyl, (halo)benzyl, (trifluoromethyl)benzoyl, N-benzylcarbamoyl, cyclohexyloxyacetoyl substituted with at least one $C_1$-$C_3$ alkyl group, ($C_1$-$C_3$ alkoxy)methyl, $C_1$-$C_3$ alkanoyl and $CH_2CH_2NHR_6$, wherein
$R_6$ is selected from H and (furan-2-yl)methyl; and
**-$NHR_7$** wherein $R_7$ is selected from H, tert-butoxycarbonyl, $C_1$-$C_3$ alkanoyl, (4-trifluoromethyl)benzoyl, N-phenylaminoacarbonyl, $CH_2R_8$, wherein
**$R_8$** is selected from phenyl, benzo[d][1,3] dioxole, pyridin-3-yl, (pyrrolidin-1-yl)methyl, -$CH_2NHR_9$ wherein
**$R_9$** is selected from H, $C_1$-$C_3$ alkyl and cycloalkyl;
**$R_2$** is selected from the group consisting of
-$COOR_{10}$ wherein
**$R_{10}$** is selected from H, $C_1$-$C_3$ alkyl and cyclohexyloxycarbonyl substituted with at least one $C_1$-$C_3$ alkyl group;
-OH; -$CONH_2$; CN; -tetrazol-5-yl, 1-($C_1$-$C_3$ alkyl)tetrazol-5-yl, -5-($C_1$-$C_3$ alkyl)1,2,4 triazol-3-yl,- 5-($C_1$-$C_3$ alkyl) 1,2,4-oxadiazol-3yl, -5-($C_1$-$C_3$ alkyl) 1,3,4-oxadiazol-2-yl;
**$R_3$** is selected from F or H,
**$R_4$** is selected from H; $CH_3$; halogen; dimethylamino; pyridin-4yl; phenyl; 2- or 4- (halo)phenyl; 2- or 4-(trifluoromethyl) phenyl; 2- and/or 4-halobenzyloxy.

According to a preferred embodiment of the invention, in said compounds of formula I, $R_5$ may be selected from H, $C_1$-$C_4$ alkyl, trifluoromethanesulfonyl, benzyl, (trifluoromethyl)benzyl, (chloro)benzyl, (trifluoromethyl)benzoyl, N-benzylcarbamoyl, cyclohexyloxyacetoyl substituted with at least one $C_1$-$C_3$ alkyl group, (methoxy)methyl, propanoyl and $CH_2CH_2NHR_6$ wherein $R_6$ is as above. Particularly preferred among these compounds are compounds wherein $R_5$ is selected from H, methyl, isobutyl, trifluoromethanesulfonyl, benzyl, 4-(trifluoromethyl)benzyl, (chloro)benzyl, 4-(trifluoromethyl)benzoyl, N-benzylcarbamoyl, 2-isopropyl-5-methylcyclohexyloxyacetoyl, (methoxy)methyl, propanoyl and $CH_2CH_2NHR_6$ wherein $R_6$ is as above. According to a further preferred embodiment of the invention, also in combination with any of the preceding embodiment, in said compounds of formula I $R_7$ may be selected from H, tert-butoxycarbonyl, acetyl, 4-(trifluoromethyl)benzoyl, N-phenylaminoacarbonyl, $CH_2R_8$, wherein
$R_8$ is selected from phenyl, benzo[d][1,3] dioxole, pyridin-3-yl, (pyrrolidin-1-yl)methyl, -$CH_2NHR_9$ wherein
$R_9$ is selected from H, $C_1$-$C_3$ alkyl and cyclopentyl.

According to a further preferred embodiment of the invention, also in combination with any one of the preceding embodiments, in said compounds of formula I $R_{10}$ may be selected from H, $C_1$-$C_3$ alkyl and 2-isopropyl-5-cyclohexyl.

According to a further preferred embodiment of the invention, also in combination with any one of the preceding embodiments, in said compounds of formula I $R_4$ may be selected from H, $CH_3$, F, Cl, dimethylamino, preferably in position para, pyridin-4yl, phenyl, 2-F-penyl, 2-trifluoromethylphenyl and 2- or 4-halobenzyloxy, wherein said halo is preferably F or Cl.

According to a further preferred embodiment of the invention, in said compounds of formula I
X is selected from S or O;
$R_1$ is selected from the group consisting of:

-$OR_5$ wherein $R_5$ is selected from H, $C_1$-$C_4$ alkyl, trifluoromethanesulfonyl, benzyl, (trifluoromethyl)benzyl, (chloro)benzyl, (trifluoromethyl)benzoyl, N-benzylcarbamoyl, cyclohexyloxyacetoyl substituted with at least one $C_1$-$C_3$

alkyl group, (methoxy)methyl, propanoyl and $-CH_2CH_2NHR_6$, wherein
$R_6$ is selected from H and (furan-2-yl)methyl
$-NHR_7$ wherein $R_7$ is selected from H, tert-butoxycarbonyl, acetyl, (4-trifluoromethyl)benzoyl, N-phenylaminocarbonyl, $CH_2R_8$, wherein
$R_8$ is selected from phenyl, benzo[d][1,3] dioxole, pyridin-3-yl, (pyrrolidin-1-yl)methyl, $-CH_2NHR_9$ wherein
$R_9$ is selected from H, $C_1$-$C_3$ alkyl and cyclopentyl
$R_2$ is selected from the group consisting of
$-COOR_{10}$ wherein
$R_{10}$ is selected from H, $C_1$-$C_3$ alkyl and 2-isopropyl-5-methylcyclohexyloxycarbonyl,
-OH; $-CONH_2$; CN; tetrazol-5-yl or 1-($C_1$-$C_3$ alkyl)tetrazol-5-yl; -5-($C_1$-$C_3$ alkyl)1,2,4 triazol-3-yl;- 5-($C_1$-$C_3$ alkyl) 1,2,4- oxadiazol-3yl; -5-($C_1$-$C_3$ alkyl) 1,3,4-oxadiazol-2-yl;
$R_3$ is selected from F or H,
$R_4$ is selected from H, F, Cl, dimethylamino, preferably in position para, pyridin-4yl, phenyl, 2- F-penyl, 2-trifluoromethylphenyl, 2- and/or 4-F-benzyloxy.

Particularly preferred compounds of the invention are compounds of formula I wherein $R_1$ is selected from:

$-OR_5$, wherein $R_5$ is selected from H, benzyl, (chloro)benzyl, (trifluoromethyl)benzoyl, $CH_2$-$CH_2NH_2$; and
$-NHCH_2CH_2CH_2R_8$ wherein $R_8$ is selected from H and $C_1$-$C_3$ alkyl.

Particularly preferred among the compounds of the invention are also compounds of formula I wherein $R_2$ is selected from $COOR_{10}$ wherein $R_{10}$ is selected from H, $C_1$-$C_3$ alkyl.

Particularly preferred among the compounds of the invention are also compounds of formula I wherein $R_3$ is H.

Particularly preferred among the above compounds are those compounds of formula I wherein:

$R_1$ is selected from:

$OR_5$, wherein $R_5$ is selected from H, benzyl, (chloro)benzyl, (trifluoromethyl)benzoyl; and
$CH_2$-$CH_2NH_2$; and
$NHCH_2CH_2CH_2R_8$ wherein $R_8$ is selected from $C_1$-$C_3$ alkyl and H;
$R_2$ is $COOR_{10}$ wherein $R_{10}$ is selected from H, $C_1$-$C_3$ alkyl
R3 is H.

According to a preferred embodiment of the invention, also in combination with any preceding embodiment, when X is S, in the above compounds of formula I when $R_1$ is OH and $R_2$ is COOH, R4 is different from Cl in meta position on the aromatic ring and when $R_1$ is OH and $R_2$ is COOH, $R_3$ and $R_4$ are not H at the same time.

According to a further preferred embodiment of the invention, also in combination with any preceding embodiments, in said compounds of formula I when $R_3$ is F, $R_3$ is in position ortho of the aromatic ring and $R_4$ is F in position para of the aromatic ring, and when $R_3$ is H, $R_4$ is in position para or meta on the aromatic ring.

According to a further preferred embodiment of the invention, the compounds of formula I are selected from:

2-(4-chlorophenyl)-4-hydroxy-1,3-thiazole-5-carboxylic acid (compound n. 1)
4-hydroxy-2-(4-methylphenyl)-1,3-thiazole-5-carboxylic acid (compound n. 2)
2-(3-fluorophenyl)-4-hydroxy-1,3-thiazole-5-carboxylic acid (compound n. 3)
2-(4-fluorophenyl)-4-hydroxy-1,3-thiazole-5-carboxylic acid (compound n. 4)
methyl 4-hydroxy-2-phenyl-1,3-thiazole-5-carboxylate (compound n. 5)
methyl 2-(2,4-difluorophenyl)-4-hydroxy-1,3-thiazole-5-carboxylate (compound n. 6)
ethyl 4-hydroxy-2-phenyl-1,3-thiazole-5-carboxylate (compound n. 7)
ethyl 2-(4-chlorophenyl)-4-hydroxy-1,3-thiazole-5-carboxylate (compound n. 8)
ethyl 4-hydroxy-2-(4-methylphenyl)-1,3-thiazole-5-carboxylate (compound n. 9)
ethyl 2-(3-fluorophenyl)-4-hydroxy-1,3-thiazole-5-carboxylate (compound n. 10)
ethyl 2-(4-fluorophenyl)-4-hydroxy-1,3-thiazole-5-carboxylate (compound n. 11)
ethyl 4-hydroxy-2-(pyridin-4-yl)-1,3-thiazole-5-carboxylate (compound n. 12)
ethyl 2-[4-(dimethylamino)phenyl]-4-hydroxy-1,3-thiazole-5-carboxylate (compound n. 13)
ethyl 2-(3-chlorophenyl)-4-hydroxy-1,3-thiazole-5-carboxylate (compound n. 14)
ethyl 4-hydroxy-2-[2'-(trifluoromethyl)biphenyl-3-yl]-1,3-thiazole-5-carboxylate (compound n. 15)
ethyl 2-(2'-fluorobiphenyl-3-yl)-4-hydroxy-1,3-thiazole-5-carboxylate (compound n. 16)
ethyl 4-hydroxy-2-[2'-(trifluoromethyl)biphenyl-4-yl]-1,3-thiazole-5-carboxylate (compound n. 17)

ethyl 2-(2'-fluorobiphenyl-4-yl)-4-hydroxy-1,3-thiazole-5-carboxylate (compound n. 18)
ethyl 2-{4-[(2-fluorobenzyl)oxy]phenyl}-4-hydroxy-1,3-thiazole-5-carboxylate (compound n. 19)
ethyl 2-{4-[(4-fluorobenzyl)oxy]phenyl}-4-hydroxy-1,3-thiazole-5-carboxylate (compound n. 20)
ethyl 2-(4-fluorophenyl)-4-{[(trifluoromethyl)sulfonyl]oxy}-1,3-thiazole-5-carboxylate (compound n. 21)
ethyl 4-methoxy-2-(4-methylphenyl)-1,3-thiazole-5-carboxylate (compound n. 22)
ethyl 2-(4-methylphenyl)-4-(2-methylpropoxy)-1,3-thiazole-5-carboxylate (compound n. 23)
ethyl 4-(benzyloxy)-2-phenyl-1,3-thiazole-5-carboxylate (compound n. 24)
ethyl 4-[(3-chlorobenzyl)oxy]-2-(4-chlorophenyl)-1,3-thiazole-5-carboxylate (compound n. 25)
ethyl 4-[(3-chlorobenzyl)oxy]-2-(3-fluorophenyl)-1,3-thiazole-5-carboxylate (compound n. 26)
ethyl 4-[(4-chlorobenzyl)oxy]-2-phenyl-1,3-thiazole-5-carboxylate (compound n. 27)
ethyl 4-[(4-chlorobenzyl)oxy]-2-(3-chlorophenyl)-1,3-thiazole-5-carboxylate (compound n. 28)
ethyl 4-[(4-chlorobenzyl)oxy]-2-(4-methylphenyl)-1,3-thiazole-5-carboxylate (compound n. 29)
ethyl 4-[(4-chlorobenzyl)oxy]-2-(3-fluorophenyl)-1,3-thiazole-5-carboxylate (compound n. 30)
ethyl 4-[(2-chlorobenzyl)oxy]-2-phenyl-1,3-thiazole-5-carboxylate (compound n. 31)
ethyl 4-[(2-chlorobenzyl)oxy]-2-(4-fluorophenyl)-1,3-thiazole-5-carboxylate (compound n. 32)
ethyl 4-[(2-chlorobenzyl)oxy]-2-(4-chlorophenyl)-1,3-thiazole-5-carboxylate (compound n. 33)
ethyl 4-[(2-chlorobenzyl)oxy]-2-(3-chlorophenyl)-1,3-thiazole-5-carboxylate (compound n. 34)
ethyl 4-[(2-chlorobenzyl)oxy]-2-(4-methylphenyl)-1,3-thiazole-5-carboxylate (compound n. 35)
ethyl 2-phenyl-4-{[4-(trifluoromethyl)benzoyl]oxy}-1,3-thiazole-5-carboxylate (compound n. 36)
ethyl 2-(3-fluorophenyl)-4-{[4-(trifluoromethyl)benzoyl]oxy}-1,3-thiazole-5-carboxylate (compound n. 37)
ethyl 2-(4-methylphenyl)-4-{[4-(trifluoromethyl)benzoyl]oxy}-1,3-thiazole-5-carboxylate (compound n. 38)
ethyl4-(2-((1R,2S,5R)-2-isopropyl-5-methylcyclohexyloxy)acetoyloxy)-2-(4-methylphenyl) -1,3-thiazole-5-carboxylate (compound n. 39)
ethyl 4-[(benzylcarbamoyl)oxy]-2-(4-chlorophenyl)-1,3-thiazole-5-carboxylate (compound n. 40)
ethyl 4-(2-aminoethoxy)-2-(4-chlorophenyl)-1,3-thiazole-5-carboxylate (compound n. 41)
ethyl 2-(4-chlorophenyl)-4-{2-[(furan-2-ylmethyl)amino]ethoxy}-1,3-thiazole-5- carboxylate (compound n. 42)
4-[(4-chlorobenzyl)oxy]-2-(4-methylphenyl)-1,3-thiazole-5-carboxylic acid (compound n. 43)
4-[(4-chlorobenzyl)oxy]-2-phenyl-1,3-thiazole-5-carboxylic acid (compound n. 44)
4-[(4-chlorobenzyl)oxy]-2-(4-chlorophenyl)-1,3-thiazole-5-carboxylic acid (compound n. 45)
4-[(4-chlorobenzyl)oxy]-2-(3-chlorophenyl)-1,3-thiazole-5-carboxylic acid (compound n. 46)
4-(benzyloxy)-2-phenyl-1,3-thiazole-5-carboxylic acid (compound n. 47)
4-[(3-chlorobenzyl)oxy]-2-(3-fluorophenyl)-1,3-thiazole-5-carboxylic acid (compound n. 48)
4-[(2-chlorobenzyl)oxy]-2-phenyl-1,3-thiazole-5-carboxylic acid (compound n. 49)
4-[(2-chlorobenzyl)oxy]-2-(4-fluorophenyl)-1,3-thiazole-5-carboxylic acid (compound n. 50)
4-[(2-chlorobenzyl)oxy]-2-(4-chlorophenyl)-1,3-thiazole-5-carboxylic acid (compound n. 51)
4-[(2-chlorobenzyl)oxy]-2-(3-chlorophenyl)-1,3-thiazole-5-carboxylic acid (compound n. 52)
4-[(2-chlorobenzyl)oxy]-2-(4-methylphenyl)-1,3-thiazole-5-carboxylic acid (compound n. 53)
4-[(2-chlorobenzyl)oxy]-2-(3-fluorophenyl)-1,3-thiazole-5-carboxylic acid (compound n. 54)
2-phenyl-4-{[4-(trifluoromethyl)benzyl]oxy}-1,3-thiazole-5-carboxylic acid (compound n. 55)
2-(3-fluorophenyl)-4-{[4-(trifluoromethyl)benzyl]oxy}-1,3-thiazole-5-carboxylic acid (compound n. 56)
2-phenyl-4-{[4-(trifluoromethyl)benzoyl]oxy}-1,3-thiazole-5-carboxylic acid (compound n. 57)
2-(3-fluorophenyl)-4-{[4-(trifluoromethyl)benzoyl]oxy}-1,3-thiazole-5-carboxylic acid (compound n. 58)
2-(4-methylphenyl)-4-{[4-(trifluoromethyl)benzoyl]oxy}-1,3-thiazole-5-carboxylic acid (compound n. 59)
4-methoxy-2-(4-methylphenyl)-1,3-thiazole-5-carboxylic acid (compound n. 60)
2-(4-methylphenyl)-4-(2-methylpropoxy)-1,3-thiazole-5-carboxylic acid (compound n. 61)
ethyl 4-[(tert-butoxycarbonyl)amino]-2-(4-fluorophenyl)-1,3-thiazole-5-carboxylate (compound n. 62)
ethyl 4-amino-2-(4-fluorophenyl)-1,3-thiazole-5-carboxylate hydrochloride (compound n. 63)
ethyl 4-(acetylamino)-2-(4-methylphenyl)-1,3-thiazole-5-carboxylate (compound n. 64)
ethyl 2-(4-methylphenyl)-4-{[4-(trifluoromethyl)benzoyl]amino]-1,3-thiazole-5-carboxylate (compound n. 65)
ethyl 2-(4-methylphenyl)-4-[(phenylcarbamoyl)amino]-1,3-thiazole-5-carboxylate (compound n. 66)
ethyl 4-[(2-aminoethyl)amino]-2-(4-methylphenyl)-1,3-thiazole-5-carboxylate (compound n. 67)
ethyl 2-(4-chlorophenyl)-4-{[2-(methylamino)ethyl]amino}-1,3-thiazole-5-carboxylate (compound n. 68)
ethyl 2-(4-chlorophenyl)-4-{[2-(propylamino)ethyl]amino]-1,3-thiazole-5-carboxylate (compound n. 69)
ethyl 4-[(2-aminoethyl)amino]-2-(4-chlorophenyl)-1,3-thiazole-5-carboxylate (compound n. 70)
ethyl 4-{[2-(methylamino)ethyl]amino}-2-(4-methylphenyl)-1,3-thiazole-5-carboxylate (compound n. 71)
ethyl 4-[(2-aminoethyl)amino]-2-[2'-(trifluoromethyl)biphenyl-4-yl]-1,3-thiazole-5-carboxylate (compound n. 72)
ethyl 4-[(2-aminoethyl)amino]-2-[2'-(trifluoromethyl)biphenyl-3-yl]-1,3-thiazole-5-carboxylate (compound n. 73)
ethyl 2-(4-chlorophenyl)-4-{[2-(cyclopentylamino)ethyl]amino}-1,3-thiazole-5-carboxylate (compound n. 74)

ethyl 2-phenyl-4-{[2-(pyrrolidin-1-yl)ethyl]amino}-1,3-thiazole-5-carboxylate (compound n. 75)
ethyl 4-(benzylamino)-2-(3-fluorophenyl)-1,3-thiazole-5-carboxylate (compound n. 76)
ethyl 4-[(1,3-benzodioxol-5-ylmethyl)amino]-2-(3-fluorophenyl)-1,3-thiazole-5-carboxylate (compound n. 77)
ethyl 2-(3-fluorophenyl)-4-[(pyridin-3-ylmethyl)amino]-1,3-thiazole-5-carboxylate (compound n. 78)
4-[(2-aminoethyl)amino]-2-(4-methylphenyl)-1,3-thiazole-5-carboxylic acid (compound n. 79)
4-{[2-(methylamino)ethyl]amino}-2-(4-methylphenyl)-1,3-thiazole-5-carboxylic acid (compound n. 80)
4-[(2-aminoethyl)amino]-2-(3-fluorophenyl)-1,3-thiazole-5-carboxylic acid (compound n. 81)
sodium 4-[(3-chlorobenzyl)oxy]-2-(3-fluorophenyl)-1,3-thiazole-5-carboxylate (compound n. 82)
sodium 4-[(4-chlorobenzyl)oxy]-2-(4-methylphenyl)-1,3-thiazole-5-carboxylate (compound n. 83)
sodium 4-(4-chlorobenzyloxy)-2-(4-chlorophenyl)-1,3-thiazole-5-carboxylate (compound n. 84)
sodium 4-(2-chlorobenzyloxy)-2-(4-chlorophenyl)-1,3-thiazole-5-carboxylate (compound n. 85)
sodium 4-(2-chlorobenzyloxy)-2-(4-methylphenyl)-1,3-thiazole-5-carboxylate (compound n. 86)
sodium 4-(2-chlorobenzyloxy)-2-(3-fluorophenyl)-1,3-thiazole-5-carboxylate (compound n. 87)
sodium 4-(4-chlorobenzyloxy)-2-(3-fluorophenyl)-1,3-thiazole-5-carboxylate (compound n. 88)
(1 R,2S,5R)-2-isopropyl-5-methylcyclohexyl-4-(benzyloxy)-2-(4-methylphenyl)-1,3-thiazole-5-carboxylate (compound n. 89)
(1 R,2S,5R)-2-isopropyl-5-methylcyclohexyl-4-hydroxy-2-(4-methylphenyl)-1,3-thiazole-5-carboxylate (compound n. 90)
ethyl 2-(4-chlorophenyl)-4-(methoxymethoxy)-1,3-thiazole-5-carboxylate (compound n. 91)
2-(4-chlorophenyl)-4-(methoxymethoxy)-1,3-thiazole-5-carboxylic acid (compound n. 92)
2-(4-chlorophenyl)-4-(methoxymethoxy)-1,3-thiazole-5-carboxamide (compound n. 93)
2-(4-chlorophenyl)-4-(methoxymethoxy)-1,3-thiazole-5-carbonitrile (compound n. 94)
2-(4-chlorophenyl)-5-(1H-tetrazol-5-yl)-1,3-thiazol-4-ol (compound n. 95)
2-(4-chlorophenyl)-5-(1-methyl-1H-tetrazol-5-yl)-1,3-thiazol-4-ol (compound n. 96)
2-(3-fluorophenyl)-5-(1-methyl-1H-tetrazol-5-yl)-1,3-thiazol-4-ol (compound n. 97)
2-(4-chlorophenyl)-5-(5-methyl-4H-1,2,4-triazol-3-yl)-1,3-thiazol-4-ol (compound n. 98)
2-(3-fluorophenyl)-5-(5-methyl-4H-1,2,4-triazol-3-yl)-1,3-thiazol-4-ol (compound n. 99)
2-(4-chlorophenyl)-5-(5-methyl-1,2,4-oxadiazol-3-yl)-1,3-thiazol-4-ol (compound n. 100)
2-(3-fluorophenyl)-5-(5-methyl-1,2,4-oxadiazol-3-yl)-1,3-thiazol-4-ol (compound n. 101)
3-{4-[(4-chlorobenzyl)oxy]-2-(3-fluorophenyl)-1,3-thiazol-5-yl}-5-methyl-1,2,4-oxadiazole (compound n. 102)
2-(4-chlorophenyl)-5-(5-methyl-1,3,4-oxadiazol-2-yl)-1,3-thiazol-4-ol (compound n. 103)
2-(3-fluorophenyl)-5-(5-methyl-1,3,4-oxadiazol-2-yl)-1,3-thiazol-4-ol (compound n. 104)
ethyl 4-hydroxy-2-phenyl-1,3-oxazole-5-carboxylate (compound n. 105)
ethyl 2-(3-fluorophenyl)-4-hydroxy-1,3-oxazole-5-carboxylate (compound n. 106)
ethyl 4-hydroxy-2-(4-methylphenyl)-1,3-oxazole-5-carboxylate (compound n. 107)
ethyl 4-[(4-chlorobenzyl)oxy]-2-phenyl-1,3-oxazole-5-carboxylate (compound n. 108)
ethyl 4-[(4-chlorobenzyl)oxy]-2-(3-fluorophenyl)-1,3-oxazole-5-carboxylate (compound n. 109)
ethyl 4-[(4-chlorobenzyl)oxy]-2-(4-methylphenyl)-1,3-oxazole-5-carboxylate (compound n. 110)
ethyl 2-phenyl-4-{[4-(trifluoromethyl)benzoyl]oxy}-1,3-oxazole-5-carboxylate (compound n. 111)
4-[(4-chlorobenzyl)oxy]-2-phenyl-1,3-oxazole-5-carboxylic acid (compound n. 112)
4-[(4-chlorobenzyl)oxy]-2-(3-fluorophenyl)-1,3-oxazole-5-carboxylic acid (compound n. 113)
4-[(4-chlorobenzyl)oxy]-2-(4-methylphenyl)-1,3-oxazole-5-carboxylic acid (compound n. 114)
2-(3-fluorophenyl)-5-(5-methyl-1,2,4-oxadiazol-3-yl)-1,3-oxazol-4-0l (compound n. 115)
3-{4-[(4-chlorobenzyl)oxy]-2-(3-fluorophenyl)-1,3-oxazol-5-yl}-5-methyl-1,2,4-oxadiazole (compound n. 116)
ethyl 2-(3-fluorophenyl)-5-hydroxy-1,3-thiazole-4-carboxylate (compound n. 117).

As it will be described in details in Example 118, the present inventors have found that the above compounds 1-117 are potent antagonists of TRPM8.

In details, all of the above compounds have been tested in a high-throughput screening (HTS) cellular-based assay for the human TRPM8 and have shown an antagonist activity with a $IC_{50}$ below 30 $\mu$M. Compounds 10 and 45 have also been tested in a calcium influx assay, which has confirmed the antagonist activity of the tested compounds.

Thus, a second object of the present invention are the above compounds of formula (I) for use as antagonists of TRPM8, preferably of human TRPM8.

In order to obtain confirmation of the data obtained *in vitro* compounds 10 and 45 have also been tested in two *in vivo* models.

In details, as will be described in Example 119, the activity of the compounds has been tested in an isovolumetric bladder model, an animal model for the evaluation of drugs active on pain induced by contractions of bladder, and in a Chronic Constriction Injury of sciatic nerve (CCI), an animal model of neuropathic pain.

In the first model, the compounds showed significant efficacy in inhibiting rhythmic bladder contractions and micturition frequency. Moreover, both the compounds did not change Amplitude of Micturition (AM) when compared to basal values, suggesting that they are selective for the afferent arm of micturition reflex with no effect on the efferent pathway.

In the second model, the tested compounds showed a significant antiallodynic activity at 2 hours.

As will be demonstrated in Example 120, the compounds of the invention show a high selectivity for TRPM8 and are thus devoid of side effects due to interference with other ion channels and GPCRs. In fact, both 10 and 45 have been demonstrated to be selective in a wide range of ion channel and GPCRs.

Furthermore, as shown in Example 121 the compounds of the invention have an optimal pharmacokinetic profile.

Thus, the compounds of the invention are particularly suitable to be used in therapy.

Accordingly, a third object of the present invention are the above compounds for use as medicaments.

A fourth object of the present invention are the above compounds for use in the prevention, reduction of the risk of, amelioration and/or treatment of a disease associated with activity of TRPM8.

According to the present invention, by "disease that is associated with activity of TRPM8" it is preferably meant a disease selected from pain, inflammatory conditions, ischaemia, neurodegeneration, stroke, urological disorders, and psychiatric disorders.

Preferably, said pain is selected from chronic pain, cancer pain, neuropathic pain, which is meant to include cold allodynia and diabetic neuropathy, postoperative pain, osteoarthritic pain, rheumatoid arthritic pain, neuralgia, neuropathies, fibromyalgia, algesia, nerve injury, migraine, headaches.

Preferably, said inflammation is selected from itch, irritable bowel disease and airways disease, the latter being preferably selected from pulmonary hypertension, COPD and asthma.

Preferably, said urological disorders are selected from painful bladder syndrome, interstitial cystitis, detrusor overactivity (also known as overactive bladder), urinary incontinence, neurogenic detrusor overactivity (also known as detrusor hyperflexia), idiopathic detrusor overactivity (also known as detrusor instability), benign prostatic hyperplasia, lower urinary tract disorders and lower urinary tract symptoms.

Preferably, said psychiatric disorders are selected from anxiety and depression.

A fifth object of the present invention are pharmaceutical compositions comprising the at least one of the above said compounds of formula I in combination with pharmaceutically acceptable excipients and/or diluents.

According to a prefereed embodiments said pharmaceutical composition is for the prevention, reduction of the risk of, amelioration and/or treatment of a disease associated with activity of TRPM8.

According to an embodiment, said pharmaceutical composition contains at least one of the above compounds of formula I as the sole active principle(s). According to an alternative embodiment, said pharmaceutical composition contains at least one of the above compounds of formula I in association with at least one other active principle.

According to a preferred embodiment of the invention, also in combination with the preceding embodiments, the pharmaceutical compositions may be for intravescical, intravenous, topical or oral administration.

The compounds of the invention of formula (I) are conveniently formulated in pharmaceutical compositions using conventional techniques and excipients such as those described in "Remington's Pharmaceutical Sciences Handbook" MACK Publishing, New York, 18th ed., 1990.

A sixth object of the present invention is a therapeutic method for the prevention, reduction of the risk of, amelioration and/or treatment of said diseases associated with activity of TRPM8 comprising the administration of the above compound of Formula I in a subject in need thereof.

The compounds of the invention can be administered as the sole active principles or in combination with other therapeutically active compounds.

The administration of the compounds of the invention can be effected by intravesical instillation, by intravenous injection, as a bolus, in dermatological preparations (creams, lotions, sprays and ointments), by inhalation as well as orally in the form of capsules, tablets, syrup, controlled- release formulations and the like.

The average daily dose depends on several factors such as the severity of the disease, the condition, age, sex and weight of the patient. The dose will vary generally from 1 to 1500 mg of compounds of formula (I) per day optionally divided in multiple administrations.

The present invention shall be illustrated by means of the following examples which are not construed to be viewed as limiting the scope of the invention.

## Examples

### Synthesis of preferred compounds

The compounds listed in Table IV have been synthetised following the procedures described in the following examples.

*Materials and methods*

All reagents were purchased from Sigma-Aldrich, Fluorochem and Alfa Aesar and used without further purification. Nuclear magnetic resonance (NMR) spectra were recorded in the indicated solvent with tetramethylsilane (TMS) as internal standard on a Bruker Avance3 400 MHz instrument. Chemical shifts are reported in parts per million (ppm) relative to the internal standard. Abbreviations are used as follows: s = singlet, d = doublet, t = triplet, q = quartet, m = multiplet, dd = doublets of doublet, br = broad. Coupling constants (J values) are given in hertz (Hz). Analytical HPLC-MS spectra were recorded on a Thermo Finnigan Surveyor coupled with a Thermo Finnigan LCQ DECA XP-PLUS apparatus and equipped with a C18 (10 μM, 4.6mm X 150mm) Phenomenex Gemini reverse phase column. The eluent mixture consisted of 10 mM (pH 4.2) ammonium formate/formic acid buffer and acetonitrile used according the gradient from 90:10 to 10:90 at a flow rate of 0.200 mL / min. All MS experiments were performed using electrospray ionization (ESI) in positive ion mode.

All reactions were monitored by thin layer chromatography (TLC) carried out on Grace Resolv Davisil silica gel plates 250 μm thick, 60 F254, visualized by using UV (254 nm) or stains such as $KMnO_4$, p-anisaldehyde, and ceric ammonium molybdate (CAM). Chromatographic purifications were carried out on silica gel columns with Grace Resolv Davisil silica 60. All organic solutions were dried over anhydrous $Na_2SO_4$ or $MgSO_4$ and concentrated on a rotary evaporator. All compounds used for biological assays are at least of 98% purity based on HPLC analytical results monitored with 220 and 254 nm wavelengths, unless otherwise noted.

**<u>General procedure A</u>**

**Example 1**

**Synthesis of 2-(4-chlorophenyl)-4-hydroxy-1,3-thiazole-5-carboxylic acid (1)**

Ethyl-2-(4-chlorophenyl)-4-hydroxy-1,3-thiazole-5-carboxylate **8** (0.5 g, 1.8 mmol) (prepared according the general Procedure B, see below) was dissolved in dioxane (3 mL) and aqueous hydrochloric acid (37%) (0.3 mL) was added. The mixture was irradiated by microwave (250W, 150°C) for 10 min, whereupon the solvent was removed under vacuum. The crude product was purified by HPLC to yield the acid (0.34 g, 74%) as a white solid.

$^1$H-NMR ($CD_3OD$) δ (ppm): 8.01 (d, 2H, *J*=8.6), 7.50 (d, 2H, *J*=8.6).

**Example 2**

**Synthesis of 4-hydroxy-2-(4-methylphenyl)-1,3-thiazole-5-carboxylic acid (2)**

Following the procedure **A** and starting from ethyl 4-hydroxy-2-(4-methylphenyl)-1,3-thiazole-5-carboxylate **9** (0.25 g, 0.94 mmol) (prepared according the general Procedure B, see below), compound **2** was obtained as a white solid following HPLC purification (154 mg, 70%). $^1$H-NMR (Acetone-$d_6$) δ (ppm): 7.94 (d, 2H, *J*=7.0), 7.33 (d, 2H, *J*=7.0), 2.42 (s, 3H).

**Example 3**

**Synthesis of 2-(3-fluorophenyl)-4-hydroxy-1,3-thiazole-5-carboxylic acid (3)**

Following the general procedure **A** and starting from ethyl 2-(3-fluorophenyl)-4-hydroxy-1,3-thiazole-5-carboxylate **10** (0.2 g, 0.738 mmol) (prepared according the general Procedure B, see below), compound **3** was obtained as a white solid following HPLC purification (120 mg, 68%). $^1$H-NMR ($CD_3OD$) δ (ppm): 13.29 (br s, 1H), 7.82-7.78 (m, 1H), 7.69-7.64 (m, 1H), 7.71-7.46 (m, 2H).

**Example 4**

**Synthesis of 2-(4-fluorophenyl)-4-hydroxy-1,3-thiazole-5-carboxylic acid (4)**

Following the general procedure **A** and starting from ethyl 2-(4-fluorophenyl)-4-hydroxy-1,3-thiazole-5-carboxylate **11** (123 mg, 0.46 mmol) (prepared according the general Procedure B, see below), compound **4** was obtained as yellow solid following HPLC purification (78 mg, 71%). $^1$H-NMR (DMSO-$d_6$) δ (ppm): 7.93 (d, 2H, *J*=7.2), 7.59 (d, 2H, *J*=7.1), 2.62 (s, 3H).

General procedure B

**Example 5**

**Synthesis of methyl 4-hydroxy-2-phenyl-1,3-thiazole-5-carboxylate (5)**

Benzenecarbothioamide (0.29 g, 2.09 mmol) and dimethyl 2-chloromalonate (447 μL, 3.5 mmol) were dissolved in dioxane (50 mL). The mixture was heated to 80°C and stirred overnight, whereupon the solvent was removed under vacuum. **5** was obtained as a yellow solid after purification of the crude product by trituration in acetonitrile (345 mg, 70%). $^1$H-NMR (dmso-$d_6$) δ (ppm): 12.3 (br s, 1H), 7.95-7.92 (m, 2H), 7.55-7.53 (m, 3H), 3.75 (s, 3H); MS (ES$^{1+}$) *m/z*: 236.53 (M+1).

**Example 6**

**Synthesis of methyl 2-(2,4-difluorophenyl)-4-hydroxy-1,3-thiazole-5-carboxylate (6)**

Following the general procedure B and starting from commercially available 2,4-difluorobenzenecarbothioamide (80 mg, 0.46 mmol) and dimethyl 2-chloromalonate (0.75 mL, 5.86 mmol), **6** was obtained as a pale yellow solid after purification of the crude product by trituration in acetonitrile (85 mg, 68%). $^1$H-NMR ($CDCl_3$, TMS) δ (ppm): 8.41-8.34 (m, 1H), 7.05-6.93 (m, 2H), 3.94 (s, 3H); MS (ES$^{1+}$) *m/z*: 272.69 (M+1).

**Example 7**

**Synthesis of ethyl 4-hydroxy-2-phenyl-1,3-thiazole-5-carboxylate (7)**

Following the general procedure B and starting from commercially available benzenecarbothioamide (0.2 g, 1.45 mmol) and diethyl chloropropanedioate (0.3 mL, 1.82 mmol), **7** was obtained as a yellow solid after purification of the crude product by trituration in acetonitrile (253 mg, 70%). $^1$H-NMR (dmso-$d_6$) δ (ppm): 12.3 (br s, 1H), 7.95-7.92 (m, 2H), 7.55-7.53 (m, 3H), 4.43 (q, 2H, *J*=7.03), 1.42 (t, 3H, *J*=7.03); MS (ES$^{1+}$) *m/z:* 250.53 (M+1); 222.42 (M-28).

**Example 8**

**Synthesis of ethyl 2-(4-chlorophenyl)-4-hydroxy-1,3-thiazole-5-carboxylate (8)**

Following the general procedure B and starting from commercially available 4-chlorobenzenecarbothioamide (2.04 g, 11.93 mmol) and the corresponding amount of diethyl chloropropanedioate, **8** was obtained as a yellow solid (2.42 g, 71 %) by trituration in acetonitrile. $^1$H-NMR ($CDCl_3$, TMS) δ (ppm) 9.96 (br s, 1H), 7.94 (d, 2H, *J*=8.6), 7.45 (d, 2H, *J*=8.6), 4.43 (q, 2H, *J*=7.0), 1.42 (t, 3H, *J*=7.0); MS (ES$^{1+}$) *m/z:* 298.36 (M-28+41), 285.42 (M+1), 257.64 (M-28).

**Example 9**

**Synthesis of ethyl 4-hydroxy-2-(4-methylphenyl)-1,3-thiazole-5-carboxylate (9)**

Following the general procedure B and starting from commercially available 4-methylbenzenecarbothioamide (123 mg, 0.81 mmol) and the corresponding amount of diethyl chloropropanedioate, **9** was obtained as a yellow solid after purification of the crude product by trituration in acetonitrile (146 mg, 68%). $^1$H-NMR ($CDCl_3$, TMS) δ (ppm): 9.94 (br s, 1H), 7.88 (d, 2H, *J*=8.1), 7.26 (d, 2H, *J*=8.1), 4.62 (q, 2H, *J*=7.0), 2.41 (s, 3H), 1.39 (t, 3H, *J*=7.0); MS (ES$^{1+}$) *m/z*: 264.30 (M+1).

**Example 10**

**Synthesis of ethyl 2-(3-fluorophenyl)-4-hydroxy-1,3-thiazole-5-carboxylate (10)**

Following the general procedure B and starting from commercially available 3-fluorobenzenecarbothioamide (223 mg, 1.44 mmol) and the corresponding amount of diethyl chloropropanedioate, **10** was obtained as a white solid after purification of the crude product by trituration in acetonitrile (250 mg, 65%). $^1$H-NMR ($CDCl_3$, TMS) δ (ppm): 9.93 (br s), 7.76-7.69 (m, 2H), 7.46-7.39 (m, 1H), 7.22-7.17 (m, 1H), 4.40 (q, 2H, *J*=7.5),1.40 (t, 3H, *J*=7.5); MS (ES$^{1+}$) *m/z:* 240.13 (M-27), 282.66 (M-27+41).

**Example 11**

**Synthesis of ethyl 2-(4-fluorophenyl)-4-hydroxy-1,3-thiazole-5-carboxylate (11)** Following the general procedure B and starting from commercially available 4-fluorobenzenecarbothioamide (243 mg, 1.57 mmol) and the corresponding amount of diethyl chloropropanedioate, **11** was obtained as a white solid after purification of the crude product by trituration in acetonitrile (280 mg, 67%). $^1$H-NMR ($CDCl_3$, TMS) 6 (ppm): 9.94 (s, 1H), 8.01-7.96 (m, 2H), 7.17-7.12 (m, 2H), 4.39 (q, 2H, *J*=7.0), 1.40 (t, 3H, *J*=7.0); MS ($ES^{1+}$) *m*/*z*: 240.23 (M-27).

**Example 12**

**Synthesis of ethyl 4-hydroxy-2-(pyridin-4-yl)-1,3-thiazole-5-carboxylate (12)**

Following the general procedure B and starting from commercially available pyridine-4-carbothioamide (217 mg, 1.57 mmol) and the corresponding amount of diethyl chloropropanedioate, **12** was obtained as a yellow solid after purification of the crude product by trituration in acetonitrile (275 mg, 70%). $^1$H-NMR (MeOD-$d_4$) δ (ppm): 9.91 (br s, 1H), 8.70 (d, 2H, *J*=5.9), 7.81 (d, 2H, *J*=5.9), 4.36 (q, 2H, *J*=7.0), 1.35 (t, 3H, *J*=7.0); MS ($ES^{1+}$) *m*/*z*: 251.81 (M+1).

**Example 13**

**Synthesis of ethyl 2-(4-(dimethylamino)phenyl)-4-hydroxythiazole-5-carboxylate (13)**

Following the general procedure B and starting from commercially available 4-(dimethylamino)benzenecarbothioamide (88 mg, 0.48 mmol) and the corresponding amount of diethyl chloropropanedioate, **13** was obtained as a white solid after purification of the crude product by trituration in acetonitrile (117 mg, 82%). $^1$H-NMR (DMSO-$d_6$) δ (ppm): 11.82 (br s, 1H), 7.76 (d, 2H, *J*=8.6), 6.77 (d, 2H, *J*=9.2), 4.28 (q, 2H, *J*=7.03), 3.02 (s, 6H), 1.26 (t, 3H, *J*=7.03); MS ($ES^{1+}$) *m*/*z*: 293.88 (M+1); 265.83 (M-28); 306.83 (M-28+41).

**Example 14**

**Synthesis of ethyl 2-(3-chlorophenyl)-4-hydroxy-1,3-thiazole-5-carboxylate (14)**

Following the general procedure B and starting from commercially available 3-chlorobenzenecarbothioamide (1.47 g, 8.54 mmol) and the corresponding amount of diethyl chloropropanedioate, **14** was obtained as a white solid after purification of the crude product by trituration in acetonitrile (1.7 g, 71%). $^1$H-NMR ($CDCl_3$, TMS) δ (ppm): 9.98 (br s, 1H), 8.01 (s, 1H), 7.87 (d, 1H, *J*=7.57), 7.49-7.33 (m, 2H), 4.43 (q, 2H, *J*=7.03), 1.42 (t, 3H, *J*=7.03); MS ($ES^{1+}$) *m*/*z*: 297.79 (M-28+41); 284.81 (M+1); 256.76 (M-28).

**Example 15**

**Synthesis of ethyl 4-hydroxy-2-[2'-(trifluoromethyl)biphenyl-3-yl]-1,3-thiazole-5-carboxylate (15)**

3-Bromobenzenecarbothioamide (1.00 g, 4.62 mmol) and diethyl chloropropanedioate (1.0 mL, 6.0 mmol) were dissolved in dioxane (35 mL). The mixture was heated at 80°C and stirred overnight, whereupon the solvent was removed under vacuum. Ethyl 2-(3-bromophenyl)-4-hydroxy-1,3-thiazole-5-carboxylate was obtained as a yellow solid (1.09 g, 72%) by trituration in acetonitrile. An oven-dried Schlenk tube equipped with a magnetic stir bar was charged with 1.5 mL of an aqueous solution of $K_2CO_3$ (2M, 3.0 mmol), tetrakis(triphenylphosphine)palladium(0) (140 mg, 0.121 mmol) and toluene (3 mL). The tube was capped with a rubber septum and immersed in an oil bath at 80 °C for 30 min. Ethyl 2-(3'-bromophenyl)-4-hydroxy-1,3-thiazole-5-carboxylate (188 mg, 0.575 mmol) and 2-trifluoromethyl-phenylboronic acid (218 mg, 1.15 mmol) were then added, and the reaction mixture stirred at 80°C. Upon complete consumption of the starting material (12 h), as judged by thin-layer chromatography analysis, the reaction mixture was filtered on a celite pad. The filtrate was diluted with ethyl acetate, and extracted with water. The organic layers were further washed with brine and dried over sodium sulfate. The product was isolated by column chromatography (hexanes/EtOAc) as a yellow solid (56 mg, 25%). $^1$H-NMR (DMSO-$d_6$) δ (ppm): 10.82 (br s, 1H), 7.96 (d, 2H, *J*=8.11 7.86 (d, 2H, *J*=7.57), 7.78-7.73 (m, 1H), 7.67-7.62 (m, 1H), 7.47-7.41 (m, 2H), 4.16 (q, 2H, *J*=7.03), 1.24 (t, 3H, *J*=7.03).

**Example 16**

**Synthesis of ethyl 2-(2'-fluorobiphenyl-3-yl)-4-hydroxy-1,3-thiazole-5-carboxylate (16)**

The compound was prepared according to the experimental procedure described for compound **15** and starting from ethyl 2-(3'-bromophenyl)-4-hydroxy-1,3-thiazole-5-carboxylate (0.14 g, 0.43 mmol) and 2 fluorophenylboronic acid (0.12 g, 0.86 mmol). Compound **16** was obtained as a yellow oil after HPLC purification (106 mg, 72%). $^1$H-NMR ($CDCl_3$, TMS) δ (ppm): 10.98 (br s, 1H), 8.00 (d, 2H, *J*=7.58), 7.71 (d, 2H, *J*=7.58), 7.57-7.47 (m, 2H), 7.39-7.35 (m, 1H), 7.28-7.17 (m, 1H), 4.41 (q, 2H, *J*=6.49), 1.41 (t, 3H, *J*=6.49).

**Example 17**

**Synthesis of ethyl 4-hydroxy-2-[2'-(trifluoromethyl)biphenyl-4-yl]-1,3-thiazole-5-carboxylate (17)**

The compound was prepared according to the experimental procedure described for compound **15** and starting from ethyl 2-(4-bromophenyl)-4-hydroxy-1,3-thiazole-5-carboxylate (0.12 g, 0.36 mmol) and 2-trifluoromethylphenylboronic acid (136 mg, 0.72 mmol). Compound **17** was obtained as a yellow solid after purification of the crude product by trituration with acetonitrile (106 mg, 75%). $^1$H-NMR (DMSO-*$d_6$)* δ (ppm): 10.23 (br s, 1H), 7.96 (d, 2H, *J*=8.11), 7.86 (d, 2H, *J*=7.57), 7.78-7.73 (m, 1H), 7.67-7.62 (m, 1H), 7.47-7.41 (m, 2H), 4.16 (q, 2H, *J*=7.03), 1.24 (t, 3H, *J*=7.03).

**Example 18**

**Synthesis of ethyl 2-(2'-fluorobiphenyl-4-yl)-4-hydroxy-1,3-thiazole-5-carboxylate (18)**

The compound was prepared according to the experimental procedure described for compound **15** and starting from ethyl 2-(4-bromophenyl)-4-hydroxy-1,3-thiazole-5-carboxylate (0.12 mg, 0.36 mmol) and 2-fluorophenylboronic acid (0.1 mg, 0.72 mmol). Compound **18** was obtained as a white solid after purification of the crude product by trituration with acetonitrile (105 mg, 85%). $^1$H-NMR (DMSO-*$d_6$*) δ (ppm): 12.27 (br s, 1H), 8.06 (d, 2H, *J*=7.57), 7.74 (d, 2H, *J*=7.57), 7.66-7.60 (m, 1H), 7.52-7.46 (m, 1H), 7.40-7.33 (m, 2H), 4.25 (q, 2H, *J*=7.03), 1.28 (t, 3H, *J*=7.03).

**Example 19**

**Synthesis of ethyl 2-{4-[(2-fluorobenzyl)oxy]phenyl}-4-hydroxy-1,3-thiazole-5-carboxylate (19)**

Following the general procedure B and starting from 4-(2'-fluorobenzyloxy)phenyl)-benzenecarbothioamide (0.4 g, 1.53 mmol) and diethyl chloropropanedioate (0.45 g, 2.29 mmol), compound **19** was obtained as a white solid after purification of the crude product by trituration in acetonitrile (446 mg, 78%). $^1$H-NMR (DMSO-*$d_6$*) δ (ppm): 12.10 (br s, 1H), 7.94 (d, 2H, *J*=8.70), 7.63-7.55 (m, 1H), 7.51-7.43 (m, 1H), 7.33-7.15 (m, 4H), 5.26 (s, 2H), 4.24 (q, 2H, *J*=7.05), 1.29 (t, 3H, *J*=7.05).

**Example 20**

**Synthesis of ethyl 2-{4-[(4-fluorobenzyl)oxy]phenyl}-4-hydroxy-1,3-thiazole-5-carboxylate (20)**

Following the general procedure B and starting from 4-(4'-fluorobenzyloxy)phenyl)-benzenecarbothioamide (0.31g, 1.19 mmol) and diethyl chloropropanedioate (0.35 g, 1.78 mmol), compound **20** was obtained as a white solid after purification of the crude product by trituration in acetonitrile (359 mg, 81%). $^1$H-NMR (DMSO-*$d_6$*) δ (ppm): 12.06 (br s, 1H), 7.90 (d, 2H, *J*=8.65), 7.55-7.49 (m, 2H), 7.27-7.20 (m, 2H), 7.14 (d, 2H, *J*=8.65), 5.18 (s, 2H), 4.22 (q, 2H, *J*=7.03), 1.25 (t, 3H, *J*=7.03).

**Example 21**

**Synthesis of ethyl 2-(4-fluorophenyl)-4-{[(trifluoromethyl)sulfonyl]oxy}-1,3-thiazole-5-carboxylate (21)**

To a solution of ethyl 2-(4-fluorophenyl)-4-hydroxy-1,3-thiazole-5-carboxylate **11** (2.3 g, 8.6 mmol) in dry $CH_2Cl_2$ (50 mL), $Et_3N$ (1.4 mL, 10.1 mmol) was added and the mixture was stirred for 40 min at room temperature. The reaction mixture was then cooled to -10°C and trifluomethanesulfonic anhydride (1.7 mL, 10.1 mmol) was added dropwise, keeping the temperature under -5°C. The reaction mixture was stirred for 12 h at room temperature. Upon complete

consumption of starting compound, the mixture was washed with a satured solution of $NH_4Cl$ (80 mL). The aqueous layer was then extracted with ethyl acetate (2 x 50 mL). The organic layers further washed with brine and dried over dry $Na_2SO_4$. Compound **21** was isolated by chromatography (hexane/EtOAc) as pale yellow solid (3.0 g, 87%). $^1$H-NMR ($CDCl_3$, TMS) $\delta$ (ppm): 7.97-7.92 (m, 2H), 7.21-7.15 (m, 2H), 4.43 (q, 2H, *J*=7.0), 1.41 (t, 3H, *J*=7.0).

**Example 22**

**Synthesis of ethyl 4-methoxy-2-(4-methylphenyl)-1,3-thiazole-5-carboxylate (22)**

Ethyl 4-hydroxy-2-(4-methylphenyl)-1,3-thiazole-5-carboxylate **9**, (0.1 g, 0.379 mmol) was dissolved in DMF (4 mL). $K_2CO_3$ (0.11 g, 0.802 mmol) was added and the mixture heated to 60°C while stirring. After 15 min. iodomethane (59$\mu$L, 0.95 mmol) was added and the mixture was stirred overnight at the same temperature. After cooling at room temperature, ethyl acetate (15 mL) was added and the mixture washed with water (2x15 mL). The organic phase was dried over dry $Na_2SO_4$ and evaporated to dryness. The crude product was purified by HPLC to yield compound **22** as a white solid (0.080 g, 76%). $^1$H-NMR (acetone-d6) $\delta$ (ppm): 7.90 (d, 2H, *J*=7.6), 7.35 (d, 2H, *J*=7.6), 4.30-4.19 (m, 2H), 3.89 (s, 3H), 2.40 (s, 3H), 1.36-1.24 (m, 3H); MS ($ES^{1+}$) *m*/*z*: 278.55 (M+1).

**Example 23**

**Synthesis of ethyl 2-(4-methylphenyl)-4-(2-methylpropoxy)-1,3-thiazole-5-carboxylate (23)**

Compound **23** was prepared according to the experimental procedure described for **22** and starting from ethyl 4-hydroxy-2-(4-methylphenyl)-1,3-thiazole-5-carboxylate **9** (85 mg, 0.32 mmol) and 1-iodo-2-methylpropane (147 mg, 0.80 mmol). Compound **23** was obtained as a white solid after HPLC purification (89 mg, 87%). $^1$H-NMR (CDCl3, TMS) $\delta$ (ppm): 7.87 (d, 2H, *J*= 8.1), 7.29 (d, 2H, *J*= 8.1), 4.35 (m, 4H), 2.42 (s, 3H), 2.20 (m,1H), 1.80 (d, 6H, *J*= 6.5), 1.38 (t, 3H, *J*= 7.0); MS ($ES^{1+}$) *m*/*z*: 320.96 (M+1), 264.79 (M-57), 236.77 (M-57-28).

**General procedure C**

**Example 24**

**Synthesis of ethyl 4-(benzyloxy)-2-phenyl-1,3-thiazole-5-carboxylate (24)**

Ethyl 4-hydroxy-2-phenyl-1,3-thiazole-5-carboxylate **7**, (0.2 g, 0.80 mmol) was dissolved in DMF (2 mL). $K_2CO_3$ (0.22 g, 1.604 mmol) was added and the mixture was heated at 60°C while stirring. After 15 min 1-(bromomethyl)benzene (164 mg, 0.96 mmol) was added and the mixture was stirred overnight at the same temperature. After cooling to room temperature, ethyl acetate (10 mL) was added and the mixture washed with water (2x15 mL). The organic phase was dried over dry $Na_2SO_4$ and evaporated to dryness. The crude product was purified by HPLC to yield compound **24** as a white solid (241 mg, 89%). $^1$H-NMR ($CDCl_3$, TMS) $\delta$ (ppm): 8.0-7.9 (m, 4H), 7.87 (d, 1H, *J*=7.0), 7.48-7.28 (m, 5H), 5.37 (s, 2H), 4.37 (q, 2H, *J*=7.0), 1.42 (t, 3H, *J*=7.0); MS ($ES^{1+}$) *m*/*z*: 340.19 (M+1).

**Example 25**

**Synthesis of ethyl 4-[(3-chlorobenzyl)oxy]-2-(4-chlorophenyl)-1,3-thiazole-5-carboxylate (25)**

The title compound was prepared according to the general procedure C. starting from ethyl 2-(4-chlorophenyl)-4-hydroxy-1,3-thiazole-5-carboxylate **8** (111 mg, 0.39 mmol) and 1-(bromomethyl)-3-chlorobenzene (96 mg, 0.47 mmol). Compound **25** was obtained as pale yellow solid after HPLC purification (138 mg, 87%). $^1$H-NMR ($CDCl_3$, TMS) $\delta$ (ppm): 7.79 (d, 2H, *J*=7.1), 7.71 (d, 1H, *J*=7.0), 7.42-7.27 (m, 5H), 5.73 (s, 2H), 4.29 (q, 2H), 1.38 (t, 3H); MS ($ES^{1+}$) *m*/*z*: 409.03 (M+1).

**Example 26**

**Synthesis of ethyl 4-[(3-chlorobenzyl)oxy]-2-(3-fluorophenyl)-1,3-thiazole-5-carboxylate (26)**

The title compound was prepared according to the general procedure C and starting from ethyl 2-(3-fluorophenyl)-4-hydroxy-1,3-thiazole-5-carboxylate **10** (0.1 g, 0.37 mmol) and 1-(bromomethyl)-3-chlorobenzene (91 mg, 0.44 mmol). Compound **26** was obtained as white solid after HPLC purification (113mg, 78%). $^1$H-NMR ($CDCl_3$, TMS) $\delta$ (ppm):

7.97 (m, 1H), 7.84-7.71 (m, 3H), 7.58-7.27 (m, 4H), 5.69 (s, 2H), 4.38 (q, 2H), 1.41 (t, 3H); MS ($ES^{1+}$) *m/z:* 392.71 (M+1).

**Example 27**

**Synthesis of ethyl 4-(4-chlorobenzyloxy)-2-phenyl-1,3-thiazole-5-carboxylate (27)**

Following the general procedure C and starting from ethyl 4-hydroxy-2-phenyl-1,3-thiazole-5-carboxylate **7**, (58 mg, 0.23 mmol), 1-chloro-4-(chloromethyl)benzene (92.6 mg, 0.57 mmol), compound **27** was obtained as a white solid after HPLC purification (75 mg, 86%). $^1$H-NMR ($CDCl_3$, TMS) δ (ppm): 7.94-7.92 (m, 2H), 7.50-7.45 (m, 5H), 7.35-7.33 (m, 2H), 5.60 (s, 2H), 4.33 (q, 2H, *J*=7.0), 1.36 (t, 2H, *J*=7.0); MS ($ES^{1+}$) *m/z:* 374.89 **(M+1).**

**Example 28**

**Synthesis of ethyl 4-(4-chlorobenzyloxy)-2-(3-chlorophenyl)-1,3-thiazole-5-carboxylate (28)**

Following the general procedure C and starting from ethyl 2-(3-chlorophenyl)-4-hydroxy-1,3-thiazole-5-carboxylate **14** (0.3 g, 1.06 mmol) and 1-chloro-4-(chloromethyl)benzene (426.7 mg, 2.65 mmol), compound **28** was obtained as white solid after HPLC purification of the crude product (302 mg, 70%). $^1$H-NMR ($CDCl_3$, TMS) δ (ppm): 7.96 (s, 1H), 7.79 (d, 1H, *J*=7.5), 7.51-7.36 (m, 6H), 5.62 (s, 2H), 4.35 (q, 2H, *J*=5.6), 1.39 (t, 3H, *J*=5.6); MS ($ES^{1+}$) *m/z:* 409.03 (M+1).

**Example 29**

**Synthesis of ethyl 4-[(4-chlorobenzyl)oxy]-2-(4-methylphenyl)-1,3-thiazole-5-carboxylate (29)**

Following the general procedure C and starting from ethyl 4-hydroxy-2-(4-methylphenyl)-1,3-thiazole-5-carboxylate **9**, (0.1 g, 0.401 mmol) and 1-chloro-4-(chloromethyl)benzene (0.161 g, 1.00 mmol), compound **29** was isolated as a white solid (0.112 g, 72%). $^1$H-NMR ($CDCl_3$, TMS) δ (ppm): 7.85 (d, 2H, *J*= 8.1), 7.51 (d, 2H, *J*= 8.6), 7.36 (d, 2H, *J*= 8.1), 7.27 (d, 2H, *J*= 7.6), 5.62 (s, 2H), 4.35 (q, 2H, *J*= 7.03), 2.42 (s, 3H), 1.38 (t, 3H, *J*= 7.03); MS ($ES^{1+}$) *m/z:* 389.02 (M+1).

**Example 30**

**Synthesis of ethyl 4-[(4-chlorobenzyl)oxy]-2-(3-fluorophenyl)-1,3-thiazole-5-carboxylate (30)**

The title compound was prepared according to the general procedure C and starting from ethyl 2-(3-fluorophenyl)-4-hydroxy-1,3-thiazole-5-carboxylate **10** (0.105 g, 0.39 mmol) and 1-(bromomethyl)-4-chlorobenzene (96 mg, 0.47 mmol. Compound **30** was obtained as yellow solid after HPLC purification (119 mg, 78%). $^1$H-NMR ($CDCl_3$, TMS) δ (ppm): 7.89 (s, 1H), 7.78 (d, 1H, *J*=7.8), 7.62-7.59 (m, 3H), 7.55-7.41 (m, 3H), 5.62 (s, 2H), 4.35 (q, 2H, *J*=5.6), 1.39 (t, 3H, *J*=5.6); MS ($ES^{1+}$) *m/z:* 392.6 (M+1).

**Example 31**

**Synthesis of ethyl 4-(2-chlorobenzyloxy)-2-phenyl-1,3-thiazole-5-carboxylate (31)**

Following the general procedure C and starting from ethyl 2-phenyl-4-hydroxy-1,3-thiazole-5-carboxylate **7**, (0.12 g, 0.48 mmol) and 1-chloro-2-(chloromethyl)benzene (0.2 g, 1.2 mmol), compound **31** was obtained as white solid after HPLC purification of the crude product (117 mg, 65%). $^1$H-NMR ($CDCl_3$, TMS) δ (ppm): 8.0-7.9 (m, 3H), 7.87 (d, 1H, *J*=7.0), 7.48-7.28 (m, 5H), 5.77 (s, 2H), 4.37 (q, 2H, *J*=7.0), 1.42 (t, 3H, *J*=7.0); MS ($ES^{1+}$) *m/z:* 374.99 (M+1).

**Example 32**

**Synthesis of ethyl 4-(2-chlorobenzyloxy)-2-(4-fluorophenyl)-1,3-thiazole-5-carboxylate (32)**

Following the general procedure C and starting from ethyl 2-(4-fluorophenyl)-4-hydroxy-1,3-thiazole-5-carboxylate **11**, (0.27 g, 1.01 mmol) and 1-chloro-2-(chloromethyl)benzene (0.41 g, 2.52 mmol), compound **32** was obtained as white solid after HPLC purification of the crude product (261 mg, 66%). $^1$H-NMR ($CDCl_3$, TMS) δ (ppm): 7.99-7.95 (m, 2H), 7.78 (d, 1H, *J*=7.5), 7.41 (d, 1H, *J*=9.0), 7.35-7.25 (m, 2H), 7.20-7.12 (m, 2H), 5.74 (s, 2H), 4.37 (q, 2H, *J*=7.3), 1.42 (t, 3H, *J*=7.0); MS ($ES^{1+}$) *m/z*: 392.97 (M+1).

### Example 33

#### Synthesis of ethyl 4-(2-chlorobenzyloxy)-2-(4-chlorophenyl)-1,3-thiazole-5-carboxylate (33)

Following the general procedure Cand starting from ethyl 2-(4-chlorophenyl)-4-hydroxy-1,3-thiazole-5-carboxylate **8** (37 mg, 0.13 mmol) and 1-chloro-2-(chloromethyl)benzene (52 mg, 0.32 mmol), compound **33** was obtained as a white solid after HPLC purification (40 mg, 75%). $^1$H-NMR ($CDCl_3$, TMS) $\delta$ (ppm): 7.91 (d, 2H, *J*=8.6), 7.76 (d, 1H, *J*=7.0), 7.46-7.29 (m, 5H), 5.75 (s, 2H), 4.37 (q, 2H, *J*=5.6), 1.40 (t, 3H, *J*=5.6); MS ($ES^{1+}$) *m/z:* 409.03 (M+1).

### Example 34

#### Synthesis of ethyl 4-(2-chlorobenzyloxy)-2-(3-chlorophenyl)-1,3-thiazole-5-carboxylate (34)

Following the general procedure C and starting from ethyl 2-(3-chlorophenyl)-4-hydroxy-1,3-thiazole-5-carboxylate **14**, (0.18 g, 0.63 mmol) and 1-chloro-2-(chloromethyl)benzene (254 mg, 1.57 mmol), compound **34** was obtained as white solid after HPLC purification of the crude product (167 mg, 65%). $^1$H-NMR ($CDCl_3$, TMS) $\delta$ (ppm): 7.99 (s, 1H), 7.85-7.76 (m, 2H), 7.48-7.28 (m, 5H), 5.76 (s, 2H), 4.38 (q, 2H, *J*=5.6), 1.41 (t, 3H, *J*=5.6); MS ($ES^{1+}$) *m/z:* 409.31 (M+1).

### Example 35

#### Synthesis of ethyl 4-[(2-chlorobenzyl)oxy]-2-(4-methylphenyl)-1,3-thiazole-5-carboxylate (35)

Following the general procedure C and starting from ethyl 2-*p*-tolyl-4-hydroxy-1,3-thiazole-5-carboxylate **9**, (0.18 g, 0.68 mmol) and 1-chloro-2-(chloromethyl)benzene (273 mg, 1.69 mmol), compound **35** was obtained as white solid after HPLC purification of the crude product (181 mg, 74%). $^1$H-NMR ($CDCl_3$, TMS) $\delta$ (ppm): 7.87 (d, 2H, *J*=8.6), 7.78 (d, 1H, *J*=7.0), 7.45-7.32 (m, 5H), 5.75 (s, 2H), 4.37 (q, 2H, *J*=7.3), 2.42 (s, 3H), 1.42 (t, 3H, *J*=7.0); MS ($ES^{1+}$) *m/z*: 389.00 (M+H).

### Example 36

#### Synthesis of ethyl 2-phenyl-4-{[4-(trifluoromethyl)benzoyl]oxy}-1,3-thiazole-5-carboxylate (36)

Ethyl 4-hydroxy-2-phenyl-1,3-thiazole-5-carboxylate **7** (0.1 g, 0.401 mmol) and pyridine (0.036 mL, 0.48 mmol) were dissolved in $CH_2Cl_2$ (5 mL). 4-(Trifluoromethyl)benzoyl chloride (0.154 g, 0.802 mmol) was slowly added, and the mixture was stirred overnight at room temperature. After solvent removal under reduced pressure, the crude product was purified by HPLC yielding the title compound as a white solid (0.126 g, 74%). $^1$H-NMR ($CDCl_3$, TMS) $\delta$ (ppm): 8.37 (d, 2H, *J*=8.1), 7.99-7.96 (m, 2H), 7.81 (d, 2H, *J*=8.1), 7.52-7.45 (m, 3H), 4.26 (q, 2H, *J*=7.6), 1.21 (t, 3H, *J*=7.6); MS ($ES^{1+}$) *m/z:* 422.99 (M+1).

### Example 37

#### Synthesis of ethyl 2-(3-fluorophenyl)-4-{[4-(trifluoromethyl)benzoyl]oxy}-1,3-thiazole-5-carboxylate (37)

The title compound was prepared according to the procedure described for compound **36** and starting from ethyl 2-(3-fluorophenyl)-4-hydroxy-1,3-thiazole-5-carboxylate **10** (83 mg, 0.31 mmol). Compound **37** was obtained as yellow solid after HPLC purification (110 mg, 81%).

$^1$H-NMR ($CDCl_3$, TMS) $\delta$ (ppm): 7.96-7.79 (m, 2H), 7.51-7.36 (m, 6H), 4.35 (q, 2H, *J*=6.8), 1.39 (t, 3H, *J*=6.6); MS ($ES^{1+}$) *m/z*: 440.33 (M+1).

### Example 38

#### Synthesis of ethyl 2-(4-methylphenyl)-4-{[4-(trifluoromethyl)benzoyl]oxy}-1,3-thiazole-5-carboxylate (38)

The title compound was prepared according to the procedure described for compound **36** and staring from ethyl 4-hydroxy-2-(4-methylphenyl)-1,3-thiazole-5-carboxylate **9** (72 mg, 0.27 mmol). Compound **38** was obtained as red solid after HPLC purification (99 mg, 83%). $^1$H-NMR ($CDCl_3$, TMS) $\delta$ (ppm): 7.83 (d, 2H, *J*= 8.1), 7.50 (d, 2H, *J*= 8.1), 7.36 (d, 2H, *J*= 8.1), 7.30 (d, 2H, *J*= 7.6), 4.31 (q, 2H, *J*= 7.03), 2.39 (s, 3H), 1.38 (t, 3H, *J*= 7.03); MS ($ES^{1+}$) *m/z*: 436.4 (M+1).

**Example 39**

**Synthesis of ethyl 4-(2-((1*R*,2*S*,5*R*)-2-isopropyl-5-methylcyclohexyloxy)acetoyloxy)-2-(4-methylphenyl)-1,3-thiazole-5-carboxylate (39)**

Following the procedure adopted for the preparation of compound **36** and starting from ethyl 4-hydroxy-2-*p*-tolyl-1,3-thiazole-5-carboxylate **9** (0.15 g, 0.57 mmol) and 2-((1*R*,2*S*,5*R*)-2-isopropyl-5-methylcyclohexyloxy)acetyl chloride (265 mg, 1.14 mmol), compound **39** was obtained as white solid after purification by HPLC of the crude product (196 mg, 75%). $^1$H-NMR ($CDCl_3$, TMS) $\delta$ (ppm): 7.85 (d, 2H, *J*= 8.1), 7.27 (d, 2H, *J*= 7.0), 4.53 (s, 2H), 4.35 (q, 2H, *J*= 7.0), 3.38 (m, 1H), 2.43 (s, 3H), 2.20-1.97 (m,1H), 1.90-1.81 (m, 2H), 1.75 (m, 3H), 1.67-1.57 (m, 2H), 1.43 (m, 1H) 1.40 (m, 3H), 1.38 (t, 3H, *J*=7.0), 1.09-1.07 (m, 6H); MS ($ES^{1+}$) *m*/*z*: 461.28 (M+1), 433.22 (M-28), 264.79 (M-196).

**Example 40**

**Synthesis of ethyl 4-[(benzylcarbamoyl)oxy]-2-(4-chlorophenyl)-1,3-thiazole-5-carboxylate (40)**

1-(isocyanatomethyl)benzene (28.7 mg, 0.21 mmol) was added to a solution of ethyl 2-(4-chlorophenyl)-4-hydroxy-1,3-thiazole-5-carboxylate **8** (50 mg, 0.18 mmol) in toluene. The resulting mixture was stirred at 80°C for 12 h and then concentrated under reduced pressure. The crude was triturated in ethyl acetate to give compound **40** as white solid (57 mg, 65%). $^1$H-NMR ($CDCl_3$, TMS) $\delta$ (ppm): 8.00-7.90 (m, 2H), 7.54-7.30 (m, 6H), 5.65 (br s, 1H), 4.53 (m, 2H), 4.36 (m, 2H), 1.38 (t, 3H, *J*= 7.03 Hz); MS ($ES^{1+}$) *m*/*z*: 418.09 (M+1), 325.91 (M-92); 284.75 (M-134).

**Example 41**

**Synthesis of ethyl 4-(2-aminoethoxy)-2-(4-chlorophenyl)-1,3-thiazole-5-carboxylate (41)**

**Following** the procedure adopted for the preparation of compound **36** and starting from ethyl 2-(4-chlorophenyl)-4-hydroxy-1,3-thiazole-5-carboxylate **8**, (0.2 g, 0.704 mmol) and 2-bromoethanamine (218 mg, 1.76 mmol), compound **41** was obtained as a brownish solid (178 mg, 77%). $^1$H-NMR (MeOD-$d_4$) $\delta$ (ppm): 7.98 (d, 2H, *J*= 8.1 Hz), 7.54 (d, 2H, *J*= 8.1 Hz), 4.82 (m, 2H), 4.38 (q, 2H, *J*= 7.0 Hz), 3.48 (m, 2H), 1.38 (t, 3H, *J*= 7.0 Hz); MS ($ES^{1+}$) *m*/*z*: 327.90 (M+1), 368.96 (M+41), 297.78 (M-43); 284.77 (M-44); 256.73 (M-44-27).

**Example 42**

**Synthesis of ethyl 2-(4-chlorophenyl)-4-{2-[(furan-2-ylmethyl)amino]ethoxy}-1,3-thiazole-5-carboxylate (42)**

Ethyl 4-(2-aminoethoxy)-2-(4-chlorophenyl)-1,3-thiazole-5-carboxylate **41** (0.15 g, 0.46 mmol) and furan-2-carbaldehyde (48 mg, 0.51 mmol) were mixed in dry MeOH (15 mL) at room temperature under nitrogen atmosphere. The mixture was stirred at room temperature for 1 h, until the aldimine formation was completed (determined by analytic HPLC). The aldimine solution in MeOH was carefully treated with solid $NaBH_4$ (0.6 g, 16 mmol). The reaction mixture was stirred for furher 2 h and quenched with a saturated aqueous solution of $NH_4Cl$. The pH of the aqueous layer was adjusted to 7 with saturated aqueous $NaHCO_3$. The reaction mixture was then diluted with ethyl acetate (20 mL) and extracted with diethyl ether. The organic extracts were washed with saturated aqueous NaCl and dried ($MgSO_4$). The solvent was evaporated to give compound **42** as a white solid (175 mg, 98%). $^1$H-NMR ($CDCl_3$, TMS) $\delta$ (ppm): 7.84 (d, 2H, *J*=8.6), 7.39 (d, 2H, *J*=8.6), 7.35 (s, 1H), 6.29-6.31 (m, 1H), 6.21-6.22 (m, 1H), 4.66 (t, 2H, *J*=5.4), 4.31 (q, 2H, *J*=7.0), 3.89 (s, 2H), 3.07 (t, 2H, *J*=5.4), 1.34 (t, 3H, *J*=7.0); MS ($ES^{1+}$) *m/z:* 407.96 (M+1).

**General procedure D**

**Example 43**

**Synthesis of 4-[(4-chlorobenzyl)oxy]-2-(4-methylphenyl)-1,3-thiazole-5-carboxylic acid (43)**

Ethyl 4-[(4-chlorobenzyl)oxy]-2-(4-methylphenyl)-1,3-thiazole-5-carboxylate **29** (0.50 g, 1.3 mmol) was dissolved in dioxane (3 mL) and 1M NaOH (1.3 mL, 1.0 eq.) was added. The mixture was stirred at room temperature overnight. Upon complete consumption of starting material, as judged by thin-layer chromatography analysis, $H_2O$ (5 mL) was added to the reaction mixture. After extraction by $CH_2Cl_2$ (3 x 5 mL), the aqueous phase was acidified with diluted HCl to pH 3-4, and extracted with EtOAc (3 x 5 mL). The organic layers were further washed with brine and dried

over dry $Na_2SO_4$. The solvent was removed under vacuum to yield the acid **43** (0.43 g, 92%) as a white solid. 1H-NMR ($CD_3OD$) δ (ppm): 7.87 (d, 2H, *J*=7.0), 7.55 (d, 2H, *J*=7.6), 7.38 (d, 2H, *J*=7.0), 7.32 (d, 2H, *J*=7.6), 5.61 (s, 2H), 2.42 (s, 3H); MS ($ES^{1+}$) *m/z:* 360.90 (M+1).

**Example 44**

**Synthesis of 4-(4-chlorobenzyloxy)-2-phenyl-1,3-thiazole-5-carboxylic acid (44)**

Following the general procedure D and starting from ethyl 4-(4-chlorobenzyloxy)-2-phenyl-1,3-thiazole-5-carboxylate **27** (0.15 g, 0.40 mmol), compound **44** was obtained as a white solid (135 mg, 98%). $^1$H-NMR (DMSO-$d_6$) δ (ppm): 12.95 (br s, 1H), 8.00-7.98 (m, 2H), 7.57-7.53 (m, 5H), 7.49-7.46 (m, 2H), 5.59 (s, 2H); MS ($ES^{1+}$) *m/z*: 346.59 (M+1), 302.66 (M-44).

**Example 45**

**Synthesis of 4-(4-chlorobenzyloxy)-2-(4-chlorophenyl)-1,3-thiazole-5-carboxylic acid (45)**

Following the general procedure D and starting from ethyl 4-(4-chlorobenzyloxy)-2-(4-chlorophenyl)-1,3-thiazole-5-carboxylate (0.11 g, 0.27 mmol), compound **45** was obtained as a white solid (99 mg, 96%). $^1$H-NMR (DMSO-$d_6$) δ (ppm): 13.02 (br s, 1H), 8.06-8.00 (m, 2H), 7.67-7.47 (m, 6H), 5.60 (s, 2H); MS ($ES^{1+}$) *m/z:* absent.

**Example 46**

**Synthesis of 4-(4-chlorobenzyloxy)-2-(3-chlorophenyl)-1,3-thiazole-5-carboxylic acid (46)**

Following the general procedure D and starting from ethyl 4-(4-chlorobenzyloxy)-2-(3-chlorophenyl)-1,3-thiazole-5-carboxylate **28** (0.1 g, 0.24 mmol), compound **46** was obtained as a white solid (87 mg, 94%). $^1$H-NMR (DMSO-$d_6$) δ (ppm): 13.04 (br s, 1H), 8.03 (s, 1H), 7.95 (d, 2H, *J*=7.57), 7.66-7.54 (m, 3H), 7.47 (d, 2H, *J*=7.57), 5.60 (s, 2H); MS ($ES^{1+}$) *m/z:* absent.

**Example 47**

**Synthesis of 4-(benzyloxy)-2-phenyl-1,3-thiazole-5-carboxylic acid (47)**

Following the procedure D and starting from ethyl 4-(benzyloxy)-2-phenyl-1,3-thiazole-5-carboxylate **24** (0.15 g, 048 mmol), compound **47** was obtained as a white solid (134 mg, 90%). $^1$H-NMR ($CDCl_3$, TMS) δ (ppm): 7.99-7-94 (m, 3H), 7.55-7.37 (m, 6H), 6.96 (brs, 1H), 5.65 (s, 2H); MS ($ES^{1+}$) *m/z*: 312.86 (M+1).

**Example 48**

**Synthesis of 4-[(3-chlorobenzyl)oxy]-2-(3-fluorophenyl)-1,3-thiazole-5-carboxylic acid (48)**

The title compound was prepared according to the general procedure D and starting from ethyl 4-[(3-chlorobenzyl)oxy]-2-(3-fluorophenyl)-1,3-thiazole-5-carboxylate **26** (58 mg, 0.14 mmol). Compound **48** was obtained as a whitish solid (46 mg, 91%). $^1$H-NMR (DMSO-$d_6$) δ (ppm): 13.12 (br s, 1H), 8.02 (s, 1H), 7.96-7.93 (m, 1H), 7.68-7.49 (m, 4H), 7.41-7.38 (m, 2H), 5.66 (s, 2H); MS ($ES^{1+}$) *m/z*: 364.7 (M+1).

**Example 49**

**Synthesis of 4-(2-chlorobenzyloxy)-2-phenyl-1,3-thiazole-5-carboxylic acid (49)**

Following the general procedure D and starting from ethyl 4-(2-chlorobenzyloxy)-2-phenyl-1,3-thiazole-5-carboxylate **31** (90 mg, 0.24 mmol), compound **49** was obtained as a white solid (81 mg, 98%). $^1$H-NMR (DMSO-$d_6$) δ (ppm): 12.97 (br s, 1H), 8.00-7.97 (m, 2H), 7.70-7.68 (m, 1H), 7.57-7.51 (m, 4H), 7.41-7.38 (m, 2H), 5.67 (s, 2H); MS ($ES^{1+}$) *m/z:* 346.6 (M+1), 263.5 (M-125+1+41), 222.5 (M-125+1).

### Example 50

#### Synthesis of 4-(2-chlorobenzyloxy)-2-(4-fluorophenyl)-1,3-thiazole-5-carboxylic acid (50)

Following the general procedure D and starting from ethyl 4-(2-chlorobenzyloxy)-2-(4-fluorophenyl)-1,3-thiazole-5-carboxylate **32** (0.11 g, 0.28 mmol), compound **50** was obtained as a white solid (97 mg, 95%). $^1$H-NMR (DMSO-$d_6$) δ (ppm): 12.97 (br s, 1H), 8.07-8.02 (m, 2H), 7.70-7.67 (m, 1H), 7.54-7.51 (m, 1H), 7.41-7.36 (m, 4H), 5.66 (s, 2H); MS (ES$^{1+}$) ***m/z:*** absent.

### Example 51

#### Synthesis of 4-(2-chlorobenzyloxy)-2-(4-chlorophenyl)-1,3-thiazole-5-carboxylic acid (51)

Following the general procedure D and starting from ethyl 4-(2-chlorobenzyloxy)-2-(4-chlorophenyl)-1,3-thiazole-5-carboxylate **33** (50 mg, 0.12 mmol), compound **51** was obtained as a yellow solid (43 mg, 92%). $^1$H-NMR (DMSO-$d_6$) δ (ppm): 13.00 (br s, 1H), 8.01 (d, 2H, *J*=8.1), 7.74-7.66 (m, 1H), 7.62 (d, 2H, *J*=8.1), 7.54-7.51 (m, 1H), 7.41-7.39 (m, 2H), 5.66 (s, 2H); MS (ES$^{1+}$) *m*/*z*: absent.

### Example 52

#### Synthesis of 4-(2-chlorobenzyloxy)-2-(3-chlorophenyl)-1,3-thiazole-5-carboxylic acid (52)

Following the general procedure D and starting from ethyl 4-(2-chlorobenzyloxy)-2-(3-chlorophenyl)-1,3-thiazole-5-carboxylate **34** (105 mg, 0.26 mmol), compound **52** was obtained as a white solid (96 mg, 98%). $^1$H-NMR (DMSO-$d_6$) δ (ppm): 13.02 (br s, 1H), 8.02 (s, 1H), 7.96-7.93 (m, 1H), 7.70-7.50 (m, 4H), 7.42-7.39 (m, 2H), 5.67 (s, 2H); MS (ES$^{1+}$) *m*/*z:* absent.

### Example 53

#### Synthesis of 4-(2-chlorobenzyloxy)-2-(4-methylphenyl)-1,3-thiazole-5-carboxylic acid (53)

Following the general procedure D and starting from ethyl 4-(2-chlorobenzyloxy)-2-(4-methylphenyl)-1,3-thiazole-5-carboxylate **35** (80 mg, 0.21 mmol), compound **53** was obtained as a white solid (72 mg, 97%). $^1$H-NMR (DMSO-$d_6$) δ (ppm): 12.92 (br s, 1H), 7.90-7.87 (m, 2H), 7.72-7.68 (m, 1H), 7.55-7.51 (m, 1H), 7.43-7.34 (m, 4H), 5.67 (s, 2H), 2.38 (s, 3H); MS (ES$^{1+}$) *m*/*z*: 360.73 (M+1).

### Example 54

#### Synthesis of 4-[(2-chlorobenzyl)oxy]-2-(3-fluorophenyl)-1,3-thiazole-5-carboxylic acid (54)

Following the general procedure D and starting from ethyl 4-[(2-chlorobenzyl)oxy]-2-(3-fluorophenyl)-1,3-thiazole-5-carboxylate (89 mg, 0.22 mmol), compound **54** was obtained as a white solid (76 mg, 92%). $^1$H-NMR (DMSO-$d_6$) δ (ppm): 12.11 (br s, 1H), 8.10 (m, 2H), 7.96-7.93 (m, 2H), 7.70-7.50 (m, 3H), 7.42-7.39 (m, 1H), 5.59 (s, 2H); MS (ES$^{1+}$) *m*/*z:* 364.7 (M+1).

### Example 55

#### Synthesis of 2-phenyl-4-{[4-(trifluoromethyl)benzyl]oxy}-1,3-thiazole-5-carboxylic acid (55)

Following the general procedure D and starting from ethyl 2-phenyl-4-{[4-(trifluoromethyl)benzyl]oxy}-1,3-thiazole-5-carboxylate (91 mg, 0.22 mmol), compound **55** was obtained as brown solid (77 mg, 93%). $^1$H-NMR (DMSO-$d_6$) δ (ppm): 12.93 (br s, 1H), 8.05-7.96 (m, 2H), 7.57-7.53 (m, 5H), 7.49-7.46 (m, 2H), 5.59 (s, 2H); MS (ES$^{1+}$) *m*/*z:* 380.49 (M+1).

**Example 56**

**Synthesis of 2-(3-fluorophenyl)-4-{[4-(trifluoromethyl)benzyl]oxy}-1,3-thiazole-5-carboxylic acid (56)**

Following the general procedure D and starting from ethyl 2-(3-fluorophenyl)-4-{[4-(trifluoromethyl)benzyl]oxy}-1,3-thiazole-5-carboxylate (101 mg, 0.23 mmol), compound **56** was obtained as pale yellow solid (82 mg, 90%). $^1$H-NMR (DMSO-$d_6$) δ (ppm): 12.83 (br s, 1H), 8.05-7.96 (d, 2H, *J*=8.0), 7.76 (m, 1H), 7.57-7.53 (m, 3H), 7.49-7.46 (d, 2H, *J*=8.1), 5.59 (s, 2H); MS (ES$^{1+}$) *m/z:* 398.4 (M+1).

**Example 57**

**Synthesis of 2-phenyl-4-{[4-(trifluoromethyl)benzoyl]oxy}-1,3-thiazole-5-carboxylic acid (57)**

Following the general procedure described for compound **36** and starting from 4-hydroxy-2-phenyl-1,3-thiazole-5-carboxylic acid (89 mg, 0.40 mmol) and 4-(trifluoromethyl)benzoyl chloride (158 mg, 0.76 mmol), compound **57** was obtained as a white solid after HPLC purification (124 mg, 79%). $^1$H-NMR ($CDCl_3$, TMS) δ (ppm): 8.36 (d, 2H, *J*=8.1), 7.99-7.96 (m, 2H), 7.81 (d, 2H, *J*=8.1), 7.52-7.45 (m, 3H), MS (ES$^{1+}$) *m*/*z:* 394.11 (M+1).

**Example 58**

**Synthesis of 2-(3-fluorophenyl)-4-{[4-(trifluoromethyl)benzoyl]oxy}-1,3-thiazole-5-carboxylic acid (58)**

Following the procedure described for compound **57** and starting from 2-(3-fluorophenyl)-4-hydroxy-1,3-thiazole-5-carboxylic acid **3** (91 mg, 0.38 mmol), compound **58** was obtained as yellow solid after HPLC purification (136 mg, 87%). $^1$H-NMR ($CDCl_3$, TMS) δ (ppm): 8.29 (d, 2H, *J*=8.1), 7.91 (d, 2H, *J*=8.1), 7.88-7.96 (m, 3H), 7.52-7.45 (m, 1H), MS (ES$^{1+}$) *m*/*z:* 412.3 (M+1).

**Example 59**

**Synthesis of 2-(4-methylphenyl)-4-{[4-(trifluoromethyl)benzoyl]oxy}-1,3-thiazole-5-carboxylic acid (59)**

Following the general procedure described for compound **57** and starting from ethyl 4-hydroxy-2-(4-methylphenyl)-1,3-thiazole-5-carboxylate **2** (0.1 g, 0.42 mmol) and 4-(trifluoromethyl)benzoyl chloride (158 mg, 0.76 mmol), compound **59** was obtained as white solid after purification by HPLC of the crude product (110 mg, 71%). $^1$H-NMR ($CDCl_3$, TMS) δ (ppm): 8.28 (d, 2H, *J*=7.6), 8.03 (d, 2H, *J*=8.1), 7.80 (d, 2H, *J*=8.1), 7.34 (d, 2H, *J*=7.6), 2.47 (s, 3H); MS (ES$^{1+}$) *m*/*z*: absent.

**Example 60**

**Synthesis of 4-methoxy-2-(4-methylphenyl) -1,3-thiazole-5-carboxylic acid (60)**

Following the general procedure D and starting from ethyl 4-methoxy-2-(4-methylphenyl)-1,3-thiazole-5-carboxylate **22** (47 mg, 0.17 mmol), compound **60** was obtained as a white solid (40 mg, 94%). $^1$H-NMR (DMSO-$d_6$): δ (ppm): 12.81 (br s, 1H), 7.88 (d, 2H, *J*=7.8), 7.35 (d, 2H, *J*=7.8), 4.11 (s, 3H), 2.38 (s, 3H); MS (ES$^{1+}$) *m/z:* 250.71 (M+1), 291.84 (M+41), 232.76 (M-18).

**Example 61**

**Synthesis of 2-(4-methylphenyl)-4-(2-methylpropoxy)-1,3-thiazole-5-carboxylic acid (61)**

Following the general procedure D and starting from ethyl 2-(4-methylphenyl)-4-(2-methylpropoxy)-1,3-thiazole-5-carboxylate **23** (0.85 g, 2.67 mmol), compound **61** was obtained as a white solid (731 mg, 94%). $^1$H-NMR ($CDCl_3$, TMS) δ (ppm): 7.86 (d, 2H, *J*=7.6), 7.28 (d, 2H, *J*=7.6), 4.46 (d, 2H, *J*=6.5), 2.43 (s, 3H), 2.15-2.30 (m, 1H), 1.09 (d, 6H, *J*=6.5); MS (ES$^{1+}$) *m/z:* 292.86 (M+1), 277.83 (M-15), 236.76 (M-56).

**General procedure E**

**Example 62**

**Synthesis of ethyl 4-[(tert-butoxycarbonyl)aminol-2-(4-fluorophenyl)-1,3-thiazole-5-carboxylate (62)**

$Pd_2(dba)_3$ (15 mg, 0.015 mmol) and Xantphos (27 mg, 0.046 mmol) were dissolved in dry THF (6 mL) under $N_2$ atmosphere. The mixture was stirred at room temperature for 20 min. 0.100 g (0.240 mmol) of ethyl 2-(4-fluorophenyl)-4-{[(trifluoromethyl)sulfonyl]oxy}-1,3-thiazole-5-carboxylate **21** (0.2 g, 0.5 mmol) was then added, and after 5 minutes, tert-butyl carbamate (70.4 mg, 0.6 mmol) was added. The mixture was irradiated by microwave (250 W, 135°C) for 1 h, whereupon the mixture was filtered on a celite pad and the solvent was removed under vacuum. The crude product was purified by flash column chromatography (eluent hexane/ethyl acetate mixture of increasing polarity) to yield the compound **62** as a yellow solid (157 mg, 86%). $^1$H-NMR ($CDCl_3$, TMS) δ (ppm): 9.26 (br s, 1H), 8.06-8.01 (m, 2H), 7.16-7.10 (m, 2H), 4.36 (q, 2H, *J* = 7.0), 1.56 (s, 9H), 1.39 (t, 3H, *J* = 7.0); MS ($ES^{1+}$) *m/z:* 311 (M-55).

**Example 63**

**Synthesis of ethyl 4-amino-2-(4-fluorophenyl)-1,3-thiazole-5-carboxylate hydrochloride (63)**

Ethyl 4-[(*tert*-butoxycarbonyl)amino]-2-(4-fluorophenyl)- 1,3- thiazole-5-carboxylate **62** (157 mg, 0.43 mmol) was dissolved in a solution of 1.25 M HCl in $CH_3OH$ and stirred for 1h at room temperature. The solvent was removed under vacuum and the compound **63** was obtained as an orange solid (121 mg, 93%). $^1$H-NMR (DMSO-$d_6$): δ 8.01-7.96 (m, 2H), 7.39-7.33 (m, 2H), 7.08 (br s, 2H), 4.27 (q, 2H, *J*=7.0 Hz), 1.27 (t, 3H, *J*=7.0 Hz); MS ($ES^{1+}$) *m/z:* 267.82 (M+1), 308.91 (M+41).

**Example 64**

**Synthesis of ethyl 4-acetamido-2-(4-methylphenyl) -1,3-thiazole-5-carboxylate (64)**

Following the general procedure E and starting from ethyl 2-(4-methylphenyl)-4-{[(trifluoromethyl)sulfonyl]oxy}-1,3-thiazole-5-carboxylate (0.1 g, 0.25 mmol) and acetamide (18 mg, 0.30 mmol), compound **64** was obtained as white solid after purification by HPLC of the crude product (56 mg, 73%). $^1$H-NMR ($CDCl_3$, TMS) δ (ppm): 7.86 (d, 2H, *J*= 8.1), 7.28 (d, 2H, *J*= 8.1), 4.39 (q, 2H, *J*= 7.0), 2.55 (br s, 3H), 2.43 (s, 3H), 1.40 (t, 3H, *J*=7.0); MS ($ES^{1+}$) *m*/*z*: 305.4 (M+1), 263.8 (M-42).

**Example 65**

**Synthesis of ethyl 2-(4-methylphenyl)-4-{[4-(trifluoromethyl)benzoyl]amino}-1,3-thiazole-5-carboxylate (65)**

Following the procedure described for compound **36** and starting from ethyl 4-amino-2-(4-methylphenyl)-1,3-thiazole-5-carboxylate (0.1 g, 0.38 mmol) and 4-(trifluoromethyl)benzoyl chloride (158 mg, 0.76 mmol), compound **65** was obtained as white solid after HPLC purification of the crude product (117 mg, 71%). $^1$H-NMR ($CDCl_3$, TMS) δ (ppm): 11.03 (br s, 1H), 8.19 (d, 2H, *J*= 8.11 Hz), 8.03 (d, 2H, *J*= 8.11 Hz), 7.84 (d, 2H, *J*= 8.11 Hz), 7.28 (d, 2H, *J*= 8.11 Hz), 4.39 (q, 1H, *J*= 7.03 Hz), 2.44 (s, 3H), 1.40 (t, 3H, *J*=7.03 Hz); MS ($ES^{1+}$) *m/z:* 435.10 (M+1).

**Example 66**

**Synthesis of ethyl 2-(4-methylphenyl)-4-[(phenylcarbamoyl)amino]-1,3-thiazole-5-carboxylate (66)**

Following the procedure described for compound **40** and starting from ethyl 4-amino-2-(4-methylphenyl)-1,3-thiazole-5-carboxylate (1.23 g, 4.68 mmol) and phenylisocyanate (557 mg, 4.68 mmol), compound **66** was obtained as a white solid (1.63 g, 88%). $^1$H-NMR ($CDCl_3$, TMS) δ (ppm): 10.97 (br s, 1H), 9.11 (br s, 1 H), 7.86 (d, 2H, *J*=8.1), 7.63 (d, 2H, *J*=8.1), 7.41-7.34 (m, 4H), 7.13 (t, 1H, *J*=7.0), 4.41 (q, 2H, *J*=7.0), 2.47 (s, 3H), 1.42 (t, 3H, *J*=7.0); MS ($ES^{1+}$) *m*/*z:* 382.44 (M+1).

**Example 67**

**Synthesis of ethyl 4-[(2-aminoethyl)amino]-2-(4-methylphenyl)-1,3-thiazole-5-carboxylate (67)**

Following the general procedure E and starting from ethyl 2-(4-methylphenyl)-4-{[(trifluoromethyl)sulfonyl]oxy}-1,3-thiazole-5-carboxylate (150 mg, 0.38 mmol) and ethane-1,2-diamine (27.4 mg, 0.45 mmol), compound **67** was obtained as pale yellow solid after purification by flash column cromatography (eluent hexane/ethyl acetate mixture of increasing polarity) of the crude product (84.7 mg, 73%). $^1$H-NMR ($CDCl_3$, TMS) δ (ppm): 7.83-7.78 (d, 2H, *J*= 8.1), 7.25-7.19 (d, 2H, *J*= 8.1), 4.25 (q, 2H, *J*= 7.0), 3.70 (t, 2H, *J*= 5.9), 2.39 (s, 3H), 1.90 (brs, 2H), 1.38 (t, 3H, *J*=7.0); MS ($ES^{1+}$) *m*/*z:* = 306.2 (M+1).

**Example 68**

**Synthesis of ethyl 2-(4-chlorophenyl)-4-{[2-(methylamino)ethyl]amino}-1,3-thiazole-5-carboxylate (68)**

Following the general procedure E and starting from ethyl 2-(4-chlorophenyl)-4-{[(trifluoromethyl)sulfonyl]oxy}-1,3-thiazole-5-carboxylate (0.1 g, 0.240 mmol) and *N*-methylethane-1,2-diamine, compound **68** was obtained as a yellow powder (60 mg, 74%). $^1$H-NMR ($CDCl_3$, TMS) δ (ppm): 7.90 (d, 2H, *J* = 8.6), 7.41 (d, 2H, *J* = 8.6), 4.30 (q, 2H, *J*=7.3), 3.84 (q, 2H, *J*=5.9), 2.99 (t, 2H, *J*=2.9), 2.57 (s, 3H), 1.36 (t, 3H, *J*=7.0); MS ($ES^{1+}$) *m*/*z:* 381.96 (M+41), 340.92 (M+1), 309.81 (M-30), 294.73 (M-45).

**Example 69**

**Synthesis of ethyl 2-(4-chlorophenyl)-4-{[2-(propylamino)ethyl]amino}-1,3-thiazole-5-carboxylate (69)**

Following the general procedure E and starting from ethyl 2-(4-chlorobenzen)-4-{[(trifluoromethyl)sulfonyl]oxy}-1,3-thiazole-5-carboxylate, (0.12 g, 0.29 mmol) and *N*-ethylethane-1,2-diamine (25.4 mg, 0.35 mmol), compound **69** was obtained as pale yellow solid after purification by flash column cromatography (eluent hexane/ethyl acetate mixture of increasing polarity) of the crude product (75 mg, 70%). $^1$H-NMR ($CDCl_3$, TMS) δ (ppm): 7.90 (d, 2H, *J* = 8.6), 7.41 (d, 2H, *J* = 8.6), 5.08 (brs, 1H), 4.30 (q, 2H, *J*=7.0), 3.93 (q, 2H, *J*=5.9), 3.02 (t, 2H, *J*=5.9), 2.89 (t, 2H, *J*=7.3), 1.78-1.66 (m, 2H), 1.34 (t, 3H, *J*=7.0), 0.96 (t, 3H, *J*=7.0); MS ($ES^{1+}$) *m*/*z:* 368.95 (M+1).

**Example 70**

**Synthesis of ethyl 4-[(2-aminoethyl)amino]-2-(4-chlorophenyl)-1,3-thiazole-5-carboxylate (70)**

The title compound was prepared according to the general procedure E and starting from ethyl 2-(4-chlorophenyl)-4-{[(trifluoromethyl)sulfonyl]oxy}-1,3-thiazole-5-carboxylate (220 mg, 0.53 mmol). Compound **70** was obtained as a pale yellow oil after HPLC purification (124 mg, 72%). MS ($ES^{1+}$) *m*/*z:* 326.78 (M+1).

**Example 71**

**Synthesis of ethyl 4-{[2-(methylamino)ethyl]amino}-2-(4-methylphenyl)-1,3-thiazole-5-carboxylate (71)**

The title compound was prepared according to the general procedure E and starting from ethyl 2-(4-methylphenyl)-4-{[(trifluoromethyl)sulfonyl]oxy}-1,3-thiazole-5-carboxylate (203 mg, 0.51 mmol). Compound **71** was obtained as whitish solid after HPLC purification in 81% yield (132 mg). MS ($ES^{1+}$) *m*/*z:* 320.55 (M+1).

**Example 72**

**Synthesis of ethyl 4-[(2-aminoethyl)amino]-2-[2'-(trifluoromethyl)biphenyl-4-yl]-1,3-thiazole-5-carboxylate (72)**

The title compound was prepared according to the general procedure E and starting from ethyl 2-[2'-(trifluoromethyl)biphenyl-4-yl]-4-{[(trifluoromethyl)sulfonyl]oxy}-1,3-thiazole-5-carboxylate (188 mg, 0.35 mmol). Compound **72** was obtained as a white solid after HPLC purification in 61% yield (95 mg). MS ($ES^{1+}$) *m*/*z:* 436.41 (M+1).

**Example 73**

**Synthesis of ethyl 4-[(2-aminoethyl)amino]-2-[2'-(trifluoromethyl)biphenyl-3-yl]-1,3-thiazole-5-carboxylate (73)**

The title compound was prepared according to the general procedure **E** and starting from ethyl 2-[2'-(trifluoromethyl)biphenyl-3-yl]-4-{[(trifluoromethyl)sulfonyl]oxy}-1,3-thiazole-5-carboxylate (156 mg, 0.29 mmol). Compound **73** was obtained as dark yellow oil after HPLC purification (72 mg, 56%). MS ($ES^{1+}$) *m/z:* 436.37 (M+1).

**Example 74**

**Synthesis of ethyl 2-(4-chlorophenyl)-4-{[2-(cyclopentylamino)ethyl]amino}-1,3-thiazole-5-carboxylate (74)**

Following the general procedure described for compound **42** and starting from ethyl 4-(2-aminoethylamino)-2-(4-chlorophenyl)-1,3-thiazole-5-carboxylate (0.5 g, 1.53 mmol) and cyclopentanecarbaldehyde (166 mg, 1.69 mmol), compound **74** was obtained as a yellow oil (447 mg, 74%). $^1$H-NMR ($CDCl_3$, TMS) $\delta$ (ppm): 7.91 (d, 2H, *J*=8.12), 7.42 (d, 2H, *J*=8.12), 6.99 (br s, 1H), 4.31 (q, 2H, *J*=7.03), 3.84-3.79 (m, 2H), 3.25-3.17 (m, 2H), 3.01-2.97 (m,2H), 2.70 (br s, 1H), 1.93-1.86 (m, 2H), 1.76-1.66 (m, 2H), 1.61-1.31 (m, 2H), 1.37 (t, 3H, *J*=7.03); MS ($ES^{1+}$) *m/z* 394.95 (M+1).

**Example 75**

**Synthesis of ethyl 2-phenyl-4-{[2-(pyrrolidin-1-yl)ethyl]amino}-1,3-thiazole-5-carboxylate (75)**

Following the general procedure **E** and starting from 5-(ethoxycarbonyl)-2-phenylthiazol-4-yl trifluoromethanesulfonate (145 mg, 0.38 mmol) and 2-(pyrrolidin-1-yl)ethanamine (51.4 mg, 0.45 mmol), compound **75** was obtained as a yellow solid after purification by flash column cromatography (eluent hexane/ethyl acetate mixture of increasing polarity) of the crude product (83 mg, 63%). $^1$H-NMR ($CDCl_3$, TMS) $\delta$ (ppm): 7.72-7.64 (m, 2H), 7.45-7.38 (m, 1H), 7.20-7.13 (m, 1H), 6.98 (br s, 1H), 4.31 (q, 2H, *J*=7.03), 3.97-3.90 (m, 2H), 3.16-3.12 (m, 2H), 3.12-3.01 (m, 4H), 2.05-1.96 (m, 4H), 1.35 (t, 3H, *J*=7.03); MS ($ES^{1+}$) *m/z:* 346.41 (M+1).

**Example 76**

**Synthesis of ethyl 4-(benzylamino)-2-(3-fluorophenyl)-1,3-thiazole-5-carboxylate (76)**

Following the general procedure **E** and starting from 5-(ethoxycarbonyl)-2-(3-fluorophenyl)thiazol-4-yl trifluoromethanesulfonate (0.15 g, 0.33 mmol) and phenylmethanamine (43 mg, 0.40 mmol), compound **76** was obtained as a yellow solid after purification by flash column cromatography (eluent hexane/ethyl acetate mixture of increasing polarity) of the crude product (96 mg, 76%). $^1$H-NMR ($CDCl_3$, TMS) $\delta$ (ppm): 7.74-7.64 (m, 2H), 7.43-7.25 (m, 6H), 7.19-7.12 (m, 1H), 4.86 (s, 2H), 4.29 (q, 2H, *J*=7.03), 1.34 (t, 3H, *J*=7.03); MS ($ES^{1+}$) *m/z:* 357.41 (M+1).

**Example 77**

**Synthesis of ethyl 4-[(1,3-benzodioxol-5-ylmethyl)amino]-2-(3-fluorophenyl)-1,3-thiazole-5-carboxylate (77)**

Following the general procedure E and starting from 5-(ethoxycarbonyl)-2-(3-fluorophenyl)thiazol-4-yl trifluoromethanesulfonate (0.15 g, 0.33 mmol) and (benzo[d][1,3]dioxol-5-yl)methanamine (60 mg, 0.40 mmol), compound **77** was obtained as a yellow solid after purification by flash column cromatography (eluent hexane/ethyl acetate mixture of increasing polarity) of the crude product (82 mg, 62%). $^1$H-NMR ($CDCl_3$, TMS) $\delta$ (ppm): 7.75-7.70 (m, 2H), 7.46-7.39 (m, 1H), 7.21-7.11 (m, 2H), 6.91-6.78 (m, 2H), 5.96 (s, 2H), 4.77 (s, 2H), 4.31 (q, 2H, *J*=7.03), 1.46 (s, 1H), 1.38 (t, 3H, *J*=7.03); MS ($ES^{1+}$) *m/z:* 401.61 (M+1).

**Example 78**

**Synthesis of ethyl 2-(3-fluorophenyl)-4-[(pyridin-3-ylmethyl)amino]-1,3-thiazole-5-carboxylate (78)**

Following the general procedure E and starting from 5-(ethoxycarbonyl)-2-(3-fluorophenyl)thiazol-4-yl trifluoromethanesulfonate (0.15 g, 0.33 mmol) and (pyridin-3-yl)methanamine (43 mg, 0.40 mmol), compound **78** was obtained as a yellow solid after purification by flash column cromatography (eluent hexane/ethyl acetate mixture of increasing polarity) of the crude product (81 mg, 69%). $^1$H-NMR ($CDCl_3$, TMS) $\delta$ (ppm): 8.67 (s, 1H), 8.52 (s, 1H), 7.74-7.65 (m,

2H), 7.48-7.36 (m, 1H), 7.32-7.12 (m, 4H), 4.85 (s, 2H), 4.30 (q, 2H, *J*=7.03), 1.36 (t, 3H, *J*=7.03); MS ($ES^{1+}$) *m/z:* 358.53 (M+1).

**Example 79**

**Synthesis of 4-[(2-aminoethyl)amino]-2-(4-methylphenyl)-1,3-thiazole-5-carboxylic acid (79)**

The title compound was prepared following the general procedure D and starting from ethyl 4-[(2-aminoethyl) amino]-2-(4-methylphenyl)-1,3-thiazole-5-carboxylate 67 (93 mg, 0.30 mmol). Compound 79 was obtained as a white solid (68 mg, 81%). $^1$H-NMR ($CDCl_3$, TMS) δ (ppm): 7.83 (d, 2H, J= 8.1), 7.22 (d, 2H, J= 8.1), 3.20 (t, 2H, J= 6.9), 2.80 (t, 2H, J= 6.9), 2.39 (s, 3H). MS ($ES^{1+}$) *m/z:* 277.8 (M+1).

Example 80

Synthesis of 4-{[2-(methylamino)ethyl]amino}-2-(4-methylphenyl)-1,3-thiazole-5-carboxylic acid (80)

Following the general procedure D and starting from ethyl 4-{[2-(methylamino)ethyl]amino}-2-(4-methylphenyl)-1,3-thiazole-5-carboxylate 71 (89 mg, 0.27 mmol), compound 80 was obtained as white solid (74 mg, 91%). MS ($ES^{1+}$) m/z: 292.8 (M+1).

Example 81

**Synthesis of 4-[(2-aminoethyl)amino]-2-(3-fluorophenyl)-1,3-thiazole-5-carboxylic acid (81)**

Following the general procedure D and starting from ethyl 4-[(2-aminoethyl)amino]-2-(3-fluorophenyl)-1,3-thiazole-5-carboxylate (100 mg, 0.32 mmol), compound **81** was obtained as white solid (80 mg, 88%). MS ($ES^{1+}$) *m/z:* 282.4 (M+1).

**General procedure F**

**Example 82**

**Synthesis of sodium 4-[(3-chlorobenzyl)oxy]-2-(3-fluorophenyl)-1,3-thiazole-5-carboxylate (82)**

**1** eq of NaOH was added to a 30 mM solution of 5-[(3-chlorobenzyl)oxy]-2-(3-fluorophenyl)-1,3-thiazole-4-carboxylic acid **48** and the mixture stirred for 30 min at room temperature. After evaporation under reduced pressure the compound **82** was isolated in form of sodium salt (19 mg, 95%). $^1$H-NMR (DMSO-$d_6$) δ (ppm): 7.89-7.82 (m, 2H), 7.70-7.61 (m, 2H), 7.57-7.53 (m, 4H), 7.42 (m, 2H), 5.42 (s, 2H).

**Example 83**

**Synthesis of sodium 4-[(4-chlorobenzyl)oxy]-2-(4-methylphenyl)-1,3-thiazole-5-carboxylate (83)**

Following the procedure F and starting from 4-[(4-chlorobenzyl)oxy]-2-(4-methylphenyl)-1,3-thiazole-5-carboxylic acid **43,** compound **83** was obtained as a white solid (54 mg, 95%). $^1$H-NMR ($CD_3OD$) δ (ppm): 7.83 (d, 2H, *J*=7.0), 7.52 (d, 2H, *J*=7.6), 7.41(d, 2H, *J*=7.0), 7.33 (d, 2H, *J*=7.6), 5.59 (s, 2H), 2.31 (s, 3H).

**Example 84**

**Synthesis of sodium 4-(4-chlorobenzyloxy)-2-(4-chlorophenyl)-1,3-thiazole-5-carboxylate (84)**

Following the general procedure F and starting from 4-(4-chlorobenzyloxy)-2-(4-chlorophenyl)-1,3-thiazole-5-carboxylic acid **45** (0.2 g, 0.526 mmol), compound **84** was obtained as a yellow solid (212 mg, 95%). $^1$H-NMR (DMSO-*$d_6$*) δ (ppm): 7.89 (d, 2H, *J*=8.65), 7.57-7.53 (m, 4H), 7.42 (d, 2H, *J*=8.65), 5.47 (s, 2H).

**Example 85**

**Synthesis of sodium 4-(2-chlorobenzyloxy)-2-(4-chlorophenyl)-1,3-thiazole-5-carboxylate (85)**

Following the general procedure F and starting from 4-(2-chlorobenzyloxy)-2-(4-chlorophenyl)-1,3-thiazole-5-carboxylic acid **51** (0.16 g, 0.421 mmol), compound **85** was obtained as a yellow solid (170 mg, 96%). $^1$H-NMR (DMSO-$d_6$) δ (ppm): 7.89-7.78 (m, 3H, *J*=8.65), 7.61 (d, 2H), 7.47-7.43 (m, 1H), 7.42 (d, 2H, *J*=8.65), 5.47 (s, 2H).

**Example 86**

**Synthesis of sodium 4-(2-chlorobenzyloxy)-2-(4-methylphenyl)-1,3-thiazole-5-carboxylate (86)**

Following the general procedure F and starting from 4-(2-chlorobenzyloxy)-2-(4-methylphenyl) -1,3-thiazole-5-carboxylic acid **53** (0.4 g, 1.11 mmol), compound **86** was obtained as a white solid (421 mg, 89%). $^1$H-NMR (DMSO-$d_6$) δ (ppm): 7.85-7.82 (m, 1H), 7.74 (d, 2H, *J*=8.11), 7.49-7.46 (m, 1H), 7.37-7.33 (m, 2H), 7.28 (d, 2H, *J*=8.11), 5.55 (s, 2H), 2.34 (s, 3H).

**Example 87**

**Synthesis of sodium 4-(2-chlorobenzyloxy)-2-(3-fluorophenyl)-1,3-thiazole-5-carboxylate (87)**

Following the general procedure F and starting from ethyl 4-(2-chlorobenzyloxy)-2-(3-fluorophenyl)-1,3-thiazole-5-carboxylate **54** (70 mg, 0.179 mmol), compound **87** was obtained as a white solid (69 mg, 92%). $^1$H-NMR (DMSO-$d_6$) δ (ppm): 7.80-7.67 (m, 3H), 7.58-7.47 (m, 2H), 7.41-7.30 (m, 3H), 5.59 (s, 2H).

**Example 88**

**Synthesis of sodium 4-(4-chlorobenzyloxy)-2-(3-fluorophenyl)-1,3-thiazole-5-carboxylate (88)**

Following the general procedure F and starting from ethyl 4-(4-chlorobenzyloxy)-2-(3-fluorophenyl)-1,3-thiazole-5-carboxylate (0.1 g, 0.255 mmol), compound **88** was obtained as a yellow solid (100 mg, 98%). $^1$H-NMR (DMSO-$d_6$) δ (ppm): 7.69-7.40 (m, 7H), 7.31-7.24 (m, 1H), 5.44 (s, 2H).

**Example 89**

**Synthesis of (1*R*,2*S*,5*R*)-2-isopropyl-5-methylcyclohexyl-4-(benzyloxy)-2-(4-methylphenyl)-1,3-thiazole-5-carboxylate (89)**

Following the general procedure described for compound **36** and starting from (1*R*,2*S*,5*R*)-2-isopropyl-5-methylcyclohexanol (91 mg, 0.58 mmol) and 4-(benzyloxy)-2-(4-methylphenyl)-1,3-thiazole-5-carbonyl chloride (0.1 g, 0.29 mmol) (obtained by treatment of the corresponding acid with $SOCl_2$ ,3.0 eq., in toluene), compound **89** was obtained as transparent oil after purification by HPLC (116 mg, 87%). $^1$H-NMR (acetone-$d_6$) δ (ppm): 7.94-7.91 (d, 2H, *J*=7.8), 7.61-7.58 (m, 2H), 7.44-7.31 (m, 5H), 5.69-5.58 (s, 2H), 4.84 (dt, 1H, $J^1$=10.8, $J^2$=4.3), 2.41 (s, 3H), 2.11-1.99 (m, 2H), 1.77-1.69 (m, 2H), 1.56-1.47 (m, 2H), 1.31-1.27 (m, 1H), 1.17-1.07 (m, 1H), 0.95-0.89 (m, 7H), 0.80 (d, 3H, *J*=7.0); MS ($ES^{1+}$) *m/z:* 465.34 (M+1).

**Example 90**

**Synthesis of (1*R*,2*S*,5*R*)-2-isopropyl-5-methylcyclohexyl-4-hydroxy-2-(4-methylphenyl)-1,3-thiazole-5-carboxylate (90)**

A solution of (1*R*,2*S*,5*R*)-2-isopropyl-5-methylcyclohexyl 4-(benzyloxy)-2-(4-methylphenyl)-1,3-thiazole-5-carboxylate **89** (0.1 g, 0.21 mmol) in dry THF was hydrogenated at atmospheric pressure in the presence of Pd/C for 1h. The mixture was then filtered through celite and the filtrate concentrated under reduced pressure to give compound **90** as transparent oil (75 mg, 95%). $^1$H-NMR ($CDCl_3$, TMS) δ (ppm): 10.02 (bs, 1H), 7.90 (d, 2H, *J*=8.1), 7.34-7.26 (m, 2H), 5.02-4.87 (m, 1H), 2.42 (s, 3H), 1.95-1.87 (m, 2H), 1.78-1.69 (m, 2H), 1.65-1.44 (m, 2H), 1.31-1.28 (m, 1H), 1.22-1.09 (m, 1H), 0.97-0.82 (m, 10H); MS ($ES^{1+}$) *m/z:* 375.09 (M+1); 236.76 (M-136).

**Example 91**

**Synthesis of ethyl 2-(4-chlorophenyl)-4-(methoxymethoxy)-1,3-thiazole-5-carboxylate (91)**

Following the general procedure C and starting from ethyl 4-hydroxy-2-(4-chlorophenyl)-1,3-thiazole-5-carboxylate **8** (0.1 g, 0.35 mmol), and chloro(methoxy)methane (56 mg, 0.70 mmol), compound **91** was obtained as a white solid (110 mg, 96%). $^1$H-NMR ($CDCl_3$, TMS) δ (ppm): 7.94 (d, 2H, *J*=8.6 ), 7.45 (d, 2H, *J*=8.6), 5.67 (s, 2H), 4.43 (q, 2H, *J*=7.0), 3.6 (s, 3H), 1.42 (t, 3H, *J*=7.0); MS ($ES^{1+}$) *m/z:* 328.85 (M+1), 256.67 (M-72), 297.74 (M-72+41).

**Example 92**

**Synthesis of 2-(4-chlorophenyl)-4-(methoxymethoxy)-1,3-thiazole-5-carboxylic acid (92)**

Following the general procedure D starting from ethyl 2-(4-chlorophenyl)-4-(methoxymethoxy)-1,3-thiazole-5-carboxylate **91** (0.1 g, 0.3 mmol), compound **92** was obtained as a white solid (86 mg, 95%). $^1$H-NMR ($CDCl_3$, TMS) δ (ppm): 7.92 (d, 2H, *J*=8.6 ), 7.43 (d, 2H, *J*=8.6), 5.42 (s, 2H), 3.71 (s, 3H); MS ($ES^{1+}$) *m/z*: 299.74 (M+1).

**Example 93**

**Synthesis of 2-(4-chlorophenyl)-4-(methoxymethoxy)-1,3-thiazole-5-carboxamide (93)**

2-(4-chlorophenyl)-4-(methoxymethoxy)-1,3-thiazole-5-carboxylic acid **92** (80 mg, 0.28 mmol) was dissolved in dry $CH_2Cl_2$ (10 mL) at 0°C, under $N_2$ atmosphere, 1,1 '-carbonyldiimidazole (0.41 mmol, 68 mg) was added at the same temperature. The mixture was warmed to room temperature and stirred for 40 min. Gaseous $NH_3$ was bubbled into the mixture and the course of the reaction was monitored by LC-MS analysis. At the end of the reaction the mixture was concentrated under reducer pressure and the crude product triturated with acetone. The resulting precipitate was collected by filtration, washed with diethyl ether and purified by flash column chromatography (eluent: dichloromethane/ methanol mixture of increasing polarity). Compound **93** was obtained as white solid (77 mg, 78%). $^1$H-NMR ($CDCl_3$, TMS) δ (ppm): 7.86 (d, 2H, *J*= 8.1), 7.41 (d, 2H, *J*=8.6), 6.98 (br s, 1H), 5.85 (br s, 1H), 5.70 (s,2H), 3.59 (s, 3H); MS ($ES^{1+}$) *m/z:* (ESI+) = 300 (M+1), 282 (M-18), 252 (M-48).

**Example 94**

**Synthesis of 2-(4-chlorophenyl)-4-(methoxymethoxy)-1,3-thiazole-5-carbonitrile (94)**

A 250 mL three-necked round bottom flask was equipped with a thermometer, flame dried, and charged with $N_2$ and a solution 2-(4-chlorophenyl)-4-(methoxymethoxy)-1,3-thiazole-5-carboxamide **93** (450 mg, 1.5 mmol) in dry $CH_2Cl_2$ (8 mL). To this solution DMSO (284 μL, 4.0 mmol) was added and the resulting pale yellow solution cooled to -78°C. A solution of $(COCl)_2$ (270 μL, 3.2 mmol) in dry $CH_2Cl_2$ (2 mL) was then added dropwise. After 15 min. stirring at -78°C, $Et_3N$ (892 μL, 6.4 mmol) was added dropwise to the mixture. The following additiona of DMSO (284 μL, 4.0 mmol), $(COCl)_2$ (270 μL, 3.2 mmol) and $Et_3N$ (500 μL, 3.5 mmol) at intervals of 1 h were necessary in order to complete the starting material consumption. The reaction was quenched by addition of water (20 mL), warming of the mixture to room temperature, and extraction of the aqueous layer with ethyl acetate (3 x 10 mL). The combined organic phases were washed with brine (30 mL), dried over dry $Na_2SO_4$ and concentrated *in vacuo.* Purification by silica gel column chromatography (hexane/acetate 9/1 to 1/1) gave **94** as a pale yellow solid (270 mg, 74%). $^1$H-NMR ($CDCl_3$, TMS) δ (ppm): 7.86 (d, 2H, *J*=7.6), 7.45 (d, 2H, *J*=7.6), 5.59 (s, 2H), 3.58 (s, 3H).

**Example 95**

**Synthesis of 2-(4-chlorophenyl)-5-(1H-tetrazol-5-yl)thiazol-4-ol (95)**

2-(4-chlorophenyl)-4-(methoxymethoxy)-1,3-thiazole-5-carbonitrile **94** (55 mg, 0.14 mmol), sodium azide (10 mg, 0.16 mmol), zinc chloride (19 mg, 0.14 mmol), and 8 mL of water were mixed in a 25 mL round-bottomed flask. The reaction mixture was vigorously stirred at 100°C for 72 h. After consumption of the starting material, 6 N HCl (100 μL) and ethyl acetate (7 mL) were added, and stirring was continued until no solid was present and the aqueous layer reached pH 1. Additional ethyl acetate was added; the organic layer was separated and the aqueous one extracted again with ethyl acetate (2 x 10 mL). The combined organic layers were dried over dry $Na_2SO_4$ and concentrated *in vacuo.* Compound **95** was obtained as pale yellow solid (28 mg, 75%) after preparative HPLC purification. $^1$H-NMR

(dmso-$d_6$) δ (ppm): 16.2 (br s, 1H), 13.0 (br s, 1H), 7.99 (d, 2H, *J*=8.6), 7.62 (d, 2H, *J*=8.6); MS ($ES^{1+}$) *m/z:* 278.8 (M+1), 231.8 (M+41), 235,6 (M-28).

**Example 96**

**Synthesis of 2-(4-chlorophenyl)-5-(1-methyl-1*H*-tetrazol-5-yl)thiazol-4-ol (96)**

A 25 mL three-necked round bottom flask was equipped with a thermometer, flame dried, and charged with $N_2$ and a solution of 2-(4-chlorophenyl)-5-(1*H*-tetrazol-5-yl)thiazol-4-ol **95** (40 mg, 0.14 mmol) in THF (10 mL). To this solution pyridine (12 μL, 0.14 mmol) were added by syringe. The resulting mixture was cooled to 0°C and stirred for 30 min. Methyl iodide (34 μL, 0.17 mmol) was added dropwise by a syringe, while keeping the temperature below 5°C. After the addition, the ice bath was removed, and the solution stirred at room temperature until all starting material was consumed. The reaction mixture was diluted with ethyl acetate (15 mL) and the reaction was cautiously quenched with HCl 0.5 N (10 mL) at 0°C. The solution was allowed to warm to room temperature, the organic layer separated and the aqueous one extracted again with ethyl acetate (2 x 10 mL). The combined organic layers were dried over dry $Mg_2SO_4$ and concentrated *in vacuo.* The resulting yellow solid was purified by preparative HPLC to afford the compound **96** as pale yellow solid (27 mg, 65%). $^1$H-NMR (dmso-$d_6$) δ (ppm): 11.98 (br s, 1H), 7.99 (d, 2H, *J*=8.6), 7.62 (d, 2H, *J*=8.6), 3.91 (s, 3H); MS ($ES^{1+}$) *m/z:* 294.75 (M+1).

**Example 97**

**Synthesis of 2-(3-fluorophenyl)-5-(1-methyl-1*H*-tetrazol-5-yl)-1,3-thiazol-4-ol (97)**

Following the procedure described for compound **96** and starting from 2-(3-fluorophenyl)-5-(1*H*-tetrazol-5-yl)-1,3-thiazol-4-ol, the compound **97** was isolated as dark-yellow oil (73 mg, 47%). $^1$H-NMR (dmso-$d_6$) δ (ppm): 11.79 (br s, 1H), 7.73-7.68 (m, 2H), 7.46-7.39 (m, 1H), 3.82 (s, 3H); MS ($ES^{1+}$) *m/z:* 278.18 (M+1).

**Example 98**

**Synthesis of 2-(4-chlorophenyl)-5-(5-methyl-4*H*-1,2,4-triazol-3-yl)-1,3-thiazol-4-ol (98)**

To a solution of 2-(4-chlorophenyl)-4-(methoxymethoxy)-1,3-thiazole-5-carbonitrile **94** (0.25 g, 0.890 mmol) in dry toluene (10 mL), a solution of $Et_3Al$ (122 μL, 0.89 mmol) in dry toluene was added dropwise and the resulting mixture stirred for 20 min at room temperature. Acetohydrazide (0.165 g, 2.22 mmol) was added and the mixture heated at 90 °C for 6 h until the starting materials had been completely consumed (as checked by TLC and LC-MS analysis). The mixture was diluted with toluene (10 mL) and transferred to a separatory funnel; the organic layer was washed with water, dried over $Na_2SO_4$, filtered, and concentrated *in vacuo* to afford a brown oil which was used in the next step without further purification. The oil was dissolved in toluene (10 mL), added to a microwave vial and irradiated by MW at 170 °C for 20 min. After consumption of the starting material, 2 mL of 6 N HCl were added and vigorous stirring continued for 1h. The organic layer was isolated and the aqueous layer extracted with ethyl acetate (2 x 10 mL). The combined organic layers were evaporated and the crude product was purified by flash chromatography on silica gel (petroleum ether/ethyl acetate 90:10 to 70:30) to afford **98** as transparent oil (125 mg, 48%). $^1$H-NMR (dmso-$d_6$) δ (ppm): 12.11 (br s, 1H), 12.98 (br s, 1H), 7.99 (d, 2H, *J*=7.6), 7.62 (d, 2H, *J*=7.6), 2.25 (s, 3H); MS ($ES^{1+}$) *m/z:* 293.74 (M+1).

**Example 99**

**Synthesis of 2-(3-fluorophenyl)-5-(5-methyl-4*H*-1,2,4-triazol-3-yl)-1,3-thiazol-4-ol (99)**

Following the procedure described for compound **98** and starting from 2-(3-fluorophenyl)-4-(methoxymethoxy)-1,3-thiazole-5-carbonitrile, the compound **99** was isolated following flash chromatography on silica gel ($CH_2Cl_2$/$CH_3OH$ 90:10) as slightly red oil (97 mg, 31%). $^1$H-NMR (dmso-$d_6$) δ (ppm): 11.93 (br s, 1H), 12.80 (br s, 1H), 7.76-7.69 (m, 2H), 7.46-7.39 (m, 1H), 7.22-7.17 (m, 1H), 2.21 (s, 3H); MS ($ES^{1+}$) *m/z:* 277.27 (M+1).

**Example 100**

**Synthesis of 2-(4-chlorophenyl)-5-(5-methyl-1,2,4-oxadiazol-3-yl)-1,3-thiazol-4-ol (100)**

A microwave vial was charged with 2-(4-chlorophenyl)-4-(methoxymethoxy)-1,3-thiazole-5-carbonitrile **94** (250

mg, 0.89 mmol), acetic acid (5 mL), hydroxylamine (117 mg, 3.56 mmol) and 2,2-dimethyl-1,3-dioxane-4,6-dione (Meldrum's acid) (131 mg, 0.91 mmol). The mixture was irradiated by MW for 10 min at 130 °C, then quenched with 10 mL of water and the precipitate was filtered and dried under vacuum at 50 °C. The solid obtained was dissolved in a mixture of HCl 6N (5 mL) and ethyl acetate (10 mL) and stirred for 1h. The two phases were separated into a separatory funnel; the organic layer was washed with water, dried over $Na_2SO_4$, filtered, and concentrated in *vacuo* to afford compound **100** as a dark yellow solid (107 mg, 41%). $^1$H-NMR (dmso-$d_6$) δ (ppm): 11.98 (br s, 1H), 7.89 (d, 2H, *J*=7.5), 7.51 (d, 2H, *J*=7.6), 2.55 (s, 3H); MS (ES$^{1+}$) *m*/*z:* 294.50 (M+1), 316.7 (M+Na).

**Example 101**

**Synthesis of 2-(3-fluorophenyl)-5-(5-methyl-1,2,4-oxadiazol-3-yl)-1,3-thiazol-4-ol (101)**

Following the experimental procedure described for compound **100** and starting from 2-(3-fluorophenyl)-4-(methoxymethoxy)-1,3-thiazole-5-carbonitrile compound **101** was isolated as white solid (177 mg, 52%). $^1$H-NMR (dmso-$d_6$) δ (ppm): 11.81 (br s, 1H), 7.76-7.69 (m, 3H), 7.22-7.17 (m, 1H), 2.33 (s, 3H); MS (ES$^{1+}$) *m*/*z:* 278.27 (M+1).

**Example 102**

**Synthesis of 3-{4-[(4-chlorobenzyl)oxy]-2-(3-fluorophenyl)-1,3-thiazol-5-yl}-5-methyl-1,2,4-oxadiazole**

**(102)** The title compound was prepared according to the general procedure C and starting from 2-(3-fluorophenyl)-5-(5-methyl-1,2,4-oxadiazol-3-yl)-1,3-thiazol-4-ol **101** (221 mg, 0.8 mmol) and 1-chloro-4-(chloromethyl)benzene (0.16 g, 1.00 mmol). Compound **102** was obtained as dark red oil (234 mg, 73%). $^1$H-NMR (dmso-$d_6$) δ (ppm): 7.83-7-79 (d, 2H, *J*=7.6), 7.76-7.69 (m, 2H), 7.63-7.59 (m, 1H), 7.51 (d, 2H, *J*=7.4), 7.22-7.17 (m, 1H), 5.23 (s, 2H), 2.33 (s, 3H); MS (ES$^{1+}$) *m*/*z:* 402.8 (M+1).

**Example 103**

**Synthesis of 2-(4-chlorophenyl)-5-(5-methyl-1,3,4-oxadiazol-2-yl)-1,3-thiazol-4-ol (103)**

To a cooled solution (0 °C) of 2-(4-chlorophenyl)-4-hydroxy-1,3-thiazole-5-carboxylic acid 1 (0.43 g, 1.68 mmol) in $CH_2Cl_2$ (15 mL), 1,1-carbonyldiimidazole (CDI, 275 mg, 1.70 mmol) was added. After stirring 1 h at 0 °C, acetohydrazide (124 mg, 1.68 mmol) and diazobicyclo-[5.4.0]undec-7-ene (DBU, 260 μL, 1.68 mmol) were added, and the mixture was allowed stirring at room temperature for 4 h. Glacial AcOH (200 μL, 3.5 mmol) was added and the reaction mixture diluted with $CH_2Cl_2$ (10 mL). The organic layer washed with saturated $NH_4Cl$ (2 x 10 mL) and water (2 x 10 mL), dried over $Na_2SO_4$, filtered and concentrated under reduced pressure to give a crude residue that, after purification by flash chromatography ($CH_2Cl_2$/$CH_3OH$ 95:5) afforded *N'*-acetyl-2-(4-chlorophenyl)-4-hydroxy-1,3-thiazole-5-carbohydrazide as pale yellow oil. The compound was dissolved in polyphosphoric acid (5 mL) in a microwave vial and irradiated by MW for 40 min at 150°C. The solution was added to an ice/water mixture and the precipitate filtered and washed with water. The solid precipitated was dried under vacuum at 50°C to afford compound **103** as a whitish solid (182 mg, 37%). $^1$H-NMR (dmso-$d_6$) δ (ppm): 11.89 (br s, 1H), 7.89 (d, 2H, *J*=7.6), 7.51 (d, 2H, *J*=7.4), 2.65 (s, 3H); MS (ES$^{1+}$) *m*/*z:* 294.50 (M+1), 316.7 (M+Na).

**Example 104**

**Synthesis of 2-(3-fluorophenyl)-5-(5-methyl-1,3,4-oxadiazol-2-yl)-1,3-thiazol-4-ol (104)**

The title compound was prepared according to the experimental procedure described for compound **103** and starting from 2-(3-fluorophenyl)-4-hydroxy-1,3-thiazole-5-carboxylic acid **3.** Compound **104** was isolated as a yellow oil (109 mg, 59%). $^1$H-NMR (dmso-$d_6$) δ (ppm): 11.79 (br s, 1H), 7.63-7.79 (m, 3H), 7.11-7.18 (m, 1H), 2.61 (s, 3H); MS (ES$^{1+}$) *m*/*z:* 278.27 (M+1).

**Example 105**

**Synthesis of ethyl 4-hydroxy-2-phenyl-1,3-oxazole-5-carboxylate (105)**

An oven-dried microwave vial was evacuated, backfilled with argon and finally charged with benzamide (0.3 g, 2.48 mmol), diethyl bromopropanedioate (1.27 μL, 7.44 mmol) and dry DMSO (3 mL). The reaction vial was sealed

and placed in the microwave reactor and irradiated for 2h at 250 °C until the complete consumption of the starting materials (as checked by TLC and GC analysis). The crude was diluted with ethyl acetate (20 mL) and washed with water (3×10 mL). The combined organic layers were dried over $Na_2SO_4$, filtered and the solvent was removed under reduced pressure. The residue was purified by flash chromatography on silica gel ($CH_2Cl_2$/$CH_3OH$ 95:5) to afford the compound **105** as colourless oil (83%). $^1$H-NMR (dmso-$d_6$) δ (ppm): 12.3 (br s, 1H), 7.95-7.92 (m, 2H), 7.55-7.53 (m, 3H), 4.43 (q, 2H, *J*=7.03), 1.42 (t, 3H, *J*=7.03); MS ($ES^{1+}$) *m*/*z:* 234.33 (M+1).

**Example 106**

**Synthesis of ethyl 2-(3-fluorophenyl)-4-hydroxy-1,3-oxazole-5-carboxylate (106)**

The title compound was prepared according to the experimental procedure described for compound **105** and starting from 3-fluorobenzamide. Compound **106** was isolated as a yellow oil (99 mg, 59%). $^1$H-NMR ($CDCl_3$, TMS) δ (ppm): 11.98 (br s, 1H), 8.01 (s, 1H), 7.77 (d, 1H, *J*=7.57), 7.49-7.30 (m, 2H), 4.40 (q, 2H, *J*=7.03), 1.52 (t, 3H, *J*=7.03); MS ($ES^{1+}$) *m*/*z:* 252.18 (M+1).

**Example 107**

**Synthesis of ethyl 4-hydroxy-2-(4-methylphenyl)-1,3-oxazole-5-carboxylate (107)**

The title compound was prepared according to the experimental procedure described for compound **105** and starting from *p*-tolylbenzamide. Compound **107** was isolated as a whitish oil (39 mg, 79%). $^1$H-NMR ($CDCl_3$, TMS) δ (ppm): 11.94 (br s, 1H), 7.88 (d, 2H, *J*=7.6), 7.26 (d, 2H, *J*=7.6), 4.62 (q, 2H, *J*=7.0), 2.41 (s, 3H), 1.39 (t, 3H, *J*=7.0); MS ($ES^{1+}$) *m*/*z:* 248.25.

**Example 108**

**Synthesis of ethyl 4-[(4-chlorobenzyl)oxy]-2-phenyl-1,3-oxazole-5-carboxylate (108)**

Following the general procedure C and starting from ethyl 4-hydroxy-2-phenyl-1,3-oxazole-5-carboxylate **105** (055 g, 2.67 mmol) and 1-chloro-4-(chloromethyl)benzene (1.074 g, 6.67 mmol), compound **108** was obtained as a white solid (0.8 g, 81%). $^1$H-NMR ($CDCl_3$, TMS) δ (ppm): 7.83-7.48 (m, 5H), 7.35 (d, 2H, *J*= 7.5), 7.27 (d, 2H, *J*= 7.6), 5.31 (s, 2H), 4.35 (q, 2H, *J*= 7.03), 1.38 (t, 3H, *J*= 7.03); MS ($ES^{1+}$) *m*/*z:* 358.80 (M+1), 380.77 (M+Na).

**Example 109**

**Synthesis of ethyl 4-[(4-chlorobenzyl)oxy]-2-(3-fluorophenyl)-1,3-oxazole-5-carboxylate (109)**

Following the general procedure C and starting from ethyl 2-(3-fluorophenyl)-4-hydroxy-1,3-oxazole-5-carboxylate **106** (110 mg, 0.43 mmol), compound **109** was obtained as a white powder (114 mg, 71%). $^1$H-NMR ($CDCl_3$, TMS) δ (ppm): 7.85 - 7.51 (m, 3H), 7.47 (m, 1H), 7.36 (d, 2H, *J*= 7.6), 7.26 (d, 2H, *J*= 7.6), 5.53 (s, 2H), 4.33 (q, 2H, *J*= 7.03), 1.38 (t, 3H, *J*= 7.03); MS ($ES^{1+}$) *m*/*z:* 376.66 (M+1)

**Example 110**

**Synthesis of ethyl 4-[(4-chlorobenzyl)oxy]-2-(4-methylphenyl)-1,3-oxazole-5-carboxylate (110)**

Following the general procedure C and starting from ethyl 4-hydroxy-2-(4-methylphenyl)-1,3-oxazole-5-carboxylate **107** (98 mg, 0.39 mmol) compound **110** was obtained as a white powder (114 mg, 79%). $^1$H-NMR ($CDCl_3$, TMS) δ (ppm): 7.82 (d, 2H, *J*= 7.6), 7.49 (d, 2H, *J*= 7.6), 7.23 (d, 2H, *J*= 7.6), 7.19 (d, 2H, *J*= 7.6), 5.32 (s, 2H), 4.30 (q, 2H, *J*= 7.03), 2.39 (s, 3H), 1.28 (t, 3H, *J*= 7.03); MS ($ES^{l+}$) *m*/*z:* 372.72 (M+1).

**Example 111**

**Synthesis of ethyl 2-phenyl-4-{[4-(trifluoromethyl)benzoyl]oxy}-1,3-oxazole-5-carboxylate (111)**

Following the procedure described for compound **36** and starting from ethyl 4-hydroxy-2-phenyl-1,3-oxazole-5-carboxylate **105** (67 mg, 0.28 mmol) compound **111** was obtained as colorless oil (77 mg, 67%). $^1$H-NMR ($CDCl_3$,

TMS) δ (ppm): 7.73 - 7.51 (m, 5H), 7.47 (d, 2H, *J*= 7.5), 7.35 (d, 2H, *J*= 7.6), 4.37 (q, 2H, *J*= 7.13), 1.48 (t, 3H, *J*= 7.11); MS ($ES^{1+}$) *m/z:* 406.28 (M+1).

**Example 112**

**Synthesis of 4-[(4-chlorobenzyl)oxy]-2-phenyl-1,3-oxazole-5-carboxylic acid (112)**

The title compound was prepared according to the general procedure D and starting from ethyl 4-[(4-chlorobenzyl)oxy]-2-phenyl-1,3-oxazole-5-carboxylate **108** (123 mg, 0.34 mmol). Compound **112** was obtained as slightly dark oil (99 mg, 88%). $^1$H-NMR (dmso-$d_6$) δ (ppm): 12.12 (br s, 1H), 7.71 - 7.49 (m, 5H), 7.41 (d, 2H, *J*= 8), 7.34 (d, 2H, *J*= 7.6), 5.61 (s, 2H); MS ($ES^{1+}$) *m/z:* 330.68 (M+1).

**Example 113**

**Synthesis of 4-[(4-chlorobenzyl)oxy]-2-(3-fluorophenyl)-1,3-oxazole-5-carboxylic acid (113)**

The title compound was prepared according to the general procedure D and starting from ethyl 4-[(4-chlorobenzyl)oxy]-2-(3-fluorophenyl)-1,3-oxazole-5-carboxylate **109** (88 mg, 0.23 mmol). Compound **113** was obtained as white solid (70 mg, 86%). $^1$H-NMR (dmso-$d_6$) δ (ppm): 11.91 (br s, 1H), 7.81 - 7.50 (m, 3H), 7.45 (m, 1H), 7.31 (d, 2H, *J*= 7.6), 7.26 (d, 2H, *J*= 7.6), 5.63 (s, 2H); MS ($ES^{1+}$) *m/z:* 348.62 (M+1).

**Example 114**

**Synthesis of 4-[(4-chlorobenzyl)oxy]-2-(4-methylphenyl)-1,3-oxazole-5-carboxylic acid (114)**

The title compound was prepared according to the general procedure D and starting from ethyl 4-[(4-chlorobenzyl)oxy]-2-(4-methylphenyl)-1,3-oxazole-5-carboxylate **110** (0.12 g, 0.32 mmol). Compound **114** was obtained as whitish solid (98 mg, 89%). $^1$H-NMR ($CD_3OD$) δ (ppm): 7.89 (d, 2H, *J*=7.6), 7.53 (d, 2H, *J*=7.6), 7.41 (d, 2H, *J*=7.5), 7.33 (d, 2H, *J*=7.6), 5.43 (s, 2H), 2.39 (s, 3H); MS ($ES^{1+}$) *m/z:* 344.66 (M+1).

**Example 115**

**Synthesis of 2-(3-fluorophenyl)-5-(5-methyl-1,2,4-oxadiazol-3-yl)-1,3-oxazol-4-ol (115)**

The title compound was prepared according to the experimental procedure described for compound **100** and starting from 2-(3-fluorophenyl)-4-hydroxy-1,3-oxazole-5-carbonitrile (76 mg, 0.25 mmol). Compound **115** was obtained as pale yellow oil (54 mg, 79%). $^1$H-NMR (dmso-$d_6$) δ (ppm): 12.1 (br s, 1H), 7.83-7.74 (m, 3H), 7.11-7.18 (m, 1H), 2.51 (s, 3H); MS ($ES^{1+}$) *m/z:* 262.21 (M+1).

**Example 116**

**Synthesis of 3-{4-[(4-chlorobenzyl)oxy]-2-(3-fluorophenyl)-1,3-oxazol-5-yl}-5-methyl-1,2,4-oxadiazole (116)**

The title compound was prepared according to the experimental procedure B and starting from 2-(3-fluorophenyl)-5-(5-methyl-1,2,4-oxadiazol-3-yl)-1,3-oxazol-4-ol **115** (98 mg, 0.37 mmol). Compound **116** was obtained as yellow oil (88 mg, 61%). $^1$H-NMR ($CDCl_3$, TMS) δ (ppm): 7.77 (m, 1H), 7.61 (m, 1H), 7.60-7.55 (m, 3H), 7.50 (m, 1H), 7.35-7.29 (m, 2H), 5.40 (s, 2H), 2.45 (s, 3H); MS ($ES^{1+}$) *m/z* 386.78 (M+1).

**Example 117**

**Synthesis of ethyl 2-(3-fluorophenyl)-5-hydroxy-1,3-thiazole-4-carboxylate (117)**

Triethylamine (3.4 mL, 24 mmol) and 4-methylbenzoyl chloride (1.59 mL, 12.0 mmol) were added to a solution of diethyl 2-aminomalonate hydrochloride (2.28 g, 10.8 mmol in $CH_2Cl_2$ (40 mL) and the resulting mixture was stirred overnight at room temperature. The mixture was washed with aqueous $NaHCO_3$ (20 mL), 1M HCl (20 mL), and water (20 mL); the organic layer was dried over anhydrous $Na_2SO_4$, filtered, and the solvent was evaporated under vacuum to yield the intermediate diethyl [(4-methylbenzoyl)amino]propanedioate as a yellow solid (96%).

Diethyl [(4-methylbenzoyl)amino]propanedioate (3.08 g, 10.5 mmol) was dissolved in THF (50 mL). Lawesson

reagent (3.0 g, 7.4 mmol) was added and the mixture stirred overnight at room temperature. After solvent removal under reduced pressure, the crude product was purified by flash column chromatography (eluent: hexane/ethyl acetate mixture of increasing polarity) to give the intermediate diethyl {[(4 - methylphenyl)carbonothioyl]amino}propanedioate (85%).

Diethyl {[(4-methylphenyl)carbonothioyl]amino}propanedioate (2.47 g, 8.00 mmol) was dissolved in dioxane (35 mL) and phosphoryl chloride (0.5 mL, 5 mmol) was added. The mixture was irradiated by microwave (250 W, 100°C) for 15 min, whereupon the solvent was removed under vacuum. The compound **117** was obtained by trituration with acetonitrile (1.67 g, 84%). $^1$H-NMR ($CDCl_3$, TMS) $\delta$ (ppm): 9.93 (br s, 1H), 7.81 (d, 2H, *J*=7.3), 7.21 (d, 2H, *J*=7.3), 4.58 (q, 2H, *J*=7.0), 2.37 (s, 3H), 1.39 (t, 3H, *J*=7.0); MS ($ES^{1+}$) *m/z:* 264.30 (M+1).

**Example 118**

*Evaluation of in vitro activity*

a. Cloning, sequencing, transfection and selection of positive clones expressing human TRPM8

A functional cell-based assay for the identification of TRPM8 receptor antagonists, optimised to allow high throughput screening at $FLIPR^{TETRA}$, was developed in HEK293 cells by stable pure clone selection and functional characterization with a fluorescent calcium sensitive dye.

TRPM8 was cloned into the multiple clonig site of pcDNA3 mammalian expression vector; the obtained construct pcDNA3/hTRPM8 was fully sequence verified and used for the transfection of HEK293 cell line. HEK293 cells stably transfected with TRPM8 gene were maintained in Minimum essential medium. The cells were transfected with the pcDNA3/hTRPM8 vector by electroporation and then selected with medium containing 0.8 mg/ml G418 for 10-15 days.

The following commercial compounds were used as TRPM8 channel reference compound to test HEK293/hTRPM8 cell line for both agonist and antagonist activity:

Activators: Menthol (SIGMA cat.# M2772) WS-3, (N-Ethyl-5-methyl-2-(1-methylethyl) cyclohexanecarboxamide) (SIGMA cat.# W345501)
Blocker: Capsazepine (SIGMA cat.# C191)

The experimental activities were performed using FLIPR instruments.

The functional clones were selected at $FLIPR^{384}$ on the basis of 1 mM menthol response. Two best responder clones were selected, diluted at a cell density of 1 cell/well and analysed at $FLIPR^{384}$ with 1 mM menthol. The TRPM8 receptor was analysed for the response to reference agonist, menthol, using a calcium-dependent fluorescence signal.

Patch clamp recordings were also obtained in voltage-clamp configuration on HEK/TRPM8 clones in order to verify the receptor pharmacology and to determine the agonist dose-response curve and $EC_{50}$ value. HEK293 cells were maintained at room temperature on an fire-polished borosilicate glass pipettes having 1.5-2.5 M$\Omega$ resistance were used to record currents following drug application. Menthol application induced a dose-dependent inward current in a selected HEK/hTRPM8 clone (calculated $EC_{50}$ value = 58$\mu$M). No menthol-induced currents were recorded in not transfected HEK293 cells.

In order to determine the capsazepine antagonist activity on menthol agonist response and to verify the antagonist response stability throughout different days of experiments, the selected clone of TRPM8 was analysed after 24h at $FLIPR^{384}$ in presence of variable concentrations of antagonist (from 100 nM to 316 $\mu$M). The selected clone showed very good stability and reproducibility of the antagonist activity (calculated $IC_{50}$ value = 20$\mu$M).

Summarizing, the best clone was characterized for :1- pharmacology: agonist $EC_{50}$ and antagonist $IC_{50}$ determination over different experiments;

2- optimal cell density and seeding time;
3- DMSO sensitivity;
4- ligand stability;
5- patch clamp analysis.

b. Screening set up for the identification of TRPM8 antagonists

The following commercial compounds were used as ligands:

Activator: Cooling Agent 10 (Takasago CAS N. 87061-04-9)
Blocker: Capsazepine (SIGMA cat # D_5879)

The experimental activities were performed using $FLIPR^{TETRA}$ instruments.

HEK293 cells stably transfected with TRPM8 gene were maintained in Minimum essential medium.

The TRPM8 cell line was analysed for the response to a library of compounds using a $Ca^{2+}$ mobilization-dependent fluorescence signal in 384 wells microtiter plate format. The analysis was performed using the $FLIPR^{TETRA}$ (MDC) with the ICCD Camera.

The execution of the assay involved the use of three microtiter plates:

1. Assay plate, containing cells loaded with dye and prepared as follows:

Cells were seeded at 15000 c/well in Poly-D-Lysine coated 384 wells Microtiter Plates in complete medium (25μl/well).
24h after seeding, the cell plates were washed with Tyrode assay buffer by the Microplate Washer and 10μL of Tyrode assay buffer was left in each well.
Cells were then loaded with 10 μL/well of the Fluo-4 NW dye solution by CyBi®-Well pipettor. Each bottle of Fluo4-NW dye (Molecular Probes cat. #F36206, component A) was re-suspended in 8mL of Tyrode assay buffer and supplemented with 100 μL of water-soluble probenecid (MolecularProbes cat.#F36206, component B).
Dye loaded cell plates were incubated for 1 h at room temperature.

**2. Compound Dilution Plate** (Figure 1), containing diluted test compounds, formulated as follows:

Column 1: wells containing Assay Buffer plus DMSO 0.5% final
Column 2: wells alternating Max Signal Control in first injection (Maximum Response: Cooling Agent 10 at $EC_{100}$, 100 μM) and Min Signal Control in first injection (Assay buffer plus 0.5% DMSO final);
Columns 3-22: wells containing Assay Buffer plus 0.5% DMSO final. To these wells the compounds to be tested were added at 3x concentration.
Column 23: alternating wells of Max Signal Control in second injection (Assay buffer) and Min Signal Control in second injection (Antagonist Capsazepine $IC_{100}$, 50 μM) in Assay buffer plus 0.5% DMSO final;
Column 24: wells containing Capsazepine (Antagonist) at 8 concentrations in duplicate at final concentrations of 50μM, 25μM, 6.25μM, 3.15μM, 1.56μM, 780nM, 309nM in Assay buffer plus 0.5% DMSO final.

**3. Activator Plate** (Figure 2), containing agonist Cooling Agent 10 at EC80, formulated as follows:

Column 1: Cooling Agent 10 (Agonist) at 8 concentrations dose response in duplicate at final concentrations of 100μM, 31.6μM, 10μM, 3.16μM, 1μM, 316nM, 100nM, 31.6nM in Assay buffer;
Columns 2-24: Cooling Agent 10 (Agonist) at $EC_{80}$ (3 fold concentrated, 20μM final) in Assay buffer.

The test was carried out according to a procedure comprising the following steps:

1. The samples contained in the wells of the Compound Plate were added to the corresponding wells of the Assay Plate by the $FLIPR^{TETPA}$, thus resulting in the addition in Columns 3-22 of the test compounds at 3x concentration to the cells of the assay plates. No mixing was performed in the assay wells and the signal of the emitted fluorescence was recorded for 300 seconds.
2. The samples contained in the wells of the Activator Plate were added to the corresponding wells of the Assay Plate by the $FLIPR^{TETRA}$, thus resulting in the addition in Columns 3-22 of the Assay Plate of the agonist compound in addition to the test compounds. The signal of the emitted fluorescence was recorded for 180 seconds.

Columns 1, 2, 23 and 24 were used as control. In particular: the "Max Signal Control in first injection" indicates the Cooling Agent 10 agonist response at $EC_{100}$, "Max Signal Control in the second injection" indicates the agonist at $EC_{80}$ (10μM) in presence of pre-injected Assay buffer, the "Min Signal Control in first injection" corresponds to Assay buffer injection and "Min Signal Control in the second injection" indicates the agonist at $EC_{80}$ (20μM) in presence of pre-injected reference antagonist Capazepine at $IC_{100}$ (50μM).

Figure 3 respresents a typical kinetic response graph obtained with all the compounds of Table IV.

During the Target Activation (TA) phase, the injection of the reference agonist at $EC_{80}$ gave an increase of fluorescent signal in MAX Signal control wells in which the assay buffer in CA was preinjected, while the response was completely inhibited in MIN Signal control wells due to the preinjection of the reference inhibitor Capsazepine.

The goal of the assay was to find antagonists of TRPM8 activity; to this aim the change of fluorescent signal during TA phase was measured.

Several parameters were computed and analyzed (Z' factor, Interplate variability, Intraplate variability, Day to Day variability, Antagonist Dose response and $IC_{50}$ determination, Agonist Dose response and $EC_{50}$ determination).

As for the antagonist Dose response and $IC_{50}$ determination, capsazepine (reference antagonist) was included as control and the $IC_{50}$ values of all the assayed compounds were calculated.

Compounds 1-117 were tested and all showed an $IC_{50}$ value < 30μ; in particular, compounds n. 1, 2, 5, 8, 9, 27, 36, 41, 43, 67, 68, 70, 83, 84 were charaterized by an $IC_{50}$ value < 10μ; compounds n. 10 and 45 showed an $IC_{50}$ value = 1 μM and 0.0002 μM, respectively.

c. Calcium Influx Assay

The ability of compounds n.10 and 45 to act as TRPM8 antagonists was also evaluated with a calcium influx assay. The effects of 7 concentrations (0.00001, 0.0001, 0.001, 0.01, 0.1, 1, and 10 μM) of compounds 10 and 45 were evaluated on TRPM8 using the following experimental procedure.

Channels were activated with menthol, as the positive control agonist, and the ability of test compound to inhibit this signal was examined and compared to the positive control antagonist, 2-APB (inserire dettagli composto). The signal elicited in the presence of the positive control agonist (10 μM menthol) was set to 100% and the signal in the presence of the positive control antagonist (200 μM 2-APB) was set to 0. The $pIC_{50}$ values of the compound 10 and 45 were 9.7 and 6, respectively. Values were considered significant if the test compound mean was three or more standard deviations away from the positive control agonist mean.

**Example 119**

*Evaluation of in vivo activity*

a. Isovolumetric Bladder Model

Female rats were anesthetized with urethane. Ureters were ligated and sectioned. A catheter was inserted through the urinary meatus into the bladder before urethral ligature. The bladder was filled first 3 times every 5 min with 100μL of a solution of solutol/NMP (2:1 w/w) containing 0.1 mg of compounds n. 10 or 45 or with 100 μL of vehicle, then with 100μL of saline every 5 min until the occurrence of rhythmic bladder contraction (RBC). A maximal volume of 3mL was infused. The intravesical pressure was followed during 1h30 after RBC appearance. For each group, Threshold Volume (TV), Micturition Frequency (MF) and Amplitude of Micturition (AM) were measured during the whole period. In the group treated with compound 10 the threshold volume (TV) was significantly increased compared to the group treated with the solvent reaching 1.5 mL of volume whereas, in the vehicle group, RBC occurred in all rats with a mean volume of 0.7±0.09 mL. Compound 10 did not change AM. No effect on the total MF (measured during 90 min) was observed. Both the molecules showed significant efficacy in the isovolumetric model in inhibiting rhythmic bladder contractions and micturition frequency. In particular, the systemic treatment with compound 10 (10 mg/kg i.v.) significantly reduced Micturition Frequency (MF) of about 36% in the first 30 min of the experiment. On the other hand, when administered by intravesical route at 2.3 mg/rat, compound 10 completely abolished the continuous RBC induced by the filling of the bladder with saline. In addition, compound 10 (2.3 mg/rat) and compound 45 (0.3 mg/rat) significantly increased the threshold volume (equivalent to the bladder capacity), reaching a higher volume of 1.5-3.0 mL if compared to that of 0.7±0.9 mL of the vehicle group. Both the compounds did not change Amplitude of Micturition (AM) when compared to basal values, suggesting that they are selective for the afferent arm of micturition reflex with no effect on the efferent pathway.

b. Chronic Constriction Model of Pain

Male Sprague-Dawley rats were used. Under pentobarbital anesthesia, the sciatic nerve was exposed at mid-thigh level (Bennett GJ et alPain. 33: 87-107, 1988). Four ligatures were loosely tied around the sciatic nerve of the left hind limb to induce enhancement of pain caused by nerve injury. Mechanical allodynia was evaluated by using a set of 8 manual von Frey monofilaments (0.4, 0.6, 1, 2, 4, 6, 8 and 15 g) 18 days after surgery and basal response was recorded.

On day 19 body weight was recorded and compounds 10 and 45 were administered by i.v. route at the dose of 10 mg/kg. 60, 120 and 180 min post dose, mechanical allodynia was tested by evaluation of the Paw Withdrawl Threshold (PWT) and % Maximum Possible Effect (MPE) was calculated according to the following formula:.

$$\% MPE = \frac{(\text{Log PWT of test-Avg Log PWT of baseline predrug})}{(\text{Log (15)-Avg log PWT of baseline predrug})} * 100$$

The mechanical allodynia was tested 1 day before treatment and at 2 hours post-dosing. One-way ANOVA followed by Dunnett's test was applied for comparison between vehicle control and test compound treated groups. *$p<0.05$ is considered significant.

Compounds 10 and 45 showed a significant anti-allodynic activity at 2 hours post-dosing in CCI rats (see Figure 4).

**Example 120**

*Selectivity analysis*

The objective of this study was to evaluate the *in vitro* effects of compounds 10 and 45 on cloned human GPCRs (G-protein coupled receptors) expressed in HEK293 or CHO cells using radioligand binding assays (compound concentration=10 μM).

Three replicates were performed for each experiment.

For each assay a concentration-response curve of the appropriate reference compound was performed in each experiment. The sample radioactivity content was measured after the addition of the scintillation cocktail Microscint 20 (PerkinElmer), by a microplate scintillation Beta-counter TopCount NXT (PerkinElmer). The atomic disintegrations per minutes evaluated with the beta counter were about 15 times higher than those found using the gamma counter. Data are expressed as percentage of control binding value (%B) and test compound inhibition was considered significant when %B was < 75% at 10μM.

**Table I**

| Receptor | Cmpd 10 (%B) | Cmpd 45 (%B) |
|---|---|---|
| human Muscarinic $M_2$ Receptor | 90 | 96 |
| human Muscarinic $M_3$ Receptor | 98 | 95 |
| human Adrenergic $\beta_1$ Receptor | 82 | 90.53 |
| human Adrenergic $\beta_2$ Receptor | 88 | 93 |
| human Adrenergic $\alpha_{1A}$ Receptor | 100 | 96 |
| human Adrenergic $\alpha_{2A}$ Receptor | 100 | 100 |
| human Serotoninergic 5-$HT_{1A}$ Receptor | 100 | 100 |
| human Histamine $H_1$ Receptor | 99.3 | 100 |
| human Histamine $H_2$ Receptor | 90 | 93.2 |
| human Cannabinoid $CB_2$ Receptor | 102 | 86.7 |
| human Bradykinin $B_1$ Receptor | 91 | 99.6 |
| human Dopamine $D_{2S}$ Receptor | 100 | 100 |
| human Dopamine $D_3$ Receptor | 87 | 97.2 |

As it is possible to note from Tab I, both compounds show a high selectivity versus a wide range of selected GPCRs (including muscarinic M3, CB2, BK1, alpha e beta adrenergic) that are well know to be involved in the pain control. These data support that the obeserved in vivo efficacy of both compounds 10 and 45 and in general of all the compounds of the invention is potential strongly dependent on the TRPM8 blockage.

In order to address more specifically the potential selectivity issues, a counterassay was carried out for 10 and 45 against TRPV1 and TRPV4 ion channels, both involved in the nociception (Jhaveri MD, et al 2005. Eur. J. Neurosci. 22 (2): 361-70, Brierley SM et al, 2008, Gastroenterology. 2008 Jun;134(7):2059-69. ).

The ability of each test article to act as an antagonist of TRPV1 was evaluated with a calcium influx assay. The

signal elicited in the presence of the positive control agonist (0.1 μM capsaicin) was set to 100% and the signal in the presence of the antagonist (5 μM ruthenium red) was set to 0. The normalized % inhibition of the test articles is shown in Table below. Values were considered significant if the test article mean was three or more standard deviations away from the positive control agonist mean..

In parallel, the ability of each test article to act as an antagonist of TRPV4 was evaluated with a calcium influx assay. The signal elicited in the presence of the positive control agonist (10 μM GSK1016790A) was set to 100% and the signal in the presence of the antagonist (5 μM ruthenium red) was set to 0. The normalized % inhibition of the test articles is shown in Table below. Values were considered significant if the test article mean was three or more standard deviations away from the positive control agonist mean (i.e., greater than 31.70% inhibition for plate 1 and 24.60% inhibition for plate 2).

Table II

| Cmpd (10 and 1 μM) | Test Conc. | Normalized % inhibition (TRPV1) | **Normalized % inhibition (TRPV4)** |
|---|---|---|---|
| 10 | 1 | 1.2 | 2.0 |
| | 10 | 2.3 | 8.9 |
| 45 | 1 | 3.1 | 5.1 |
| | 10 | 6.3 | 3.1 |

The data strongly highlight the great selectivity of molecules 10 and 45 towards both TRPV1 and TRPV4, thus confirming their selective mechanism of action.

**Example 121**

*ADME evaluation*

The pharmacokinetic profiles of compounds 10 and 45 were evaluated. The result are summarised in Table III:

Table III

| **Compound 10** | | | | | | | | |
|---|---|---|---|---|---|---|---|---|
| **Log D** | **CYP (% inhibition) @10μM** | | **hERG binding @10μM** | **Microsome stability (% remaining) @1μM** | | **$t_{1/2}$ i.v. rat (min)** | **Plasma Protein Binding (% @10μM)** | |
| 1.59 | 1A2 | <5 | No effect | rat | 4 | 50 | rat | 98 |
| | 2C9 | <5 | | | 5 | | | |
| | 2C19 | <5 | | human | | | human | 97 |
| | 2D6 | <5 | | | 5 | | | |
| | 3A4 | <5 | | | 5 | | | |
| **Compound 45** | | | | | | | | |
| **Log D** | **CYP (% inhibition) @10μM** | | **hERG binding @10μM** | **Plasma stability (% remaining) @10μM** | | **$t_{1/2}$ i.v. rat (min), F(%)** | **Plasma Protein Binding (% @10μM)** | |
| 2.7 | 1A2 | <5 | No effect | rat | 9 | 240, 60 | rat | 99 |
| | 2C9 | <5 | | | 5 | | | |
| | 2C19 | <5 | | human | 1 | | human | 99 |
| | 2D6 | <5 | | | 0 | | | |
| | 3A4 | <5 | | | 0 | | | |

Both molecules show no effect towards any human cytochrome isoform at the maximal concentration of 10 uM thus excluding potential drug drug interaction.

In addition, none effect was observed towards hERG channel thus excluding potential cardiotoxic effect during the clinical development.

The low logD values of 10 makes it particularly suitable when ip, iv and i ves applications are required, especially in the treatment of urological disorders. At the same time, the relatively high plasma half-life (4 h) and the high oral bioavailability (F= 60%) could makes it the ideal candidate for the treatment of chronic diseases, like inflammatory and neuropathic pain.

Table IV

| Compound | X | $R^1$ | $R^2$ | $R^3$ | $R^4$ |
|---|---|---|---|---|---|
| **1** | S | OH | COOH | H | *p* - Cl |
| **2** | S | OH | COOH | H | *p*-$CH_3$ |
| **3** | S | OH | COOH | H | *m* - F |
| **4** | S | OH | COOH | H | *p* - F |
| **5** | S | OH | $COOCH_3$ | H | H |
| **6** | S | OH | COO $CH_3$ | 2-F | 4-F |
| **7** | S | OH | $COOCH_2CH_3$ | H | H |
| **8** | S | OH | $COOCH_2CH_3$ | H | *p* - Cl |
| **9** | S | OH | $COOCH_2CH_3$ | H | *p* - $CH_3$ |
| **10** | S | OH | $COOCH_2CH_3$ | H | *m* - F |
| **11** | S | OH | $COOCH_2CH_3$ | H | *p* - F |
| **12** | S | OH | $COOCH_2CH_3$ | H | 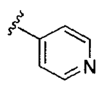 |
| **13** | S | OH | $COOCH_2CH_3$ | H | *p* - $N(CH_3)_2$ |
| **14** | S | OH | $COOCH_2CH_3$ | H | *m* - Cl |
| **15** | S | OH | $COOCH_2CH_3$ | H | *m* - (2-$CF_3$-$C_6H_5$) |
| **16** | S | OH | $COOCH_2CH_3$ | H | *m* - (2-F-$C_6H_5$) |
| **17** | S | OH | $COOCH_2CH_3$ | H | *p*-(2-$CF_3$-$C_6H_5$) |
| **18** | S | OH | $COOCH_2CH_3$ | H | *p* -(2 - F -$C_6H_5$) |
| **19** | S | OH | $COOCH_2CH_3$ | H | *p*-(2-F-$OCH_2C_6H_5$) |
| **20** | S | OH | $COOCH_2CH_3$ | H | *p* - (4-F-O $CH_2C_6H_5$) |
| **21** | S | $F_3CSO_2O$ | $COOCH_2CH_3$ | H | *p* - F |
| **22** | S | $OCH_3$ | $COOCH_2CH_3$ | H | *p* - $CH_3$ |
| **23** | S | 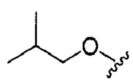 | $COOCH_2CH_3$ | H | *p* - $CH_3$ |
| **24** | S | 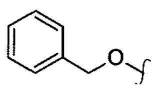 | $COOCH_2CH_3$ | H | H |
| **25** | S | 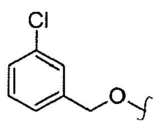 | $COOCH_2CH_3$ | H | *p* - Cl |

(continued)

| Compound | X | $R^1$ | $R^2$ | $R^3$ | $R^4$ |
|---|---|---|---|---|---|
| **26** | S | 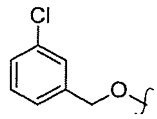 | $COOCH_2CH_3$ | H | *m* - F |
| **27** | S | 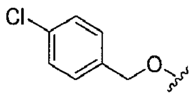 | $COOCH_2CH_3$ | H | H |
| **28** | S | 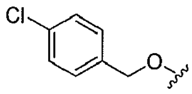 | $COOCH_2CH_3$ | H | *m* - Cl |
| **29** | S | 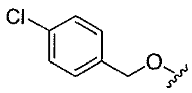 | $COOCH_2CH_3$ | H | *p* - $CH_3$ |
| **30** | S | 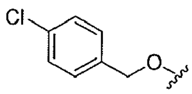 | $COOCH_2CH_3$ | H | *m* - F |
| **31** | S | 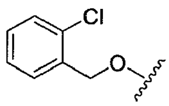 | $COOCH_2CH_3$ | H | H |
| **32** | S | 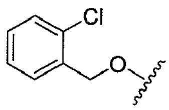 | $COOCH_2CH_3$ | H | *p* - F |
| **33** | S | 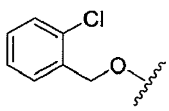 | $COOCH_2CH_3$ | H | *p* - Cl |
| **34** | S | 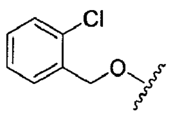 | $COOCH_2CH_3$ | H | *m* - Cl |
| **35** | S | 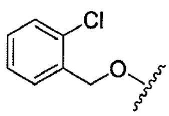 | $COOCH_2CH_3$ | H | *p* - $CH_3$ |
| **36** | S | 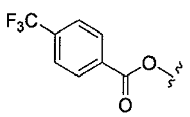 | $COOCH_2CH_3$ | H | H |
| **37** | S | 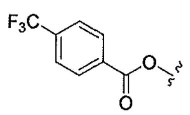 | $COOCH_2CH_3$ | H | *m* - F |
| **38** | S | 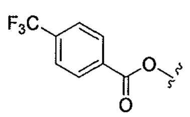 | $COOCH_2CH_3$ | H | *p* - $CH_3$ |

(continued)

| Compound | X | $R^1$ | $R^2$ | $R^3$ | $R^4$ |
|---|---|---|---|---|---|
| **39** | S | 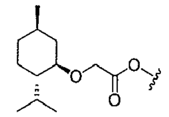 | $COOCH_2CH_3$ | H | *p* - $CH_3$ |
| **40** | S | 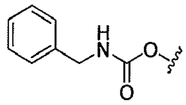 | $COOCH_2CH_3$ | H | *p* - Cl |
| **41** | S | 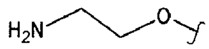 | $COOCH_2CH_3$ | H | *p* - Cl |
| **42** | S | 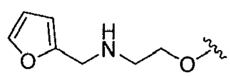 | $COOCH_2CH_3$ | H | *p* - Cl |
| **43** | S | 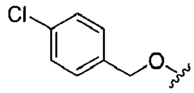 | COOH | H | *p* - $CH_3$ |
| **44** | S | 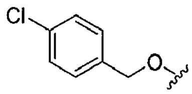 | COOH | H | H |
| **45** | S | 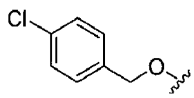 | COOH | H | *p* - Cl |
| **46** | S | 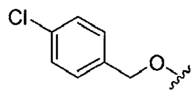 | COOH | H | *m* - Cl |
| **47** | S | 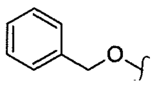 | COOH | H | H |
| **48** | S | 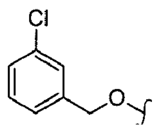 | COOH | H | *m* - F |
| **49** | S | 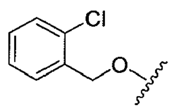 | COOH | H | H |
| **50** | S | 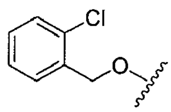 | COOH | H | *p* - F |
| **51** | S | 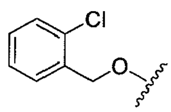 | COOH | H | *p* - Cl |
| **52** | S | 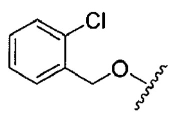 | COOH | H | *m* - Cl |

(continued)

| Compound | X | $R^1$ | $R^2$ | $R^3$ | $R^4$ |
|---|---|---|---|---|---|
| **53** | S | 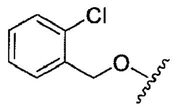 | COOH | H | *p* - $CH_3$ |
| **54** | S | 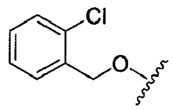 | COOH | H | *m* - F |
| **55** | S | 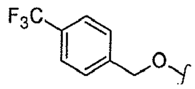 | COOH | H | H |
| **56** | S | 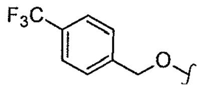 | COOH | H | *m* - F |
| **57** | S | 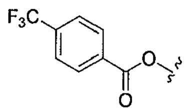 | COOH | H | H |
| **58** | S | 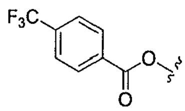 | COOH | H | *m* - F |
| **59** | S | 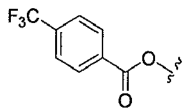 | COOH | H | *p* - $CH_3$ |
| **60** | S | $OCH_3$ | COOH | H | *p* - $CH_3$ |
| **61** | S | 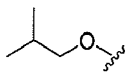 | COOH | H | *p* - $CH_3$ |
| **62** | S | 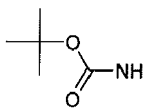 | $COOCH_2CH_3$ | H | *p* - F |
| **63** | S | $NH_2$ | $COOCH_2CH_3$ | H | *p* - F |
| **64** | S | 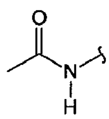 | $COOCH_2CH_3$ | H | *p* - $CH_3$ |
| **65** | S | 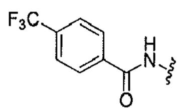 | $COOCH_2CH_3$ | H | *p* - $CH_3$ |
| **66** | S | 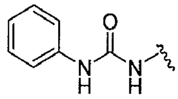 | $COOCH_2CH_3$ | H | *p* - $CH_3$ |
| **67** | S | 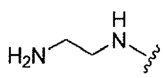 | $COOCH_2CH_3$ | H | *p* - $CH_3$ |

(continued)

| Compound | X | $R^1$ | $R^2$ | $R^3$ | $R^4$ |
|---|---|---|---|---|---|
| **68** | S | 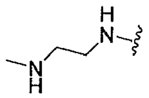 | $COOCH_2CH_3$ | H | *p* - Cl |
| **69** | S | 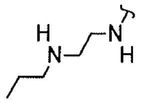 | $COOCH_2CH_3$ | H | *p* - Cl |
| **70** | S | 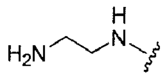 | $COOCH_2CH_3$ | H | *p* - Cl |
| **71** | S | 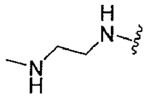 | $COOCH_2CH_3$ | H | *p* - $CH_3$ |
| **72** | S | 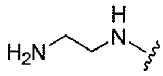 | $COOCH_2CH_3$ | H | *p* - (2-$CF_3$-$C_6H_5$) |
| **73** | S | 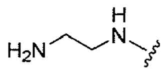 | $COOCH_2CH_3$ | H | *m* - (2-$CF_3$ -$C_6H_5$) |
| **74** | S | 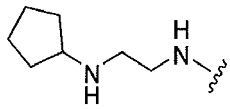 | $COOCH_2CH_3$ | H | *p* - Cl |
| **75** | S | 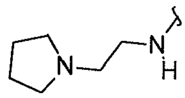 | $COOCH_2CH_3$ | H | H |
| **76** | S | 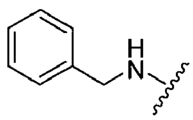 | $COOCH_2CH_3$ | H | *m* - F |
| **77** | S | 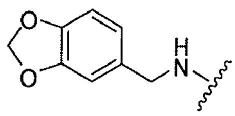 | $COOCH_2CH_3$ | H | *m* - F |
| **78** | S | 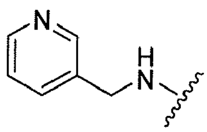 | $COOCH_2CH_3$ | H | *m* - F |
| **79** | S | 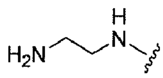 | COOH | H | *p* - $CH_3$ |
| **80** | S | 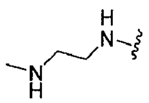 | COOH | H | *p* - $CH_3$ |
| **81** | S | 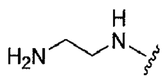 | COOH | H | *m* - F |

(continued)

| Compound | X | R1 | R2 | R3 | R4 |
|---|---|---|---|---|---|
| **82** | S | 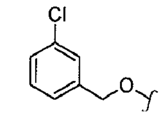 | COONa | H | *m* - F |
| **83** | S | 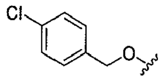 | COONa | H | *p* - $CH_3$ |
| **84** | S | 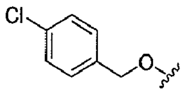 | COONa | H | *p* - Cl |
| **85** | S | 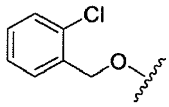 | COONa | H | *p* - Cl |
| **86** | S | 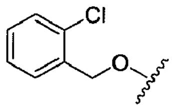 | COONa | H | *p* - $CH_3$ |
| **87** | S | 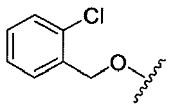 | COONa | H | *m* - F |
| **88** | S | 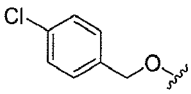 | COONa | H | *m* - F |
| **89** | S | 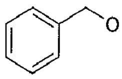 | 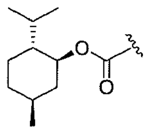 | H | *p* - $CH_3$ |
| **90** | S | OH | 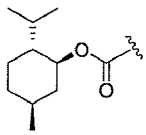 | H | *p* - $CH_3$ |
| **91** | S | 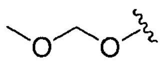 | $COOCH_2CH_3$ | H | *p* - Cl |
| **92** | S | 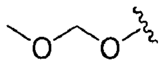 | COOH | H | *p* - Cl |
| **93** | S | 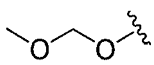 | $C(O)NH_2$ | H | *p* - Cl |
| **94** | S | 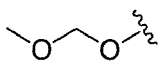 | CN | H | *p* - Cl |
| **95** | S | OH | 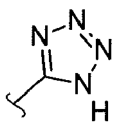 | H | *p* - Cl |

(continued)

| Compound | X | $R^1$ | $R^2$ | $R^3$ | $R^4$ |
|---|---|---|---|---|---|
| **96** | S | OH | 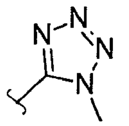 | H | *p* - Cl |
| **97** | S | OH | 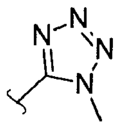 | H | *m* - F |
| **98** | S | OH | 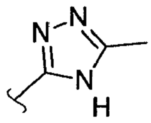 | H | *p* - Cl |
| **99** | S | OH | 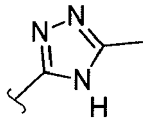 | H | *m* - F |
| **100** | S | OH | 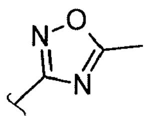 | H | *p* - Cl |
| **101** | S | OH | 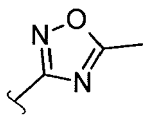 | H | *m* - F |
| **102** | S | 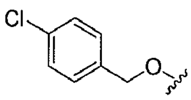 | 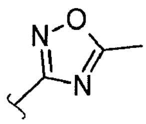 | H | *m* - F |
| **103** | S | OH | 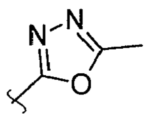 | H | *p* - Cl |
| **104** | S | OH | 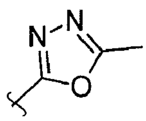 | H | *m* - F |
| **105** | O | OH | $COOCH_2CH_3$ | H | H |
| **106** | O | OH | $COOCH_2CH_3$ | H | *m* - F |
| **107** | O | OH | $COOCH_2CH_3$ | H | *p* - $CH_3$ |
| **108** | O | 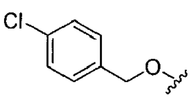 | $COOCH_2CH_3$ | H | H |
| **109** | O | 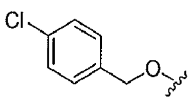 | $COOCH_2CH_3$ | H | *m* - F |

| | | | | | |
|---|---|---|---|---|---|
| **110** | O | 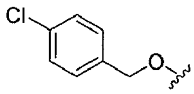 | $COOCH_2CH_3$ | H | *p* - $CH_3$ |

(continued)

| Compound | X | $R^1$ | $R^2$ | $R^3$ | $R^4$ |
|---|---|---|---|---|---|
| **111** | O | 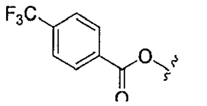 | $COOCH_2CH_3$ | H | H |
| **112** | O | 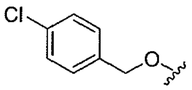 | COOH | H | H |
| **113** | O | 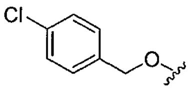 | COOH | H | *m* - F |
| **114** | O | 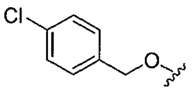 | COOH | H | *p* - $CH_3$ |
| **115** | O | OH | 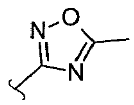 | H | *m* - F |
| **116** | O | 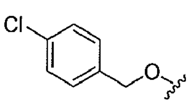 | 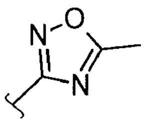 | H | *m* - F |
| **117** | S | $OCOCH_2CH_3$ | OH | H | *p* - $CH_3$ |

## Claims

**1.** Compound having formula I:

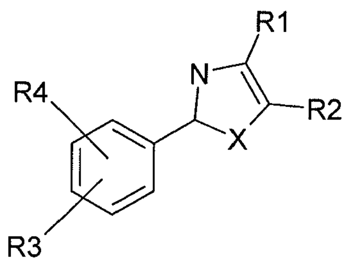

**(I)**

and pharmaceutically acceptable salts thereof,
wherein:

**X** is selected from S or O;
**$R_1$** is selected from the group consisting of:
**-$OR_5$** wherein $R_5$ is selected from H; $C_1$-$C_4$ alkyl, trifluoromethanesulfonyl, benzyl, (trifluoromethyl)benzyl, (halo) benzyl, (trifluoromethyl)benzoyl, N-benzylcarbamoyl, cyclohexyloxyacetoyl substituted with at least one $C_1$-$C_3$ alkyl group, ($C_1$-$C_3$ alkoxy)methyl, $C_1$-$C_3$ alkanoyl and $CH_2CH_2NHR_6$, wherein
$R_6$ is selected from H and (furan-2-yl)methyl;
**-$NHR_7$** wherein $R_7$ is selected from H, tert-butoxycarbonyl, $C_1$-$C_3$ alkanoyl, (4-trifluoromethyl)benzoyl, N-phenylaminoacarbonyl , $CH_2R_8$, wherein
$R_8$ is selected from phenyl, benzo[d][1,3] dioxole, pyridin-3-yl, (pyrrolidin-1-yl)methyl, -$CH_2NHR_9$ wherein

$R_9$ is selected from H, $C_1$-$C_3$ alkyl and cycloalkyl;
**$R_2$** is selected from the group consisting of
-$COOR_{10}$ wherein
$R_{10}$ is selected from H, $C_1$-$C_3$ alkyl and cyclohexyloxycarbonyl substituted with at least one $C_1$-$C_3$ alkyl group;
-OH; -$CONH_2$; CN
-tetrazol-5-yl or 1-($C_1$-$C_3$ alkyl)tetrazol-5-yl,
-5-($C_1$-$C_3$ alkyl)1,2,4 triazol-3-yl,
- 5-($C_1$-$C_3$ alkyl) 1,2,4- oxadiazol-3yl,
-5-($C_1$-$C_3$ alkyl) 1,3,4-oxadiazol-2-yl;
**$R_3$** is selected from F or H,
**$R_4$** is selected from H, $CH_3$, halogen, dimethylamino, pyridin-4yl, phenyl, 2- or 4- (halo)penyl, 2- or 4-(trifluoromethyl)phenyl and 2- and/or 4-halobenzyloxy
**f**or use in the prevention and/or treatment of a disease associated with the activity of TRPM8.

**2.** Compound of formula I:

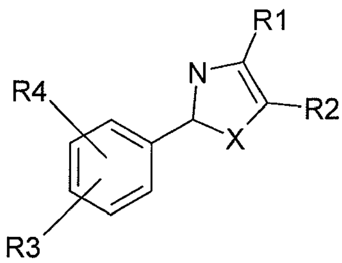

**(I)**

and pharmaceutically acceptable salts thereof,
wherein:

X is selected from S or O;
**$R_1$** is selected from the group consisting of:
-**$OR_5$** wherein $R_5$ is selected from H; $C_1$-$C_4$ alkyl, trifluoromethanesulfonyl, benzyl, (trifluoromethyl)benzyl, (halo) benzyl, (trifluoromethyl)benzoyl, N-benzylcarbamoyl, cyclohexyloxyacetoyl substituted with at least one $C_1$-$C_3$ alkyl group, ($C_1$-$C_3$ alkoxy)methyl, $C_1$-$C_3$ alkanoyl and $CH_2CH_2NHR_6$, wherein
$R_6$ is selected from H and (furan-2-yl)methyl;
-**$NHR_7$** wherein $R_7$ is selected from H, tert-butoxycarbonyl, $C_1$-$C_3$ alkanoyl, (4-trifluoromethyl)benzoyl, N-phenylaminoacarbonyl , $CH_2R_7$, wherein
$R_8$ is selected from phenyl, benzo[d][1,3] dioxole, pyridin-3-yl, (pyrrolidin-1-yl)methyl, -$CH_2NHR_8$ wherein
$R_9$ is selected from H, $C_1$-$C_3$ alkyl and cycloalkyl;
**$R_2$** is selected from the group consisting of
-$COOR_{10}$ wherein
$R_{10}$ is selected from H, $C_1$-$C_3$ alkyl and cyclohexyloxycarbonyl substituted with at least one $C_1$-$C_3$ alkyl group;
-OH; -$CONH_2$; CN
-tetrazol-5-yl or 1-($C_1$-$C_3$ alkyl)tetrazol-5-yl,
-5-($C_1$-$C_3$ alkyl)1,2,4 triazol-3-yl,
- 5-($C_1$-$C_3$ alkyl) 1,2,4- oxadiazol-3yl,
-5-($C_1$-$C_3$ alkyl) 1,3,4-oxadiazol-2-yl;
**$R_3$** is selected from F or H,
**$R_4$** is selected from H, $CH_3$, halogen, dimethylamino, pyridin-4yl, phenyl, 2- or 4- (halo)penyl, 2- or 4-(trifluoromethyl)phenyl, 2- and/or 4-halobenzyloxy;
with the proviso that when X is S:
when $R_1$ is OH and $R_2$ is COOH, $R_4$ is not Cl in meta position on the aromatic ring; and
when $R_1$ is OH and $R_2$ is COOH, $R_3$ and $R_4$ cannot be H at the same time;
for use as a medicament.

**3.** A compound as claimed in claim 1 or 2 wherein

when $R_3$ is F, $R_3$ is in position ortho of the aromatic ring and $R_4$ is F in position para of the aromatic ring.

**4.** A compound as claimed in claims 1 or 2 wherein
when $R_3$ is H, $R_4$ is in position para or meta on the aromatic ring.

**5.** A compound as claimed in anyone of claims 1 to 4 wherein
$R_5$ is selected from H; $C_1$-$C_4$ alkyl; trifluoromethanesulfonyl; benzyl; (trifluoromethyl)benzyl, (chloro)benzyl; (trifluoromethyl)benzoyl, N-benzylcarbamoyl, cyclohexyloxyacetoyl substituted with at least one $C_1$-$C_3$ alkyl group, (methoxy)methyl, propanoyl and $-CH_2CH_2NHR_6$;
wherein $R_6$ is selected from H and (furan-2-yl)methyl;

**6.** A compound as claimed in anyone of claims 1 to 5 wherein
$R_7$ is selected from H, tert-butoxycarbonyl, acetyl, (4-trifluoromethyl)benzoyl; N-phenylaminoacarbonyl, $CH_2R_8$, wherein
$R_8$ is selected from phenyl, benzo[d][1,3] dioxole, pyridin-3-yl, (pyrrolidin-1-yl)methyl, $-CH_2NHR_9$ wherein
$R_9$ is selected from H, $C_1$-$C_3$ alkyl and cyclopentyl.

**7.** A compound as claimed in anyone of claims 1 to 6 wherein:

$R_{10}$ is selected from H; $C_1$-$C_3$ alkyl and 2-isopropyl-5-methyl-cyclohexyl.

**8.** A compounds as claimed anyone of claims 1 to 7 wherein:

$R_4$ is selected from H; $CH_3$; F; Cl; dimethylamino; pyridin-4yl; phenyl, 2-F-penyl, 2-trifluoromethylphenyl; 2- or 4-halobenzyloxy.

**9.** A compound as claimed in anyone of claims 1 to 8 wherein
$R_1$ i selected from:

$OR_5$, wherein $R_5$ is selected from H, benzyl, (chloro)benzyl, (trifluoromethyl)benzoyl; and
$CH_2$-$CH_2NH_2$; and
$NHCH_2CH_2CH_2R_8$ wherein $R_8$ is selected from $C_1$-$C_3$ alkyl and H;

**10.** A compound as claimed in claims 1 to 9 wherein $R_2$ is $COOR_{10}$ wherein $R_{10}$ is selected from H, $C_1$-$C_3$ alkyl.

**11.** A compound as claimed in claims 1 to 10 wherein $R_3$ is H.

**12.** A compounds as claimed in claim 1 selected from:

2-(4-chlorophenyl)-4-hydroxy-1,3-thiazole-5-carboxylic acid (1)
4-hydroxy-2-(4-methylphenyl)-1,3-thiazole-5-carboxylic acid (2)
2-(3-fluorophenyl)-4-hydroxy-1,3-thiazole-5-carboxylic acid (3)
2-(4-fluorophenyl)-4-hydroxy-1,3-thiazole-5-carboxylic acid (4)
methyl 4-hydroxy-2-phenyl-1,3-thiazole-5-carboxylate (5)
methyl 2-(2,4-difluorophenyl)-4-hydroxy-1 ,3-thiazole-5-carboxylate (6)
ethyl 4-hydroxy-2-phenyl-1,3-thiazole-5-carboxylate (7)
ethyl 2-(4-chlorophenyl)-4-hydroxy-1,3-thiazole-5-carboxylate (8)
ethyl 4-hydroxy-2-(4-methylphenyl)-1,3-thiazole-5-carboxylate (9)
ethyl 2-(3-fluorophenyl)-4-hydroxy-1,3-thiazole-5-carboxylate (10)
ethyl 2-(4-fluorophenyl)-4-hydroxy-1 ,3-thiazole-5-carboxylate (11)
ethyl 4-hydroxy-2-(pyridin-4-yl)-1,3-thiazole-5-carboxylate (12)
ethyl 2-[4-(dimethylamino)phenyl]-4-hydroxy-1,3-thiazole-5-carboxylate (13)
ethyl 2-(3-chlorophenyl)-4-hydroxy-1,3-thiazole-5-carboxylate (14)
ethyl 4-hydroxy-2-[2'-(trifluoromethyl)biphenyl-3-yl]-1,3-thiazole-5-carboxylate (15)
ethyl 2-(2'-fluorobiphenyl-3-yl)-4-hydroxy-1,3-thiazole-5-carboxylate (16)
ethyl 4-hydroxy-2-[2'-(trifluoromethyl)biphenyl-4-yl]-1,3-thiazole-5-carboxylate (17)
ethyl 2-(2'-fluorobiphenyl-4-yl)-4-hydroxy-1,3-thiazole-5-carboxylate (18)
ethyl 2-{4-[(2-fluorobenzyl)oxy]phenyl}-4-hydroxy-1,3-thiazole-5-carboxylate (19)

ethyl 2-{4-[(4-fluorobenzyl)oxy]phenyl}-4-hydroxy-1,3-thiazole-5-carboxylate (20)
ethyl 2-(4-fluorophenyl)-4-{[(trifluoromethyl)sulfonyl]oxy}-1,3-thiazole-5-carboxylate (21)
ethyl 4-methoxy-2-(4-methylphenyl)-1,3-thiazole-5-carboxylate (22)
ethyl 2-(4-methylphenyl)-4-(2-methylpropoxy)-1,3-thiazole-5-carboxylate (23)
ethyl 4-(benzyloxy)-2-phenyl-1,3-thiazole-5-carboxylate (24)
ethyl 4-[(3-chlorobenzyl)oxy]-2-(4-chlorophenyl)-1,3-thiazole-5-carboxylate (25)
ethyl 4-[(3-chlorobenzyl)oxy]-2-(3-fluorophenyl)-1,3-thiazole-5-carboxylate (26)
ethyl 4-[(4-chlorobenzyl)oxy]-2-phenyl-1,3-thiazole-5-carboxylate (27)
ethyl 4-[(4-chlorobenzyl)oxy]-2-(3-chlorophenyl)-1,3-thiazole-5-carboxylate (28)
ethyl 4-[(4-chlorobenzyl)oxy]-2-(4-methylphenyl)-1,3-thiazole-5-carboxylate (29)
ethyl 4-[(4-chlorobenzyl)oxy]-2-(3-fluorophenyl)-1,3-thiazole-5-carboxylate (30)
ethyl 4-[(2-chlorobenzyl)oxy]-2-phenyl-1,3-thiazole-5-carboxylate (31)
ethyl 4-[(2-chlorobenzyl)oxy]-2-(4-fluorophenyl)-1,3-thiazole-5-carboxylate (32)
ethyl 4-[(2-chlorobenzyl)oxy]-2-(4-chlorophenyl)-1,3-thiazole-5-carboxylate (33)
ethyl 4-[(2-chlorobenzyl)oxy]-2-(3-chlorophenyl)-1,3-thiazole-5-carboxylate (34)
ethyl 4-[(2-chlorobenzyl)oxy]-2-(4-methylphenyl)-1,3-thiazole-5-carboxylate (35)
ethyl 2-phenyl-4-{[4-(trifluoromethyl)benzoyl]oxy}1,3-thiazole-5-carboxylate (36)
ethyl 2-(3-fluorophenyl)-4-{[4-(trifluoromethyl)benzoyl]oxy}-1,3-thiazole-5-carboxylate (37)
ethyl 2-(4-methylphenyl)-4-{[4-(trifluoromethyl)benzoyl]oxy}-1,3-thiazole-5-carboxylate (38)
ethyl4-(2-((1R,2S,5R)-2-isopropyl-5-methylcyclohexyloxy)acetoyloxy)-2-(4-methylphenyl) -1,3-thiazole-5-carboxylate (39)
ethyl 4-[(benzylcarbamoyl)oxy]-2-(4-chlorophenyl)-1,3-thiazole-5-carboxylate (40) ethyl 4-(2-aminoethoxy)-2-(4-chlorophenyl)-1,3-thiazole-5-carboxylate (41)
ethyl 2-(4-chlorophenyl)-4-{2-[(furan-2-ylmethyl)amino]ethoxy}-1,3-thiazole-5- carboxylate (42)
4-[(4-chlorobenzyl)oxy]-2-(4-methylphenyl)-1,3-thiazole-5-carboxylic acid (43)
4-[(4-chlorobenzyl)oxy]-2-phenyl-1,3-thiazole-5-carboxylic acid (44)
4-[(4-chlorobenzyl)oxy]-2-(4-chlorophenyl)-1,3-thiazole-5-carboxylic acid (45)
4-[(4-chlorobenzyl)oxy]-2-(3-chlorophenyl)-1,3-thiazole-5-carboxylic acid (46)
4-(benzyloxy)-2-phenyl-1,3-thiazole-5-carboxylic acid (47)
4-[(3-chlorobenzyl)oxy]-2-(3-fluorophenyl)-1,3-thiazole-5-carboxylic acid (48)
4-[(2-chlorobenzyl)oxy]-2-phenyl-1,3-thiazole-5-carboxylic acid (49)
4-[(2-chlorobenzyl)oxy]-2-(4-fluorophenyl)-1,3-thiazole-5-carboxylic acid (50)
4-[(2-chlorobenzyl)oxy]-2-(4-chlorophenyl)-1,3-thiazole-5-carboxylic acid (51)
4-[(2-chlorobenzyl)oxy]-2-(3-chlorophenyl)-1,3-thiazole-5-carboxylic acid (52)
4-[(2-chlorobenzyl)oxy]-2-(4-methylphenyl)-1,3-thiazole-5-carboxylic acid (53)
4-[(2-chlorobenzyl)oxy]-2-(3-fluorophenyl)-1,3-thiazole-5-carboxylic acid (54)
2-phenyl-4-{[4-(trifluoromethyl)benzyl]oxy}-1,3-thiazole-5-carboxylic acid (55)
2-(3-fluorophenyl)-4-{[4-(trifluoromethyl)benzyl]oxy}-1,3-thiazole-5-carboxylic acid (56)
2-phenyl-4-{[4-(trifluoromethyl)benzoyl]oxy}-1,3-thiazole-5-carboxylic acid (57)
2-(3-fluorophenyl)-4-{[4-(trifluoromethyl)benzoyl]oxy}1,3-thiazole-5-carboxylic acid (58)
2-(4-methylphenyl)-4-{[4-(trifluoromethyl)benzoyl]oxy}1,3-thiazole-5-carboxylic acid (59)
4-methoxy-2-(4-methylphenyl)-1,3-thiazole-5-carboxylic acid (60)
2-(4-methylphenyl)-4-(2-methylpropoxy)-1,3-thiazole-5-carboxylic acid (61)
ethyl 4-[(tert-butoxycarbonyl)amino]-2-(4-fluorophenyl)-1,3-thiazole-5-carboxylate (62)
ethyl 4-amino-2-(4-fluorophenyl)-1,3-thiazole-5-carboxylate hydrochloride (63)
ethyl 4-(acetylamino)-2-(4-methylphenyl)-1,3-thiazole-5-carboxylate (64)
ethyl 2-(4-methylphenyl)-4-{[4-(trifluoromethyl)benzoyl]amino}-1,3-thiazole-5-carboxylate (65)
ethyl 2-(4-methylphenyl)-4-[(phenylcarbamoyl)amino]-1,3-thiazole-5-carboxylate (66)
ethyl 4-[(2-aminoethyl)amino]-2-(4-methylphenyl)-1,3-thiazole-5-carboxylate (67)
ethyl 2-(4-chlorophenyl)-4-{[2-(methylamino)ethyl]amino}-1,3-thiazole-5-carboxylate (68)
ethyl 2-(4-chlorophenyl)-4-{[2-(propylamino)ethyl]amino}1,3-thiazole-5-carboxylate (69)
ethyl 4-[(2-aminoethyl)amino]-2-(4-chlorophenyl)-1,3-thiazole-5-carboxylate (70)
ethyl 4-{[2-(methylamino)ethyl]amino}-2-(4-methylphenyl)-1,3-thiazole-5-carboxylate (71)
ethyl 4-[(2-aminoethyl)amino]-2-[2'-(trifluoromethyl)biphenyl-4-yl]-1,3-thiazole-5-carboxylate (72)
ethyl 4-[(2-aminoethyl)amino]-2-[2'-(trifluoromethyl)biphenyl-3-yl]-1,3-thiazole-5-carboxylate (73)
ethyl 2-(4-chlorophenyl)-4-{[2-(cyclopentylamino)ethyl]amino}-1,3-thiazole-5-carboxylate (74)
ethyl 2-phenyl-4-{[2-(pyrrolidin-1-yl)ethyl]amino}-1,3-thiazole-5-carboxylate (75)
ethyl 4-(benzylamino)-2-(3-fluorophenyl)-1,3-thiazole-5-carboxylate (76)

ethyl 4-[(1,3-benzodioxol-5-ylmethyl)amino]-2-(3-fluorophenyl)-1,3-thiazole-5-carboxylate (77)
ethyl 2-(3-fluorophenyl)-4-[(pyridin-3-ylmethyl)amino]-1,3-thiazole-5-carboxylate (78)
4-[(2-aminoethyl)amino]-2-(4-methylphenyl)-1,3-thiazole-5-carboxylic acid (79)
4-{[2-(methylamino)ethyl]aminoy2-(4-methylphenyl)-1,3-thiazole-5-carboxylic acid (80)
4-[(2-aminoethyl)amino]-2-(3-fluorophenyl)-1,3-thiazole-5-carboxylic acid (81)
sodium 4-[(3-chlorobenzyl)oxy]-2-(3-fluorophenyl)-1,3-thiazole-5-carboxylate (82)
sodium 4-[(4-chlorobenzyl)oxy]-2-(4-methylphenyl)-1,3-thiazole-5-carboxylate (83)
sodium 4-(4-chlorobenzyloxy)-2-(4-chlorophenyl)-1,3-thiazole-5-carboxylate (84)
sodium 4-(2-chlorobenzyloxy)-2-(4-chlorophenyl)-1,3-thiazole-5-carboxylate (85)
sodium 4-(2-chlorobenzyloxy)-2-(4-methylphenyl)-1,3-thiazole-5-carboxylate (86)
sodium 4-(2-chlorobenzyloxy)-2-(3-fluorophenyl)-1,3-thiazole-5-carboxylate (87)
sodium 4-(4-chlorobenzyloxy)-2-(3-fluorophenyl)-1,3-thiazole-5-carboxylate (88)
(1R,2S,5R)-2-isopropyl-5-methylcyclohexyl-4-(benzyloxy)-2-(4-methylphenyl)-1,3-thiazole-5-carboxylate (89)
(1R,2S,5R)-2-isopropyl-5-methylcyclohexyl-4-hydroxy-2-(4-methylphenyl)-1,,3-thiazole-5-carboxylate (90)
ethyl 2-(4-chlorophenyl)-4-(methoxymethoxy)-1,3-thiazole-5-carboxylate (91)
2-(4-chlorophenyl)-4-(methoxymethoxy)-1,3-thiazole-5-carboxylic acid (92)
2-(4-chlorophenyl)-4-(methoxymethoxy)-1,3-thiazole-5-carboxamide (93)
2-(4-chlorophenyl)-4-(methoxymethoxy)-1,3-thiazole-5-carbonitrile (94)
2-(4-chlorophenyl)-5-(1H-tetrazol-5-yl)-1,3-thiazol-4-ol (95)
2-(4-chlorophenyl)-5-(1-methyl-1H-tetrazol-5-yl)-1,3-thiazol-4-ol (96)
2-(3-fluorophenyl)-5-(1-methyl-1H-tetrazol-5-yl)-1,3-thiazol-4-ol (97)
2-(4-chlorophenyl)-5-(5-methyl-4H-1,2,4-triazol-3-yl)-1,3-thiazol-4-ol (98)
2-(3-fluorophenyl)-5-(5-methyl-4H-1,2,4-triazol-3-yl)-1,3-thiazol-4-ol (99)
2-(4-chlorophenyl)-5-(5-methyl-1,2,4-oxadiazol-3-yl)-1,3-thiazol-4-ol (100)
2-(3-fluorophenyl)-5-(5-methyl-1,2,4-oxadiazol-3-yl)-1,3-thiazol-4-ol (101)
3-{4-[(4-chlorobenzyl)oxy]-2-(3-fluorophenyl)-1,3-thiazol-5-yl}-5-methyl-1,2,4-oxadiazole (102)
2-(4-chlorophenyl)-5-(5-methyl-1,3,4-oxadiazol-2-yl)-1,3-thiazol-4-ol (103)
2-(3-fluorophenyl)-5-(5-methyl-1,3,4-oxadiazol-2-yl)-1,3-thiazol-4-ol (104)
ethyl 4-hydroxy-2-phenyl-1,3-oxazole-5-carboxylate (105)
ethyl 2-(3-fluorophenyl)-4-hydroxy-1,3-oxazole-5-carboxylate (106)
ethyl 4-hydroxy-2-(4-methylphenyl)-1,3-oxazole-5-carboxylate (107)
ethyl 4-[(4-chlorobenzyl)oxy]-2-phenyl-1,3-oxazole-5-carboxylate (108)
ethyl 4-[(4-chlorobenzyl)oxy]-2-(3-fluorophenyl)-1,3-oxazole-5-carboxylate (109)
ethyl 4-[(4-chlorobenzyl)oxy]-2-(4-methylphenyl)-1,3-oxazole-5-carboxylate (110)
ethyl 2-phenyl-4-{[4-(trifluoromethyl)benzoyl]oxy}-1,3-oxazole-5-carboxylate (111)
4-[(4-chlorobenzyl)oxy]-2-phenyl-1,3-oxazole-5-carboxylic acid (112)
4-[(4-chlorobenzyl)oxy]-2-(3-fluorophenyl)-1,3-oxazole-5-carboxylic acid (113)
4-[(4-chlorobenzyl)oxy]-2-(4-methylphenyl)-1,3-oxazole-5-carboxylic acid (114)
2-(3-fluorophenyl)-5-(5-methyl-1,2,4-oxadiazol-3-yl)-1,3-oxazol-4-ol(115)
3-{4-[(4-chlorobenzyl)oxy]-2-(3-fluorophenyl)-1,3-oxazol-5-yl}-5-methyl-1,2,4-oxadiazole (116)
ethyl 2-(3-fluorophenyl)-5-hydroxy-1,3-thiazole-4-carboxylate (117).

**13.** A compound as claimed in claim 2, selected from:

2-(4-chlorophenyl)-4-hydroxy-1,3-thiazole-5-carboxylic acid (1)
4-hydroxy-2-(4-methylphenyl)-1,3-thiazole-5-carboxylic acid (2)
2-(3-fluorophenyl)-4-hydroxy-1,3-thiazole-5-carboxylic acid (3)
2-(4-fluorophenyl)-4-hydroxy-1,3-thiazole-5-carboxylic acid (4)
methyl 4-hydroxy-2-phenyl-1,3-thiazole-5-carboxylate (5)
methyl 2-(2,4-difluorophenyl)-4-hydroxy-1,3-thiazole-5-carboxylate (6)
ethyl 2-(3-fluorophenyl)-4-hydroxy-1,3-thiazole-5-carboxylate (10)
ethyl 2-(4-fluorophenyl)-4-hydroxy-1,3-thiazole-5-carboxylate (11)
ethyl 4-hydroxy-2-(pyridin-4-yl)-1,3-thiazole-5-carboxylate (12)
ethyl 2-[4-(dimethylamino)phenyl]-4-hydroxy-1,3-thiazole-5-carboxylate (13)
ethyl 4-hydroxy-2-[2'-(trifluoromethyl)biphenyl-3-yl]-1,3-thiazole-5-carboxylate (15)
ethyl 2-(2'-fluorobiphenyl-3-yl)-4-hydroxy-1,3-thiazole-5-carboxylate (16)
ethyl 4-hydroxy-2-[2'-(trifluoromethyl)biphenyl-4-yl]-1,3-thiazole-5-carboxylate (17)
ethyl 2-(2'-fluorobiphenyl-4-yl)-4-hydroxy-1,3-thiazole-5-carboxylate (18)

ethyl 2-{4-[(2-fluorobenzyl)oxy]phenyl}-4-hydroxy-1,3-thiazole-5-carboxylate (19)
ethyl 2-{4-[(4-fluorobenzyl)oxy]phenyl}-4-hydroxy-1,3-thiazole-5-carboxylate (20)
ethyl 2-(4-fluorophenyl)-4-{[(trifluoromethyl)sulfonyl]oxy}-1,3-thiazole-5-carboxylate (21)
ethyl 4-methoxy-2-(4-methylphenyl)-1,3-thiazole-5-carboxylate (22)
ethyl 2-(4-methylphenyl)-4-(2-methylpropoxy)-1,3-thiazole-5-carboxylate (23)
ethyl 4-(benzyloxy)-2-phenyl-1,3-thiazole-5-carboxylate (24)
ethyl 4-[(3-chlorobenzyl)oxy]-2-(4-chlorophenyl)-1,3-thiazole-5-carboxylate (25)
ethyl 4-[(3-chlorobenzyl)oxy]-2-(3-fluorophenyl)-1,3-thiazole-5-carboxylate (26)
ethyl 4-[(4-chlorobenzyl)oxy]-2-(4-methylphenyl)-1,3-thiazole-5-carboxylate (29)
ethyl 4-[(4-chlorobenzyl)oxy]-2-(3-fluorophenyl)-1,3-thiazole-5-carboxylate (30)
ethyl 4-[(2-chlorobenzyl)oxy]-2-(4-fluorophenyl)-1,3-thiazole-5-carboxylate (32)
ethyl 4-[(2-chlorobenzyl)oxy]-2-(4-chlorophenyl)-1,3-thiazole-5-carboxylate (33)
ethyl 4-[(2-chlorobenzyl)oxy]-2-(3-chlorophenyl)-1,3-thiazole-5-carboxylate (34)
ethyl 4-[(2-chlorobenzyl)oxy]-2-(4-methylphenyl)-1,3-thiazole-5-carboxylate (35)
ethyl 2-phenyl-4-{[4-(trifluoromethyl)benzoyl]oxy}-1,3-thiazole-5-carboxylate (36)
ethyl 2-(3-fluorophenyl)-4-{[4-(trifluoromethyl)benzoyl]oxy}-1,3-thiazole-5-carboxylate (37)
ethyl 2-(4-methylphenyl)-4-{[4-(trifluoromethyl)benzoyl]oxy}-1,3-thiazole-5-carboxylate (38)
ethyl4-(2-((1R,2S,5R)-2-isopropyl-5-methylcyclohexyloxy)acetoyloxy)-2-(4-methylphenyl) -1,3-thiazole-5-carboxylate (39)
ethyl 4-[(benzylcarbamoyl)oxy]-2-(4-chlorophenyl)-1,3-thiazole-5-carboxylate (40)
ethyl 4-(2-aminoethoxy)-2-(4-chlorophenyl)-1,3-thiazole-5-carboxylate (41)
ethyl 2-(4-chlorophenyl)-4-{2-[(furan-2-ylmethyl)amino]ethoxy}-1,3-thiazole-5- carboxylate (42)
4-[(4-chlorobenzyl)oxy]-2-(4-methylphenyl)-1,3-thiazole-5-carboxylic acid (43)
4-[(4-chlorobenzyl)oxy]-2-phenyl-1,3-thiazole-5-carboxylic acid (44)
4-[(4-chlorobenzyl)oxy]-2-(4-chlorophenyl)-1,3-thiazole-5-carboxylic acid (45)
4-[(4-chlorobenzyl)oxy]-2-(3-chlorophenyl)-1,3-thiazole-5-carboxylic acid (46)
4-(benzyloxy)-2-phenyl-1,3-thiazole-5-carboxylic acid (47)
4-[(3-chlorobenzyl)oxy]-2-(3-fluorophenyl)-1,3-thiazole-5-carboxylic acid (48)
4-[(2-chlorobenzyl)oxy]-2-phenyl-1,3-thiazole-5-carboxylic acid (49)
4-[(2-chlorobenzyl)oxy]-2-(4-fluorophenyl)-1,3-thiazole-5-carboxylic acid (50)
4-[(2-chlorobenzyl)oxy]-2-(4-chlorophenyl)-1,3-thiazole-5-carboxylic acid (51)
4-[(2-chlorobenzyl)oxy]-2-(3-chlorophenyl)-1,3-thiazole-5-carboxylic acid (52)
4-[(2-chlorobenzyl)oxy]-2-(4-methylphenyl)-1,3-thiazole-5-carboxylic acid (53)
4-[(2-chlorobenzyl)oxy]-2-(3-fluorophenyl)-1,3-thiazole-5-carboxylic acid (54)
2-phenyl-4-{[4-(trifluoromethyl)benzyl]oxy}-1,3-thiazole-5-carboxylic acid (55)
2-(3-fluorophenyl)-4-{[4-(trifluoromethyl)benzyl]oxy}-1,3-thiazole-5-carboxylic acid (56)
2-phenyl-4-{[4-(trifluoromethyl)benzoyl]oxy}-1,3-thiazole-5-carboxylic acid (57)
2-(3-fluorophenyl)-4-{[4-(trifluoromethyl)benzoyl]oxy}-1,3-thiazole-5-carboxylic acid (58)
2-(4-methylphenyl)-4-{[4-(trifluoromethyl)benzoyl]oxy}-1,3-thiazole-5-carboxylic acid (59)
4-methoxy-2-(4-methylphenyl)-1,3-thiazole-5-carboxylic acid (60)
2-(4-methylphenyl)-4-(2-methylpropoxy)-1,3-thiazole-5-carboxylic acid (61)
ethyl 4-[(tert-butoxycarbonyl)amino]-2-(4-fluorophenyl)-1,3-thiazole-5-carboxylate (62)
ethyl 4-amino-2-(4-fluorophenyl)-1,3-thiazole-5-carboxylate hydrochloride (63)
ethyl 4-(acetylamino)-2-(4-methylphenyl)-1,3-thiazole-5-carboxylate (64)
ethyl 2-(4-methylphenyl)-4-{[4-(trifluoromethyl)benzoyl]amino}-1,3-thiazole-5-carboxylate (65)
ethyl 2-(4-methylphenyl)-4-[(phenylcarbamoyl)amino]-1,3-thiazole-5-carboxylate (66)
ethyl 4-[(2-aminoethyl)amino]-2-(4-methylphenyl)-1,3-thiazole-5-carboxylate (67)
ethyl 2-(4-chlorophenyl)-4-{[2-(methylamino)ethyl]amino}-1,3-thiazole-5-carboxylate (68)
ethyl 2-(4-chlorophenyl)-4-{[2-(propylamino)ethyl]amino}-1,3-thiazole-5-carboxylate (69)
ethyl 4-[(2-aminoethyl)amino]-2-(4-chlorophenyl)-1,3-thiazole-5-carboxylate (70)
ethyl 4-{[2-(methylamino)ethyl]amino}-2-(4-methylphenyl)-1,3-thiazole-5-carboxylate (71)
ethyl 4-[(2-aminoethyl)amino]-2-[2'-(trifluoromethyl)biphenyl-4-yl]-1,3-thiazole-5-carboxylate (72)
ethyl 4-[(2-aminoethyl)amino]-2-[2'-(trifluoromethyl)biphenyl-3-yl]-1,3-thiazole-5-carboxylate (73)
ethyl 2-(4-chlorophenyl)-4-{[2-(cyclopentylamino)ethyl]amino}-1,3-thiazole-5-carboxylate (74)
ethyl 2-phenyl-4-{[2-(pyrrolidin-1-yl)ethyl]amino}-1,3-thiazole-5-carboxylate (75)
ethyl 4-(benzylamino)-2-(3-fluorophenyl)-1,3-thiazole-5-carboxylate (76)
ethyl 4-[(1,3-benzodioxol-5-ylmethyl)amino)-2-(3-fluorophenyl)-1,3-thiazole-5-carboxylate (77)
ethyl 2-(3-fluorophenyl)-4-[(pyridin-3-ylmethyl)amino]-1,3-thiazole-5-carboxylate (78)

4-[(2-aminoethyl)amino]-2-(4-methylphenyl)-1,3-thiazole-5-carboxylic acid (79)
4-{[2-(methylamino)ethyl]amino}-2-(4-methylphenyl)-1,3-thiazole-5-carboxylic acid (80)
4-[(2-aminoethyl)amino]-2-(3-fluorophenyl)-1,3-thiazole-5-carboxylic acid (81)
sodium 4-[(3-chlorobenzyl)oxy]-2-(3-fluorophenyl)-1,3-thiazole-5-carboxylate (82)
sodium 4-[(4-chlorobenzyl)oxy]-2-(4-methylphenyl)-1,3-thiazole-5-carboxylate (83)
sodium 4-(4-chlorobenzyloxy)-2-(4-chlorophenyl)-1,3-thiazole-5-carboxylate (84)
sodium 4-(2-chlorobenzyloxy)-2-(4-chlorophenyl)-1,3-thiazole-5-carboxylate (85)
sodium 4-(2-chlorobenzyloxy)-2-(4-methylphenyl)-1,3-thiazole-5-carboxylate (86)
sodium 4-(2-chlorobenzyloxy)-2-(3-fluorophenyl)-1,3-thiazole-5-carboxylate (87)
sodium 4-(4-chlorobenzyloxy)-2-(3-fluorophenyl)-1,3-thiazole-5-carboxylate (88)
(1R,2S,5R)-2-isopropyl-5-methylcyclohexyl-4-(benzyloxy)-2-(4-methylphenyl)-1,3-thiazole-5-carboxylate (89)
(1R,2S,5R)-2-isopropyl-5-methylcyclohexyl-4-hydroxy-2-(4-methylphenyl)-1,,3-thiazole-5-carboxylate (90)
ethyl 2-(4-chlorophenyl)-4-(methoxymethoxy)-1,3-thiazole-5-carboxylate (91)
2-(4-chlorophenyl)-4-(methoxymethoxy)-1,3-thiazole-5-carboxylic acid (92)
2-(4-chlorophenyl)-4-(methoxymethoxy)-1,3-thiazole-5-carboxamide (93)
2-(4-chlorophenyl)-4-(methoxymethoxy)-1,3-thiazole-5-carbonitrile (94)
2-(4-chlorophenyl)-5-(1H-tetrazol-5-yl)-1,3-thiazol-4-ol (95)
2-(4-chlorophenyl)-5-(1-methyl-1H-tetrazol-5-yl)-1,3-thiazol-4-ol (96)
2-(3-fluorophenyl)-5-(1-methyl-1H-tetrazol-5-yl)-1,3-thiazol-4-ol (97)
2-(4-chlorophenyl)-5-(5-methyl-4H-1,2,4-triazol-3-yl)-1,3-thiazol-4-ol (98)
2-(3-fluorophenyl)-5-(5-methyl-4H-1,2,4-triazol-3-yl)-1,3-thiazol-4-ol (99)
2-(4-chlorophenyl)-5-(5-methyl-1,2,4-oxadiazol-3-yl)-1,3-thiazol-4-ol (100)
2-(3-fluorophenyl)-5-(5-methyl-1,2,4-oxadiazol-3-yl)-1,3-thiazol-4-ol (101)
3-{4-[(4-chlorobenzyl)oxy]-2-(3-fluorophenyl)-1,3-thiazol-5-yl}-5-methyl-1,2,4-oxadiazole (102)
2-(4-chlorophenyl)-5-(5-methyl-1,3,4-oxadiazol-2-yl)-1,3-thiazol-4-ol (103)
2-(3-fluorophenyl)-5-(5-methyl-1,3,4-oxadiazol-2-yl)-1,3-thiazol-4-ol (104)
ethyl 4-hydroxy-2-phenyl-1,3-oxazole-5-carboxylate (105)
ethyl 2-(3-fluorophenyl)-4-hydroxy-1,3-oxazole-5-carboxylate (106)
ethyl 4-hydroxy-2-(4-methylphenyl)-1,3-oxazole-5-carboxylate (107)
ethyl 4-[(4-chlorobenzyl)oxy]-2-phenyl-1,3-oxazole-5-carboxylate (108)
ethyl 4-[(4-chlorobenzyl)oxy]-2-(3-fluorophenyl)-1,3-oxazole-5-carboxylate (109)
ethyl 4-[(4-chlorobenzyl)oxy]-2-(4-methylphenyl)-1,3-oxazole-5-carboxylate (110)
ethyl 2-phenyl-4-{[4-(trifluoromethyl)benzoyl]oxy}1,3-oxazole-5-carboxylate (111)
4-[(4-chlorobenzyl)oxy]-2-phenyl-1,3-oxazole-5-carboxylic acid (112)
4-[(4-chlorobenzyl)oxy]-2-(3-fluorophenyl)-1,3-oxazole-5-carboxylic acid (113)
4-[(4-chlorobenzyl)oxy]-2-(4-methylphenyl)-1,3-oxazole-5-carboxylic acid (114)
2-(3-fluorophenyl)-5-(5-methyl-1,2,4-oxadiazol-3-yl)-1,3-oxazol-4-ol (115)
3-{4-[(4-chlorobenzyl)oxy]-2-(3-fluorophenyl)-1,3-oxazol-5-yl}-5-methyl-1,2,4-oxadiazole (116)
ethyl 2-(3-fluorophenyl)-5-hydroxy-1,3-thiazole-4-carboxylate (117)

**14.** A compound selected from:

2-(4-chlorophenyl)-4-hydroxy-1,3-thiazole-5-carboxylic acid (1)
4-hydroxy-2-(4-methylphenyl)-1,3-thiazole-5-carboxylic acid (2)
2-(3-fluorophenyl)-4-hydroxy-1,3-thiazole-5-carboxylic acid (3)
2-(4-fluorophenyl)-4-hydroxy-1,3-thiazole-5-carboxylic acid (4)
methyl 2-(2,4-difluorophenyl)-4-hydroxy-1,3-thiazole-5-carboxylate (6)
ethyl 2-(3-fluorophenyl)-4-hydroxy-1 ,3-thiazole-5-carboxylate (10)
ethyl 2-(4-fluorophenyl)-4-hydroxy-1,3-thiazole-5-carboxylate (11)
ethyl 4-hydroxy-2-(pyridin-4-yl)-1,3-thiazole-5-carboxylate (12)
ethyl 2-[4-(dimethylamino)phenyl]-4-hydroxy-1,3-thiazole-5-carboxylate (13)
ethyl 4-hydroxy-2-[2'-(trifluoromethyl)biphenyl-3-yl]-1,3-thiazole-5-carboxylate (15)
ethyl 2-(2'-fluorobiphenyl-3-yl)-4-hydroxy-1,3-thiazole-5-carboxylate (16)
ethyl 4-hydroxy-2-[2'-(trifluoromethyl)biphenyl-4-yl]-1,3-thiazole-5-carboxylate (17)
ethyl 2-(2'-fluorobiphenyl-4-yl)-4-hydroxy-1,3-thiazole-5-carboxylate (18)
ethyl 2-{4-[(2-fluorobenzyl)oxy]phenyl}-4-hydroxy-1,3-thiazole-5-carboxylate (19)
ethyl 2-{4-[(4-fluorobenzyl)oxy]phenyl}-4-hydroxy-1,3-thiazole-5-carboxylate (20)
ethyl 2-(4-fluorophenyl)-4-{[(trifluoromethyl)sulfonyl]oxy}-1,3-thiazole-5-carboxylate (21)

ethyl 4-methoxy-2-(4-methylphenyl)-1,3-thiazole-5-carboxylate (22)
ethyl 2-(4-methylphenyl)-4-(2-methylpropoxy)-1,3-thiazole-5-carboxylate (23)
ethyl 4-(benzyloxy)-2-phenyl-1,3-thiazole-5-carboxylate (24)
ethyl 4-[(3-chlorobenzyl)oxy]-2-(4-chlorophenyl)-1,3-thiazole-5-carboxylate (25)
ethyl 4-[(3-chlorobenzyl)oxy]-2-(3-fluorophenyl)-1,3-thiazole-5-carboxylate (26)
ethyl 4-[(4-chlorobenzyl)oxy]-2-(4-methylphenyl)-1,3-thiazole-5-carboxylate (29)
ethyl 4-[(4-chlorobenzyl)oxy]-2-(3-fluorophenyl)-1,3-thiazole-5-carboxylate (30)
ethyl 4-[(2-chlorobenzyl)oxy]-2-(4-fluorophenyl)-1,3-thiazole-5-carboxylate (32)
ethyl 4-[(2-chlorobenzyl)oxy]-2-(4-chlorophenyl)-1,3-thiazole-5-carboxylate (33)
ethyl 4-[(2-chlorobenzyl)oxy]-2-(3-chlorophenyl)-1,3-thiazole-5-carboxylate (34)
ethyl 4-[(2-chlorobenzyl)oxy]-2-(4-methylphenyl)-1,3-thiazole-5-carboxylate (35)
ethyl 2-phenyl-4-{[4-(trifluoromethyl)benzoyl]oxy}-1,3-thiazole-5-carboxylate (36)
ethyl 2-(3-fluorophenyl)-4-{[4-(trifluoromethyl)benzoyl]oxy}-1,3-thiazole-5-carboxylate (37)
ethyl 2-(4-methylphenyl)-4-{[4-(trifluoromethyl)benzoyl]oxy}-1,3-thiazole-5-carboxylate (38)
ethyl4-(2-((1R,2S,5R)-2-isopropyl-5-methylcyclohexyloxy)acetoyloxy)-2-(4-methylphenyl) -1,3-thiazole-5-carboxylate (39)
ethyl 4-[(benzylcarbamoyl)oxy]-2-(4-chlorophenyl)-1,3-thiazole-5-carboxylate (40)
ethyl 4-(2-aminoethoxy)-2-(4-chlorophenyl)-1,3-thiazole-5-carboxylate (41)
ethyl 2-(4-chlorophenyl)-4-{2-[(furan-2-ylmethyl)amino]ethoxy}-1,3-thiazole-5- carboxylate (42)
4-[(4-chlorobenzyl)oxy]-2-(4-methylphenyl)-1,3-thiazole-5-carboxylic acid (43)
4-[(4-chlorobenzyl)oxy]-2-phenyl-1,3-thiazole-5-carboxylic acid (44)
4-[(4-chlorobenzyl)oxy]-2-(4-chlorophenyl)-1,3-thiazole-5-carboxylic acid (45)
4-[(4-chlorobenzyl)oxy]-2-(3-chlorophenyl)-1,3-thiazole-5-carboxylic acid (46)
4-(benzyloxy)-2-phenyl-1,3-thiazole-5-carboxylic acid (47)
4-[(3-chlorobenzyl)oxy]-2-(3-fluorophenyl)-1,3-thiazole-5-carboxylic acid (48)
4-[(2-chlorobenzyl)oxy]-2-phenyl-1,3-thiazole-5-carboxylic acid (49)
4-[(2-chlorobenzyl)oxy]-2-(4-fluorophenyl)-1,3-thiazole-5-carboxylic acid (50)
4-[(2-chlorobenzyl)oxy]-2-(4-chlorophenyl)-1,3-thiazole-5-carboxylic acid (51)
4-[(2-chlorobenzyl)oxy]-2-(3-chlorophenyl)-1,3-thiazole-5-carboxylic acid (52)
4-[(2-chlorobenzyl)oxy]-2-(4-methylphenyl)-1,3-thiazole-5-carboxylic acid (53)
4-[(2-chlorobenzyl)oxy]-2-(3-fluorophenyl)-1,3-thiazole-5-carboxylic acid (54)
2-phenyl-4-{[4-(trifluoromethyl)benzyl]oxy}-1,3-thiazole-5-carboxylic acid (55)
2-(3-fluorophenyl)-4-{[4-(trifluoromethyl)benzyl]oxy}1,3-thiazole-5-carboxylic acid (56)
2-phenyl-4-{[4-(trifluoromethyl)benzoyl]oxy}1,3-thiazole-5-carboxylic acid (57)
2-(3-fluorophenyl)-4-{[4-(trifluoromethyl)benzoyl]oxy}-1,3-thiazole-5-carboxylic acid (58)
2-(4-methylphenyl)-4-{[4-(trifluoromethyl)benzoyl]oxy}-1,3-thiazole-5-carboxylic acid (59)
4-methoxy-2-(4-methylphenyl)-1,3-thiazole-5-carboxylic acid (60)
2-(4-methylphenyl)-4-(2-methylpropoxy)-1,3-thiazole-5-carboxylic acid (61)
ethyl 4-[(tert-butoxycarbonyl)amino]-2-(4-fluorophenyl)-1,3-thiazole-5-carboxylate (62)
ethyl 4-amino-2-(4-fluorophenyl)-1,3-thiazole-5-carboxylate hydrochloride (63)
ethyl 4-(acetylamino)-2-(4-methylphenyl)-1,3-thiazole-5-carboxylate (64)
ethyl 2-(4-methylphenyl)-4-{[4-(trifluoromethyl)benzoyl]amino}1,3-thiazole-5-carboxylate (65)
ethyl 2-(4-methylphenyl)-4-[(phenylcarbamoyl)amino]-1,3-thiazole-5-carboxylate (66)
ethyl 4-[(2-aminoethyl)amino]-2-(4-methylphenyl)-1,3-thiazole-5-carboxylate (67)
ethyl 2-(4-chlorophenyl)-4-{[2-(methylamino)ethyl]amino}-1,3-thiazole-5-carboxylate (68)
ethyl 2-(4-chlorophenyl)-4-{[2-(propylamino)ethyl]amino}-1,3-thiazole-5-carboxylate (69)
ethyl 4-[(2-aminoethyl)amino]-2-(4-chlorophenyl)-1,3-thiazole-5-carboxylate (70)
ethyl 4-{[2-(methylamino)ethyl]amino}-2-(4-methylphenyl)-1,3-thiazole-5-carboxylate (71)
ethyl 4-[(2-aminoethyl)amino]-2-[2'-(trifluoromethyl)biphenyl-4-yl]-1,3-thiazole-5-carboxylate (72)
ethyl 4-[(2-aminoethyl)amino]-2-[2'-(trifluoromethyl)biphenyl-3-yl]-1,3-thiazole-5-carboxylate (73)
ethyl 2-(4-chlorophenyl)-4-{[2-(cyclopentylamino)ethyl]amino}-1,3-thiazole-5-carboxylate (74)
ethyl 2-phenyl-4-{[2-(pyrrolidin-1-yl)ethyl]amino}-1,3-thiazole-5-carboxylate (75)
ethyl 4-(benzylamino)-2-(3-fluorophenyl)-1,3-thiazole-5-carboxylate (76)
ethyl 4-[(1,3-benzodioxol-5-ylmethyl)amino]-2-(3-fluorophenyl)-1,3-thiazole-5-carboxylate (77)
ethyl 2-(3-fluorophenyl)-4-[(pyridin-3-ylmethyl)amino]-1,3-thiazole-5-carboxylate (78)
4-[(2-aminoethyl)amino]-2-(4-methylphenyl)-1,3-thiazole-5-carboxylic acid (79)
4-{[2-(methylamino)ethyl]amino}-2-(4-methylphenyl)-1,3-thiazole-5-carboxylic acid (80)
4-[(2-aminoethyl)amino]-2-(3-fluorophenyl)-1,3-thiazole-5-carboxylic acid (81)

sodium 4-[(3-chlorobenzyl)oxy]-2-(3-fluorophenyl)-1,3-thiazole-5-carboxylate (82)
sodium 4-[(4-chlorobenzyl)oxy]-2-(4-methylphenyl)-1,3-thiazole-5-carboxylate (83)
sodium 4-(4-chlorobenzyloxy)-2-(4-chlorophenyl)-1,3-thiazole-5-carboxylate (84)
sodium 4-(2-chlorobenzyloxy)-2-(4-chlorophenyl)-1,3-thiazole-5-carboxylate (85)
sodium 4-(2-chlorobenzyloxy)-2-(4-methylphenyl)-1,3-thiazole-5-carboxylate (86)
sodium 4-(2-chlorobenzyloxy)-2-(3-fluorophenyl)-1,3-thiazole-5-carboxylate (87)
sodium 4-(4-chlorobenzyloxy)-2-(3-fluorophenyl)-1,3-thiazole-5-carboxylate (88)
(1 R,2S,5R)-2-isopropyl-5-methylcyclohexyl-4-(benzyloxy)-2-(4-methylphenyl)-1,3-thiazole-5-carboxylate (89)
(1R,2S,5R)-2-isopropyl-5-methylcyclohexyl-4-hydroxy-2-(4-methylphenyl)-1"3-thiazole-5-carboxylate (90)
ethyl 2-(4-chlorophenyl)-4-(methoxymethoxy)-1,3-thiazole-5-carboxylate (91)
2-(4-chlorophenyl)-4-(methoxymethoxy)-1,3-thiazole-5-carboxylic acid (92)
2-(4-chlorophenyl)-4-(methoxymethoxy)-1,3-thiazole-5-carboxamide (93)
2-(4-chlorophenyl)-4-(methoxymethoxy)-1,3-thiazole-5-carbonitrile (94)
2-(4-chlorophenyl)-5-(1H-tetrazol-5-yl)-1,3-thiazol-4-ol (95)
2-(4-chlorophenyl)-5-(1-methyl-1H-tetrazol-5-yl)-1,3-thiazol-4-ol (96)
2-(3-fluorophenyl)-5-(1-methyt-1H-tetrazol-5-yl)-1,3-thiazol-4-ol (97)
2-(4-chlorophenyl)-5-(5-methyl-4H-1,2,4-triazol-3-yl)-1,3-thiazol-4-ol (98)
2-(3-fluorophenyl)-5-(5-methyl-4H-1,2,4-triazol-3-yl)-1,3-thiazol-4-ol (99)
2-(4-chlorophenyl)-5-(5-methyl-1,2,4-oxadiazol-3-yl)-1,3-thiazol-4-ol (100)
2-(3-fluorophenyl)-5-(5-methyl-1,2,4-oxadiazol-3-yl)-1,3-thiazol-4-ol (101)
3-{4-[(4-chlorobenzyl)oxy]-2-(3-fluorophenyl)-1,3-thiazol-5-yl}-5-methyl-1,2,4-oxadiazole (102)
2-(4-chlorophenyl)-5-(5-methyl-1,3,4-oxadiazol-2-yl)-1,3-thiazol-4-ol (103)
2-(3-fluorophenyl)-5-(5-methyl-1,3,4-oxadiazol-2-yl)-1,3-thiazol-4-ol (104)
ethyl 4-hydroxy-2-phenyl-1,3-oxazole-5-carboxylate (105)
ethyl 2-(3-fluorophenyl)-4-hydroxy-1,3-oxazole-5-carboxylate (106)
ethyl 4-hydroxy-2-(4-methylphenyl)-1,3-oxazole-5-carboxylate (107)
ethyl 4-[(4-chlorobenzyl)oxy]-2-phenyl-1,3-oxazole-5-carboxylate (108)
ethyl 4-[(4-chlorobenzyl)oxy]-2-(3-fluorophenyl)-1,3-oxazole-5-carboxylate (109)
ethyl 4-[(4-chlorobenzyl)oxy]-2-(4-methylphenyl)-1,3-oxazole-5-carboxylate (110)
ethyl 2-phenyl-4-{[4-(trifluoromethyl)benzoyl]oxy}-1,3-oxazole-5-carboxylate (111)
4-[(4-chlorobenzyl)oxy]-2-phenyl-1,3-oxazole-5-carboxylic acid (112)
4-[(4-chlorobenzyl)oxy]-2-(3-fluorophenyl)-1,3-oxazole-5-carboxylic acid (113)
4-[(4-chlorobenzyl)oxy]-2-(4-methylphenyl)-1,3-oxazole-5-carboxylic acid (114)
2-(3-fluorophenyl)-5-(5-methyl-1,2,4-oxadiazol-3-yl)-1,3-oxazol-4-ol (115)
3-{4-[(4-chlorobenzyl)oxy]-2-(3-fluorophenyl)-1,3-oxazol-5-yl}-5-methyl-1,2,4-oxadiazole (116) ethyl 2-(3-fluorophenyl)-5-hydroxy-1,3-thiazole-4-carboxylate (117)

**15.** A compound as claimed in anyone of claims 1 to 14, for use in the prevention and/or treatment of a disease selected from the group consisting of inflammatory conditions, ischaemia, pain, urological disorders, stroke, psychiatric disorders and neurodegeneration.

**16.** A compound as claimed in claim 15, wherein said disease is selected from chronic pain, neuropathic pain, postoperative pain, cancer pain, osteoarthritic pain, rheumatoid arthritic pain, neuralgia, fibromyalgia, neuropathies, fibromyalgia, algesia, nerve injury, migraine, headache, itch, irritable bowel disease, respiratory diseases, pulmonary hypertension, COPD, asthma, painful bladder syndrome, interstitial cystitis, detrusor overactivity, urinary incontinence, neurogenic detrusor overactivity, idiopathic detrusor overactivity, benign prostatic hyperplasia, lower urinary tract disorders and lower urinary tract symptoms, anxiety, depression.

**17.** A pharmaceutical composition comprising as the active ingredient at least one compound according to any one of claims 1 to 16 in combination with pharmaceutically acceptable excipients and/or diluents.

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| A | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| B | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| C | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| D | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| E | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| F | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| G | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| H | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| I | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| J | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| K | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| L | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| M | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| N | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |
| O | TYR | $EC_{100}$ | TYR | TYR | TYR | TYR | TYR | TYR | TYR | TYR | TYR | TYR | TYR | TYR | TYR | TYR | TYR | TYR | TYR | TYR | TYR | TYR | TYR | ANT |
| P | TYR | TYR | TYR | TYR | TYR | TYR | TYR | TYR | TYR | TYR | TYR | TYR | TYR | TYR | TYR | TYR | TYR | TYR | TYR | TYR | TYR | TYR | $IC_{100}$ | ANT |

Figure 1

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 |
|---|---|---|---|---|---|---|---|---|---|---|---|---|---|---|---|---|---|---|---|---|---|---|---|---|
| A | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| B | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| C | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| D | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| E | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| F | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| G | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| H | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| I | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| J | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| K | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| L | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| M | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| N | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| O | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |
| P | AGO | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ | $EC_{80}$ |

Figure 2

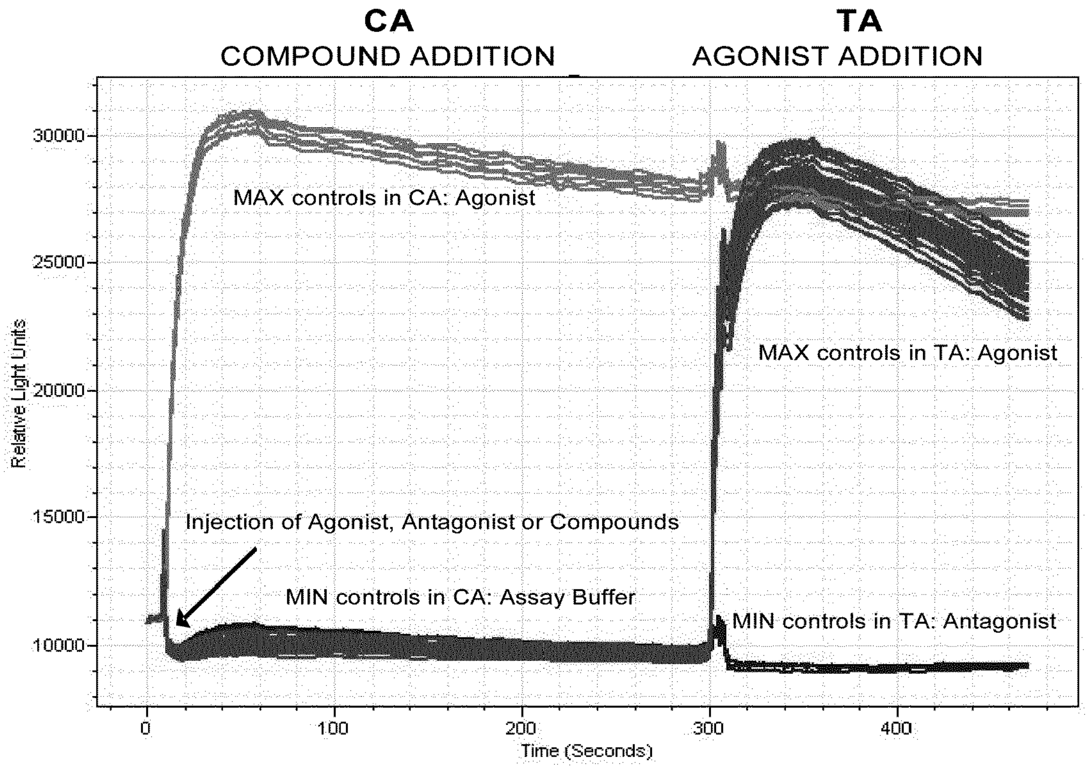


Figure 3

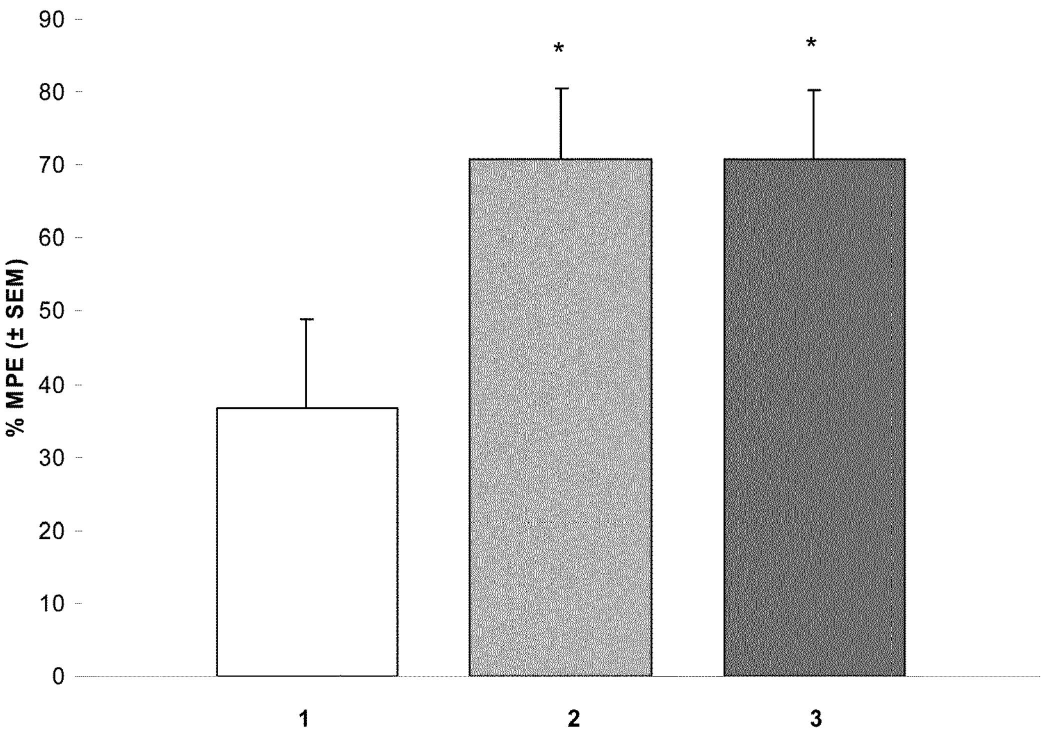


Figure 4

Europäisches Patentamt
European Patent Office
Office européen des brevets

# EUROPEAN SEARCH REPORT

Application Number
EP 11 19 4365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 614 520 A (KONDO SHIRO [JP] ET AL) 25 March 1997 (1997-03-25)<br>* abstract *<br>* column 1, lines 10-16 *<br>* column 1, line 45 - page 2, line 37 *<br>* column 37; example 21 *<br>* claims 1-15 *<br>----- | 1-17 | INV.<br>A61K31/421<br>A61K31/422<br>A61K31/426<br>A61K31/427<br>C07D263/40<br>C07D263/48<br>C07D277/34<br>C07D277/42<br>C07D277/56<br>C07D417/02<br>C07D413/02<br>C07D403/02<br>A61P29/00 |
| X | FRANCIS A J KERDESKY ET AL: "4-Hydroxythiazole inhibitors of 5-Lipoxygenase", JOURNAL OF MEDICINAL CHEMISTRY, AMERICAN CHEMICAL SOCIETY, US, vol. 34, no. 7, 1 January 1991 (1991-01-01), pages 2158-2165, XP002423682, ISSN: 0022-2623, DOI: 10.1021/JM00111A035<br>* abstract *<br>* page 2158, left-hand column, paragraph first *<br>* page 2159, right-hand column; example 70; table I *<br>* page 2161, right-hand column, lines 4-18 *<br>----- | 1-5, 7-12,15, 17 | |
| | | | TECHNICAL FIELDS SEARCHED (IPC) |
| A | US 4 153 607 A (EILINGSFELD HEINZ ET AL) 8 May 1979 (1979-05-08)<br>* column 8; example 5 *<br>----- | 1-17 | A61K<br>C07D<br>A61P |
| A | EP 1 321 463 A1 (SHIRE BIOCHEM INC [CA] VIROCHEM PHARMA INC [CA]) 25 June 2003 (2003-06-25)<br>* page 11, paragraphs 95,97 *<br>----- | 1-17 | |
| | -/-- | | |

The present search report has been drawn up for all claims

2

| Place of search | Date of completion of the search | Examiner |
|---|---|---|
| Munich | 16 May 2012 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 19 4365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RONKIN S M ET AL: "Discovery of pyrazolthiazoles as novel and potent inhibitors of bacterial gyrase", BIOORGANIC & MEDICINAL CHEMISTRY LETTERS, PERGAMON, ELSEVIER SCIENCE, GB, vol. 20, no. 9, 1 May 2010 (2010-05-01), pages 2828-2831, XP027012843, ISSN: 0960-894X [retrieved on 2010-03-15] * page 2830; figure 2; compounds 11,12 * ----- | 1-17 | |
| A | ILYIN A P ET AL: "One-step assembly of carbamoyl substituted annulated 1,4-oxazepines", TETRAHEDRON LETTERS, ELSEVIER, AMSTERDAM, NL, vol. 47, no. 15, 10 April 2006 (2006-04-10), pages 2649-2653, XP025003908, ISSN: 0040-4039, DOI: 10.1016/J.TETLET.2006.01.158 [retrieved on 2006-04-10] * page 2650; figure 1; compound 1ac * ----- | 1-17 | TECHNICAL FIELDS SEARCHED (IPC) |
| A | HERTZOG D L ET AL: "The discovery and optimization of pyrimidinone-containing MCH R1 antagonists", BIOORGANIC & MEDICINAL CHEMISTRY LETTERS, PERGAMON, ELSEVIER SCIENCE, GB, vol. 16, no. 18, 15 September 2006 (2006-09-15), pages 4723-4727, XP025107256, ISSN: 0960-894X, DOI: 10.1016/J.BMCL.2006.07.008 [retrieved on 2006-09-15] * page 4725; figure 4; compound 24 * ----- -/-- | 1-17 | |

The present search report has been drawn up for all claims

| Place of search | Date of completion of the search | Examiner |
|---|---|---|
| Munich | 16 May 2012 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 19 4365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | O TSUGE: "Studies of acyl and thioacyl isocyanates-XII The reactions of benzoyl and thiobenzoyl isocyanates with sulfonium ylides and with diazoalkanes", TETRAHEDRON, vol. 29, no. 14, 1 January 1973 (1973-01-01), pages 1983-1990, XP55026677, ISSN: 0040-4020, DOI: 10.1016/0040-4020(73)80134-6 * page 1985; table 2; compounds 7a,7b * ----- | 1-17 | |
| A | DRIDI K ET AL: "REACTION OF MERCAPTOACETATE AND HALIDES CONTAINING ACTIVATED METHYLENES WITH THIOCARBAMOYLIMIDATES: A NOVEL APPROACH TO THE SYNTHESIS OF AMINOTHIAZOLE DERIVATIVES", SYNTHETIC COMMUNICATIONS, TAYLOR & FRANCIS GROUP, PHILADELPHIA, PA, vol. 1, no. 28, 1 January 1998 (1998-01-01), pages 167-174, XP008005382, ISSN: 0039-7911, DOI: 10.1080/00397919808005086 * page 168; compound 2c * ----- -/-- | 1-17 | TECHNICAL FIELDS SEARCHED (IPC) |

The present search report has been drawn up for all claims

| Place of search | Date of completion of the search | Examiner |
|---|---|---|
| Munich | 16 May 2012 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 11 19 4365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOYD G V ED - BELLUS D (ED): "PRODUCT CLASS 12: OXAZOLES", 1 January 2002 (2002-01-01), SCIENCE OF SYNTHESIS. CATEGORY 2 : HETARENES AND RELATED RING SYSTEMS, FIVE-MEMBERED HETARENES WITH ONE CHALCOGEN AND ONE ADDITIONAL HETEROATOM; [METHODS OF MOLECULAR TRANSFORMATIONS], STUTTGART : GEORG THIEME VERLAG, DE, PAGE(S) 383 - 479, XP001156115, ISBN: 978-3-13-112261-2 * the whole document * ----- | 1-17 | |
| A | WO 2010/125831 A1 (RAQUALIA PHARMA INC [JP]; INOUE TADASHI [JP]; OHMI MASASHI [JP]; KAWAM) 4 November 2010 (2010-11-04) * the whole document * ----- | 1-17 | |
| A | US 2010/160289 A1 (MACIELAG MARK J [US] ET AL) 24 June 2010 (2010-06-24) * the whole document * ----- | 1-17 | TECHNICAL FIELDS SEARCHED (IPC) |

The present search report has been drawn up for all claims

2

| Place of search | Date of completion of the search | Examiner |
|---|---|---|
| Munich | 16 May 2012 | Garabatos-Perera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 19 4365

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2012

| Patent document cited in search report | Publication date | Patent family member(s) | Publication date |
|---|---|---|---|
| US 5614520 A | 25-03-1997 | NONE | |
| US 4153607 A | 08-05-1979 | CH 635847 A5 | 29-04-1983 |
| | | DE 2713573 A1 | 05-10-1978 |
| | | GB 1596149 A | 19-08-1981 |
| | | JP 53119889 A | 19-10-1978 |
| | | US 4153607 A | 08-05-1979 |
| EP 1321463 A1 | 25-06-2003 | NONE | |
| WO 2010125831 A1 | 04-11-2010 | CA 2757761 A1 | 04-11-2010 |
| | | CN 102427810 A | 25-04-2012 |
| | | EP 2424517 A1 | 07-03-2012 |
| | | US 2012094964 A1 | 19-04-2012 |
| | | WO 2010125831 A1 | 04-11-2010 |
| US 2010160289 A1 | 24-06-2010 | CA 2747662 A1 | 15-07-2010 |
| | | CN 102325750 A | 18-01-2012 |
| | | EP 2379496 A1 | 26-10-2011 |
| | | US 2010160289 A1 | 24-06-2010 |
| | | WO 2010080398 A1 | 15-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006040136 A
- WO 2007017092 A
- WO 2007017093 A
- WO 2007017094 A
- WO 2007080109 A
- WO 2007134107 A
- WO 2009012430 A
- WO 2010103381 A
- WO 2010125831 A


### Non-patent literature cited in the description


- **MCKEMY D.D. et al.** *Nature,* 2002, vol. 416, 52-58
- **PEIER A.M. et al.** *Cell,* 2002, vol. 108, 705-715
- **NILIUS B. et al.** *J. Physiol.,* 2005, vol. 567, 35-44
- **VANDEN ABEELE F. et al.** *J. Biol.Chem.,* 2006, vol. 281, 40174-40182
- **DE PETROCELLIS L. et al.** *Exp.Cell. Res.,* 2007, vol. 313, 1911-1920
- **ROHACS T. et al.** *Nat. Neurosci.,* 2005, vol. 8, 626-634
- **DE GROAT W.C. et al.** *Urology,* 1997, vol. 50, 36-52
- **EVERAERTS W. et al.** *Neurol. Urodyn.,* 2008, vol. 27, 264-73
- **NILIUS B. et al.** *Science STKE,* 2005, vol. 295, re8
- **VOETS T. et al.** *Nat. Chem. Biol.,* 2005, vol. 1, 85-92
- **MUKERJI G. et al.** *Urology,* 2006, vol. 6, 31-36
- **LAZZERI M. et al.** *Ther. Adv. Urol.,* 2009, vol. 1, 33-42
- **NILIUS B. et al.** *Biochim. Biophys. Acta,* 2007, vol. 1772, 805-12
- **WISSENBACH U. et al.** *Biol. Cell.,* 2004, vol. 96, 47-54
- **NILIUS B. et al.** *Physiol. Rev.,* 2007, vol. 87, 165-217
- **PROUDFOOT C.J. et al.** *Curr. Biol.,* 2006, vol. 16, 1591-1605
- Remington's Pharmaceutical Sciences Handbook. MACK Publishing, 1990
- **BENNETT GJ et al.** *Pain,* 1988, vol. 33, 87-107
- **JHAVERI MD et al.** *Eur. J. Neurosci.,* 2005, vol. 22 (2), 361-70
- **BRIERLEY SM et al.** *Gastroenterology,* June 2008, vol. 134 (7), 2059-69